Europäisches Patentamt
European Patent Office
Office européen des brevets

(11) Veröffentlichungsnummer: **0 005 142 A1**

(12) # EUROPÄISCHE PATENTANMELDUNG

(21) Anmeldenummer: 79100363.5

(22) Anmeldetag: 08.02.79

(51) Int. Cl.²: **C 07 D 295/08,** A 61 K 31/495, C 07 C 89/02, C 07 C 93/06, C 07 C 103/38, C 07 C 103/60, C 07 D 295/12, C 07 D 295/14, C 07 D 295/18, C 07 D 405/12

(30) Priorität: 08.02.78 US 875966

(43) Veröffentlichungstag der Anmeldung: 14.11.79 Patentblatt 79/23

(84) Benannte Vertragsstaaten: BE CH DE FR IT LU NL SE

(71) Anmelder: F.Hoffmann-La Roche & Co. Aktiengesellschaft, Abt. VIII-Pt, CH-4002 Basel (CH)

(72) Erfinder: Fahrenholtz, Kenneth E., 28 Winding Lane, Bloomfield N.J. 07003 (US)
Erfinder: Guthrie, Robert W., 102 Alberta Drive, Saddle Brook N.J. 07665 (US)
Erfinder: Kierstead, Richard W., 30 Willowbrook Drive, North Caldwell N.J. 07006 (US)
Erfinder: Tilley, Jefferson W., 19 Evergreen Drive, North Caldwell N.J. 07006 (US)

(74) Vertreter: Lederer, Franz, Dr. et al, Patentanwälte Lederer, Meyer Lucile-Grahn-Strasse 22, D-8000 München 80 (DE)

(54) **Phenylpiperazinderivate, Verfahren zu ihrer Herstellung, Zwischenprodukte, Arzneimittel enthaltend solche Phenylpiperazinderivate und ihre pharmazeutische Verwendung.**

(57) Phenylpiperazin-Derivate der Formel

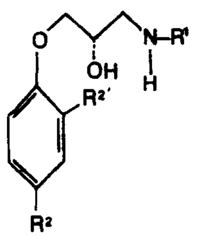

I

worin $R^1$ Niederalkyl, einer der Reste $R^2$ und $R^{2'}$ Wasserstoff und der andere die Gruppe

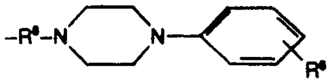

$R^8$ eine der Gruppen $-O-(CH_2)_n-$, $-NH\underset{O}{\underset{\|}{C}}-(CH_2)_n-$ und $-CH_2\underset{O}{\underset{\|}{C}}NH(CH_2)_n-$, $R^9$ Wasserstoff oder Niederalkoxy und n eine ganze Zahl von 2 bis 20 bedeuten,
die Racemate davon und die pharmazeutisch annehmbaren Salze dieser Verbindungen besitzen wertvolle $\alpha$- und $\beta$-adrenergisch blockierende und sekretionshemmende Wirkung und können demnach, insbesondere in Form pharmazeutischer Präparate, zur Bekämpfung von Bluthochdruck und Spasmen verwendet werden. Diese Verbindungen können nach verschiedenen Verfahren hergestellt werden.

EP 0 005 142 A1

0005142
8. Feb. 1979

F. Hoffmann-La Roche & Co. Aktiengesellschaft, Basel, Schweiz

RAN 4028/1

**BEZEICHNUNG GEÄNDERT**
**siehe Titelseite**

Neue Phenylpiperazinderivate, Verfahren und Zwischenprodukte zu deren Herstellung und Arzneimittel enthaltend ein solches Phenylpiperazinderivat sowie die Verwendung eines solchen Phenylpiperazinderivates bei der Bekämpfung von Krankheiten.

Die vorliegende Erfindung betrifft Phenylpiperazin-Derivate der allgemeinen Formel

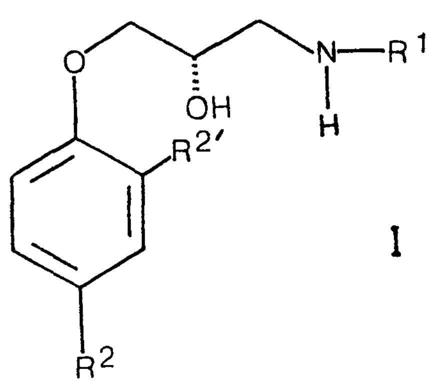

I

JPM/30.1.79

worin $R^1$ Niederalkyl, einer der Reste $R^2$ und $R^{2'}$ Wasserstoff und der andere die Gruppe

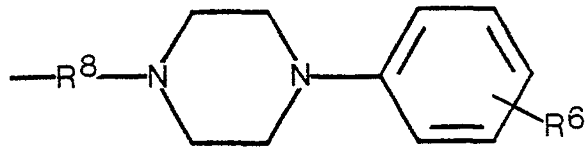

,

$R^8$ eine der Gruppen $-O-(CH_2)_n-$, $-NHC(=O)-(CH_2)_n-$ und $-CH_2C(=O)NH(CH_2)_n-$, $R^6$ Wasserstoff oder Niederalkoxy und n eine ganze Zahl von 2 bis 20 bedeuten, deren Racemate sowie die pharmazeutisch annehmbaren Säureadditionssalze dieser Verbindungen.

Der in dieser Beschreibung verwendete Ausdruck "Niederalkyl" umfasst gerad- und verzweigtkettige Kohlenwasserstoffgruppen mit 1 bis 10 Kohlenstoffatomen; bevorzugt sind verzweigtkettige Kohlenwasserstoffgruppen mit 3 oder 4 Kohlenstoffatomen, wie z.B. Isopropyl oder tert.-Butyl.

Der Ausdruck "Niederalkoxy" bezeichnet gerad- oder verzweigtkettige gesättigte Alkyläthergruppen mit 1 bis 7 Kohlenstoffatomen, vorzugsweise 1 bis 4 Kohlenstoffatomen, wie Methoxy, Aethoxy, Propoxy usw.

Der Ausdruck "Halo" bzw. "Halogen" bezeichnet die vier Formen Brom, Chlor, Fluor und Jod; bevorzugt sind Brom und Chlor.

Der nachfolgend verwendete Ausdruck "Schutzgruppe" bezeichnet bekannte Gruppen, wie Arylsulfonyl, beispielsweise Brosyl, Tosyl, Benzolsufonyl, Alkylsulfonyl, z.B. Mesyl, oder Benzyl usw.

Der nachfolgend verwendete Ausdruck "Abgangsgruppe" bezeichnet bekannte Gruppen, wie Halogen, vorzugsweise Chlor oder Brom, Alkylsulfonyloxy, z.B. Mesyloxy, oder Arylsulfonyloxy, z.B. Tosyloxy, usw.

Von den Verbindungen der obigen Formel I und deren Racematen sind diejenigen Verbindungen der Formel I bevorzugt, worin $R^{2'}$ Wasserstoff bedeutet, d.h. die Verbindungen der Formel

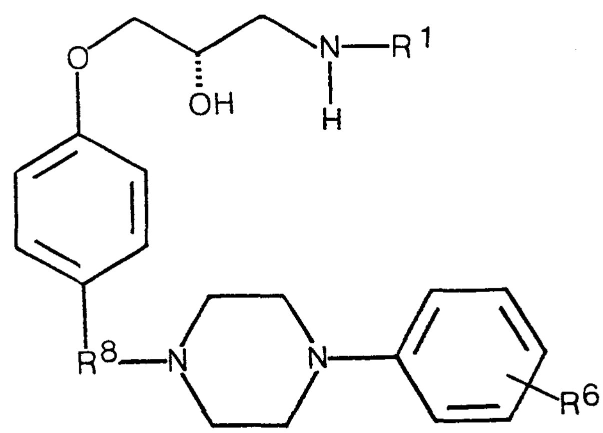

I'

worin $R^1$, $R^6$ und $R^8$ die oben angegebene Bedeutung haben,

deren Racemate sowie die pharmazeutisch annehmbaren Säureadditionssalze dieser Verbindungen.

Eine weitere bevorzugte Klasse von Verbindungen der Formel I und deren Racematen sind diejenigen Verbindungen, worin $R^1$ eine verzweigtkettige Niederalkylgruppe, vorzugsweise mit 3 oder 4 Kohlenstoffatomen, besonders bevorzugt Isopropyl oder tert.Butyl, bedeutet. $R^8$ bedeutet vorzugsweise eine der Gruppen $-O(CH_2)_n-$ oder $-CH_2\overset{O}{\overset{\|}{C}}NH(CH_2)_n-$, worin n eine ganze Zahl von 2 bis 10, vorzugsweise die Zahl 2, bedeutet. $R^6$ bedeutet vorzugsweise Wasserstoff.

Aus den obigen Ausführungen folgt, dass von den Verbindungen der obigen Formel I und deren Racematen diejenigen Verbindungen besonders bevorzugt sind, worin $R^{2'}$ Wasserstoff, $R^1$ eine verzweigtkettige Niederalkylgruppe mit 3 oder 4 Kohlenstoffatomen, vorzugsweise Isopropyl oder tert.Butyl,

$R^8$ eine der Gruppen $-O(CH_2)_n-$ und $-CH_2\underset{\underset{O}{\|}}{C}NH(CH_2)_n-$ , n eine ganze Zahl von 2 bis 10, vorzugsweise 2, und $R^6$ Wasserstoff bedeuten. Von diesen besonders bevorzugten Verbindungen sind die (S)-Isomeren ganz besonders bevorzugt.

Die bevorzugtesten Verbindungen der Formel I sind:

(S)-1-[2-(4-(2-Hydroxy-3-isopropylaminopropoxy)-phenoxy)äthyl]-4-phenylpiperazin,

(S)-1-[2-(4-(2-Hydroxy-3-tert-butylaminopropoxy)-phenoxy)äthyl]-4-phenylpiperazin,

(S)-1-[2-(4-(2-Hydroxy-3-isopropylaminopropoxy)-phenoxy)äthyl]-4-(2-methoxyphenyl)piperazin,

(R,S)-1-[2-(4-(2-Hydroxy-3-isopropylaminopropoxy)-phenoxy)äthyl]-4-phenylpiperazin,

(S)-1-[6-(4-(2-Hydroxy-3-isopropylaminopropoxy)-phenoxy)hexyl]-4-phenylpiperazin,

(S)-1-[11-(4-(2-Hydroxy-3-isopropylaminopropoxy)-phenoxy)undecanyl]-4-phenylpiperazin,

(S)-1-[3-(2-(2-Hydroxy-3-isopropylaminopropoxy)-phenoxy)propyl]-4-phenylpiperazin,

(S)-1-[6-(2-(2-Hydroxy-3-isopropylaminopropoxy)-phenoxy)hexyl]-4-phenylpiperazin,

4-[(2-Hydroxy-3-isopropylaminopropoxy)phenyl]-N-[2-(4-phenyl-1-piperazinyl)äthyl]acetamid,

4-[(2-Hydroxy-3-isopropylaminopropoxy)phenyl]-N-[6-(4-phenyl-1-piperazinyl)hexyl]acetamid,

4-[(2-Hydroxy-3-isopropylaminopropoxy)phenyl]-N-[11-(4-phenyl-1-piperazinyl)undecyl]acetamid,

(S)-4-(2-Hydroxy-3-isopropylaminopropoxy)phenyl-N-[2-(4-phenyl-1-piperazinyl)äthyl]acetamid,

(S)-4-(2-Hydroxy-3-isopropylaminopropoxy)phenyl-N-[6-(4-phenyl-1-piperazinyl)hexyl]acetamid,

(R,S)-N-[4-(2-Hydroxy-3-isopropylaminopropoxy)-phenyl]-4-phenyl-1-piperazinpropanamid,

(R,S)-N-[4-(2-Hydroxy-3-isopropylaminopropoxy)-

phenyl]-4-phenyl-1-piperazinhexanamid,

(R,S)-N-[4-(2-Hydroxy-3-isopropylaminopropoxy)-phenyl]-4-phenyl-1-piperazinundecanamid.

Erfindungsgemäss können die Verbindungen der obigen Formel I, deren Racemate und ihre pharmazeutisch annehmbaren Säureadditionssalze hergestellt werden, indem man

a) eine Verbindung der allgemeinen Formel

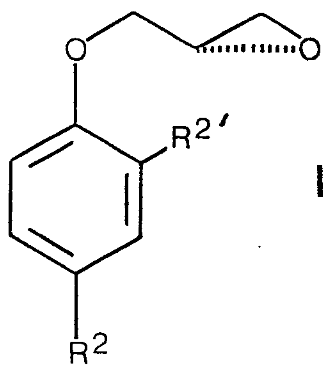

II

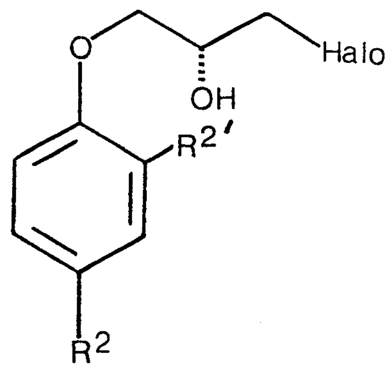

II'

worin $R^2$ und $R^{2'}$ die oben angegebene Bedeutung haben, oder ein entsprechendes Racemat mit einem Amin der allgemeinen Formel

$$R^1NH_2 \qquad III$$

worin $R^1$ die oben angegebene Bedeutung hat, umsetzt, oder

b) zur Herstellung einer Verbindung der obigen Formel I oder eines entsprechenden Racemates, worin $R^8$ die Gruppe $-O(CH_2)_n-$ bedeutet und die übrigen Symbole die oben angegebene Bedeutung haben, in einer Verbindung der allgemeinen Formel

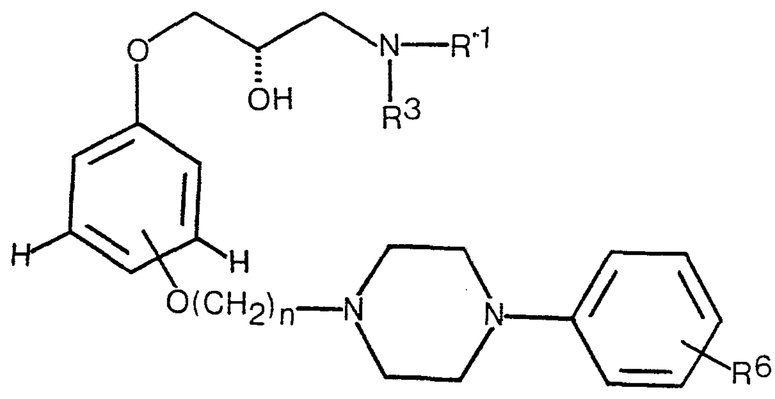

IV

worin $R^3$ eine Schutzgruppe bedeutet und $R^1$, $R^6$ und n die oben angegebene Bedeutung haben,

oder in einem entsprechenden Racemat die Schutzgruppe reduktiv abspaltet, oder

c) zur Herstellung einer Verbindung der obigen Formel I oder eines entsprechenden Racemates, worin $R^8$ die Gruppe $-O-(CH_2)_n-$ bedeutet und die übrigen Symbole die oben angegebene Bedeutung haben, eine Verbindung der allgemeinen Formel

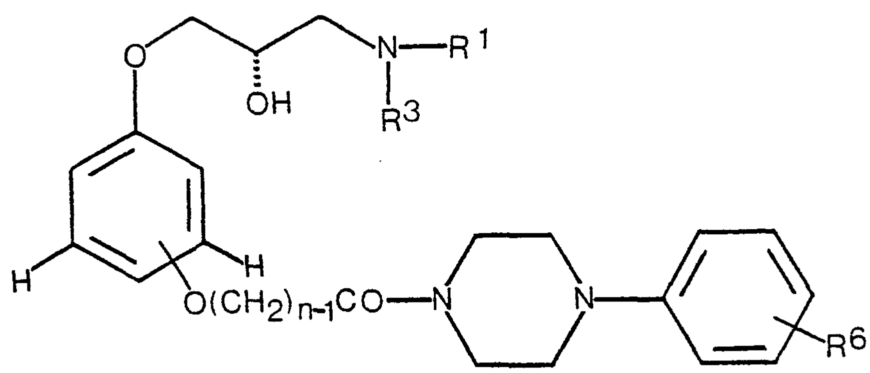

V

worin $R^1$, $R^3$, $R^6$ und n die oben angegebene Bedeutung haben,

oder ein entsprechendes Racemat mit einem geeigneten Reduktionsmittel behandelt, oder

d) zur Herstellung einer Verbindung der obigen Formel I oder eines entsprechenden Racemates, worin $R^8$ die Gruppe

$-O(CH_2)_n-$ bedeutet und die übrigen Symbole die oben angegebene Bedeutung haben, eine Verbindung der allgemeinen Formel

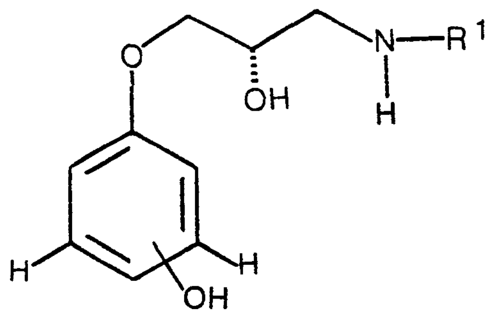

VI

worin $R^1$ die oben angegebene Bedeutung hat, oder ein entsprechendes Racemat mit einer Verbindung der allgemeinen Formel

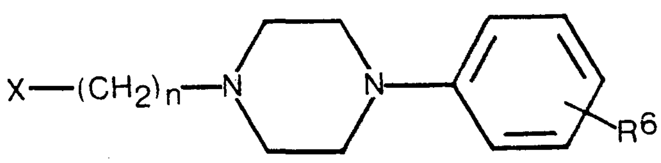

VII

worin X eine Abgangsgruppe bedeutet und $R^6$ und n die oben angegebene Bedeutung haben, behandelt, oder

e) zur Herstellung einer Verbindung der obigen Formel I oder eines entsprechenden Racemates, worin $R^8$ die Gruppe $-CH_2CONH(CH_2)_n-$ bedeutet und die übrigen Symbole die oben angegebene Bedeutung haben, eine Verbindung der allgemeinen Formel.

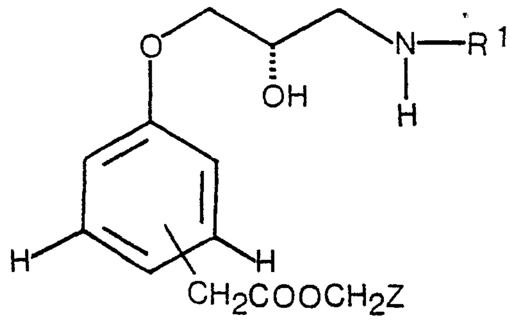

VIII

worin Z Wasserstoff oder Cyan bedeutet und $R^1$ die oben angegebene Bedeutung hat,

oder ein entsprechendes Racemat mit einer Verbindung der allgemeinen Formel

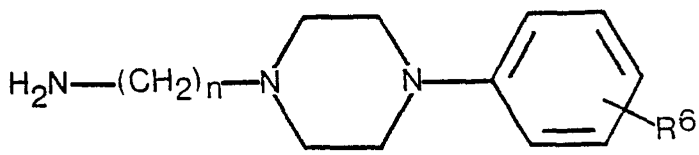

IX

worin $R^6$ und n die oben angegebene Bedeutung haben, behandelt, oder

f) zur Herstellung einer Verbindung der obigen Formel I oder eines entsprechenden Racemates, worin $R^8$ die Gruppe $-NHCO(CH_2)_n-$ bedeutet und die übrigen Symbole die oben angegebene Bedeutung haben, eine Verbindung der allgemeinen Formel

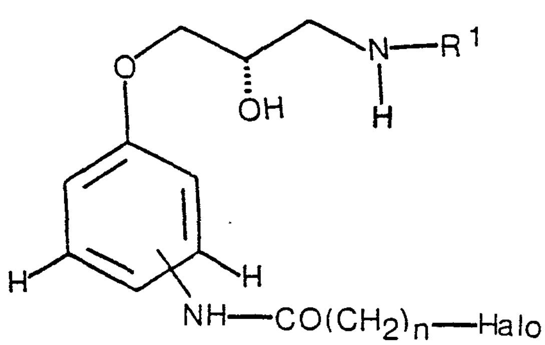

X

worin $R^1$ und n die oben angegebene Bedeutung haben, oder ein entsprechendes Racemat mit einer Verbindung der allgemeinen Formel

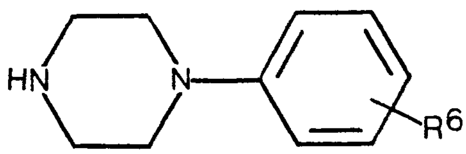

XI

worin $R^6$ die oben angegebene Bedeutung hat, behandelt, oder

g) zur Herstellung einer Verbindung der obigen Formel I oder eines entsprechenden Racemates, worin $R^8$ die Gruppe $-NHCO(CH_2)_2-$ bedeutet und die übrigen Symbole die oben angegebene Bedeutung haben, eine Verbindung der allgemeinen Formel

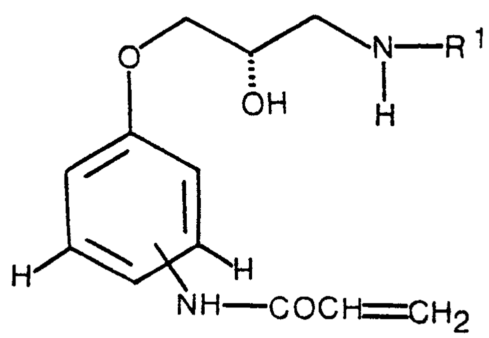

XII

worin $R^1$ die oben angegebenen Bedeutung hat, oder ein entsprechendes Racemat mit einer Verbindung der obigen Formel XI behandelt, oder

h) zur Herstellung des Racemates einer Verbindung der obigen Formel I, worin $R^8$ die Gruppe $-O(CH_2)_2-$ bedeutet und die übrigen Symbole die oben angegebene Bedeutung haben, eine Verbindung der allgemeinen Formel

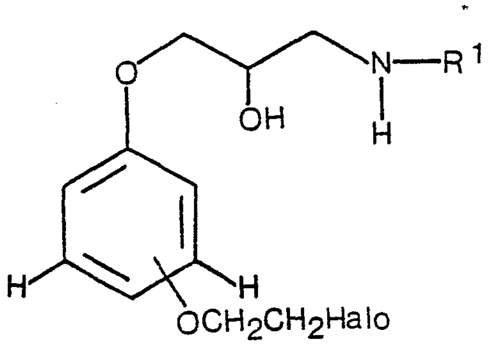

XIII

worin $R^1$ die oben angegebene Bedeutung hat, mit einer Verbindung der obigen Formel XI behandelt, und

i) erwünschtenfalls, eine erhaltene racemische Mischung in die optischen Antipoden auftrennt und

j) erwünschtenfalls, eine erhaltene Verbindung in ein pharmazeutisch annehmbares Säureadditionssalz überführt.

Die folgenden Reaktionsschemas erläutern die Verfahren zur Herstellung der neuen Endprodukte gemäss vorliegender Erfindung. In diesen Reaktionsschemas haben die Symbole $R^1$, $R^6$ und n die oben angegebene Bedeutung,

Q bedeutet die Gruppe 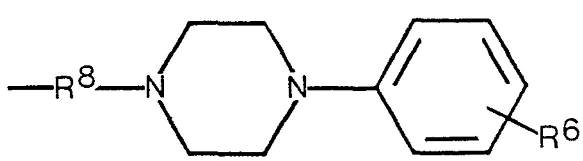 ,

$R^{3'}$ bedeutet Wasserstoff oder eine Schutzgruppe, $R^4$ bedeutet Halogen, eine Alkyl- oder Arylsulfonyloxygruppe und $R^5$ bedeutet Wasserstoff oder eine Alkyl- oder Arylsulfonyloxygruppe. Es ist darauf hinzuweisen, dass die in den Schemas 1-5 erläuterten Reaktionen selbstverständlich auch mit den entsprechenden racemischen Verbindungen durchgeführt werden können. In ähnlicher Weise können die

in den Schemas 6-9 erläuterten Reaktionen auch mit den optisch aktiven Verbindungen durchgeführt werden. Ueberdies ist darauf hinzuweisen, dass die nachfolgenden Schemas lediglich die Herstellung von Verbindungen der obigen Formel I erläutern, worin $R^{2'}$ Wasserstoff bedeutet; doch können die Isomeren davon, d.h. die Verbindungen der obigen Formel I, worin $R^2$ Wasserstoff bedeutet, in analoger Weise hergestellt werden; in Bezug auf die Schemas 4, 7, 8 und 9 können jedoch infolge der nahen Stellung der Seitenketten Probleme entstehen. Zu erwähnen wäre schliesslich noch, dass die Halohydrine durch Umsetzung von Epoxiden mit einer Halogenwasserstoffsäure erhalten werden können; die Halohydrine können durch Behandlung mit einer Base in die Epoxide übergeführt werden.

SCHEMA 1

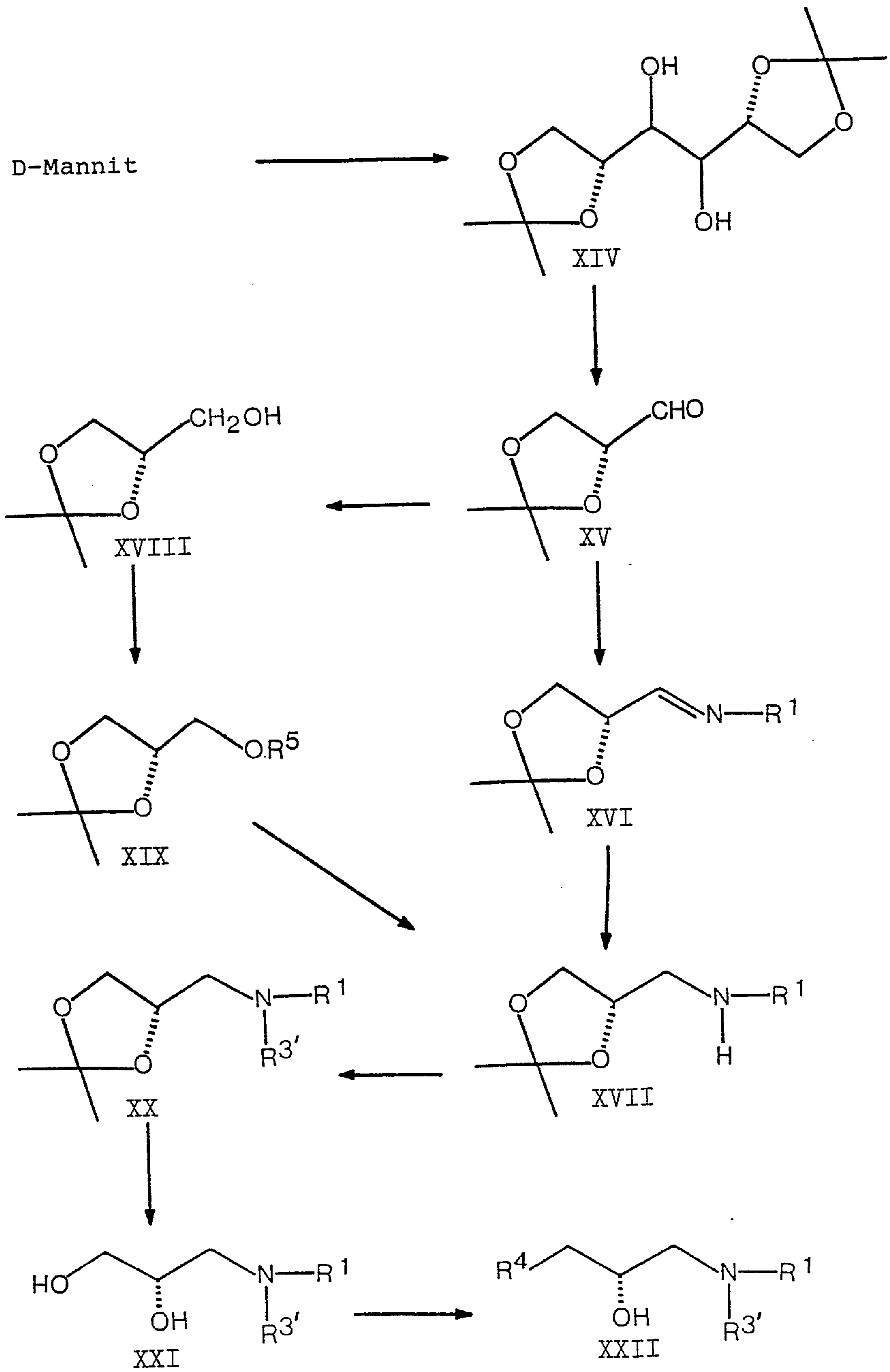

D-Mannit

XIV

XV

XVIII

XIX

XVI

XVII

XX

XXI

XXII

SCHEMA 2

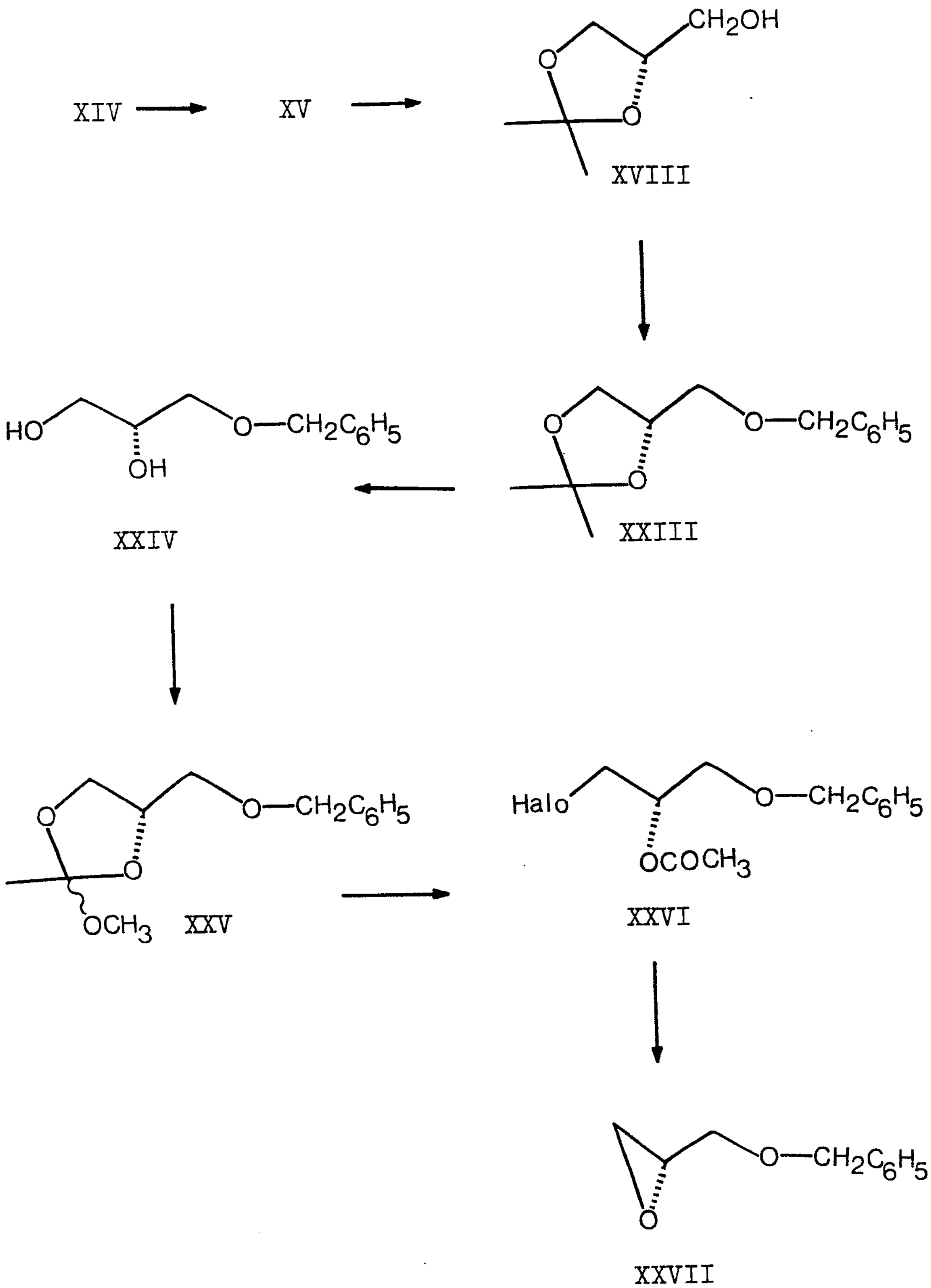

XIV XV XVIII

XXIV XXIII

XXV XXVI

XXVII

SCHEMA 3

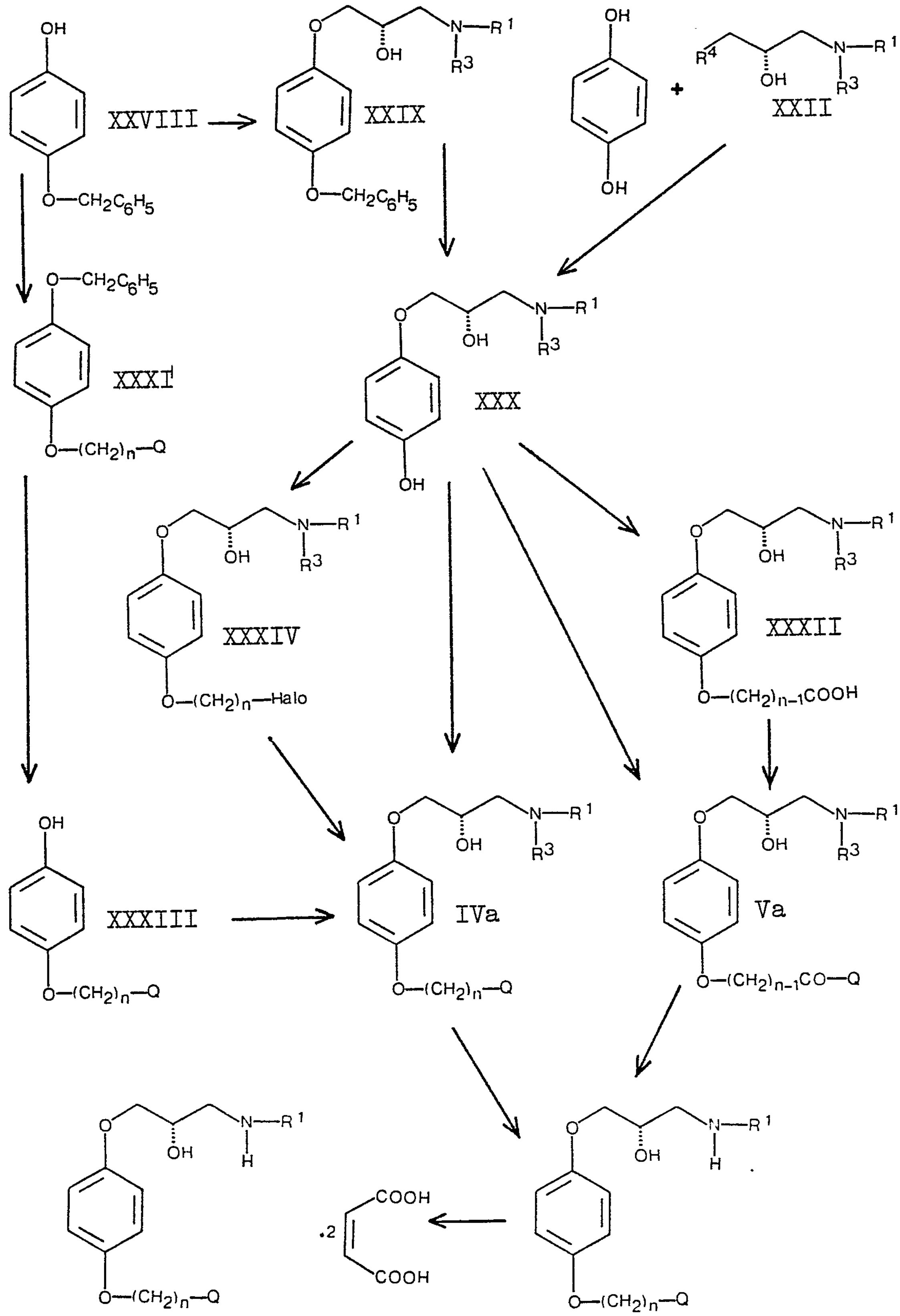

XXVIII

XXIX

XXII

XXXI'

XXX

XXXIV

XXXII

XXXIII

IVa

Va

Ia'

Ia

SCHEMA 4

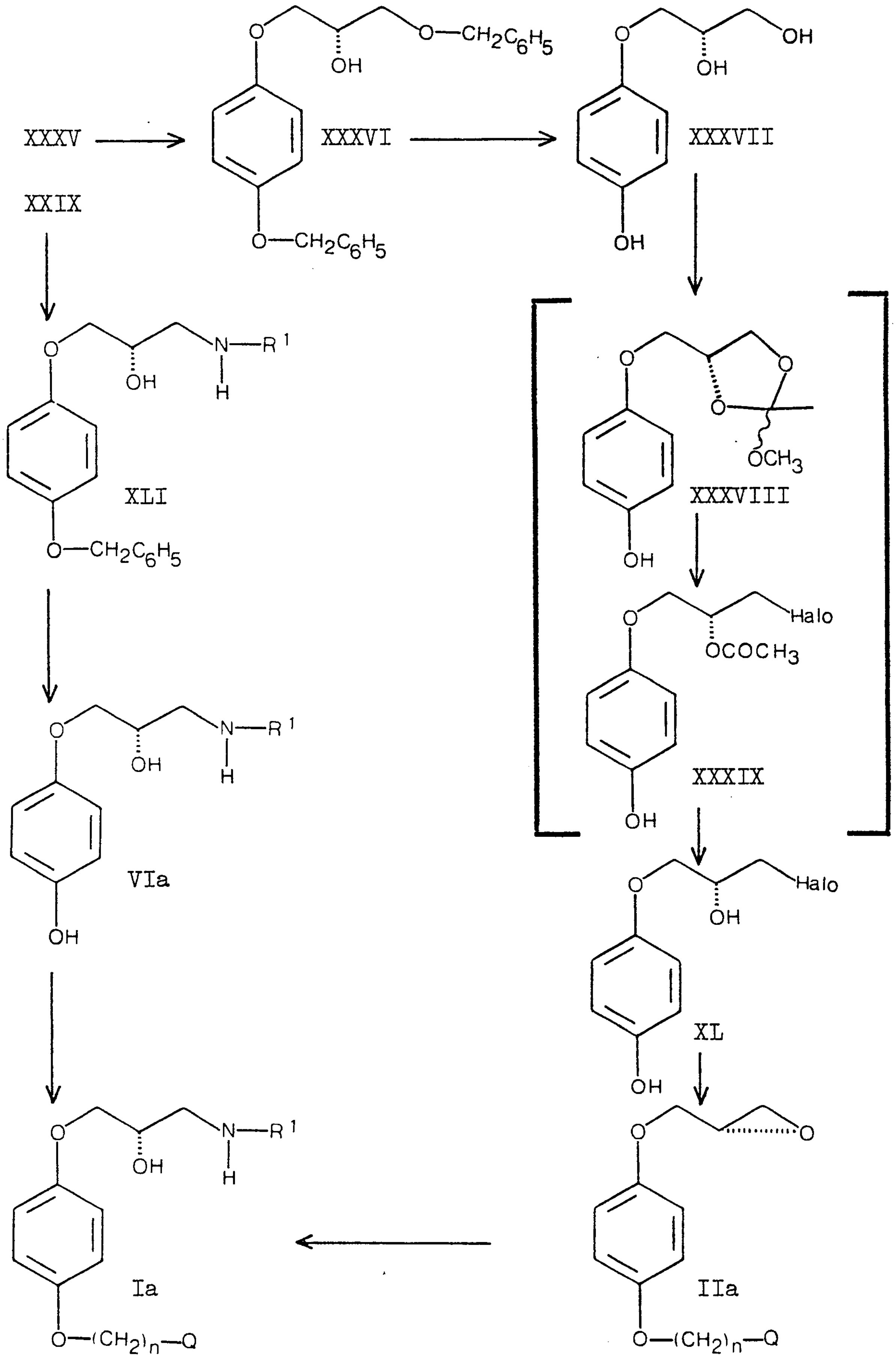

XXXV

XXIX

XXXVI

XXXVII

XLI

XXXVIII

XXXIX

VIa

XL

Ia

IIa

SCHEMA 5

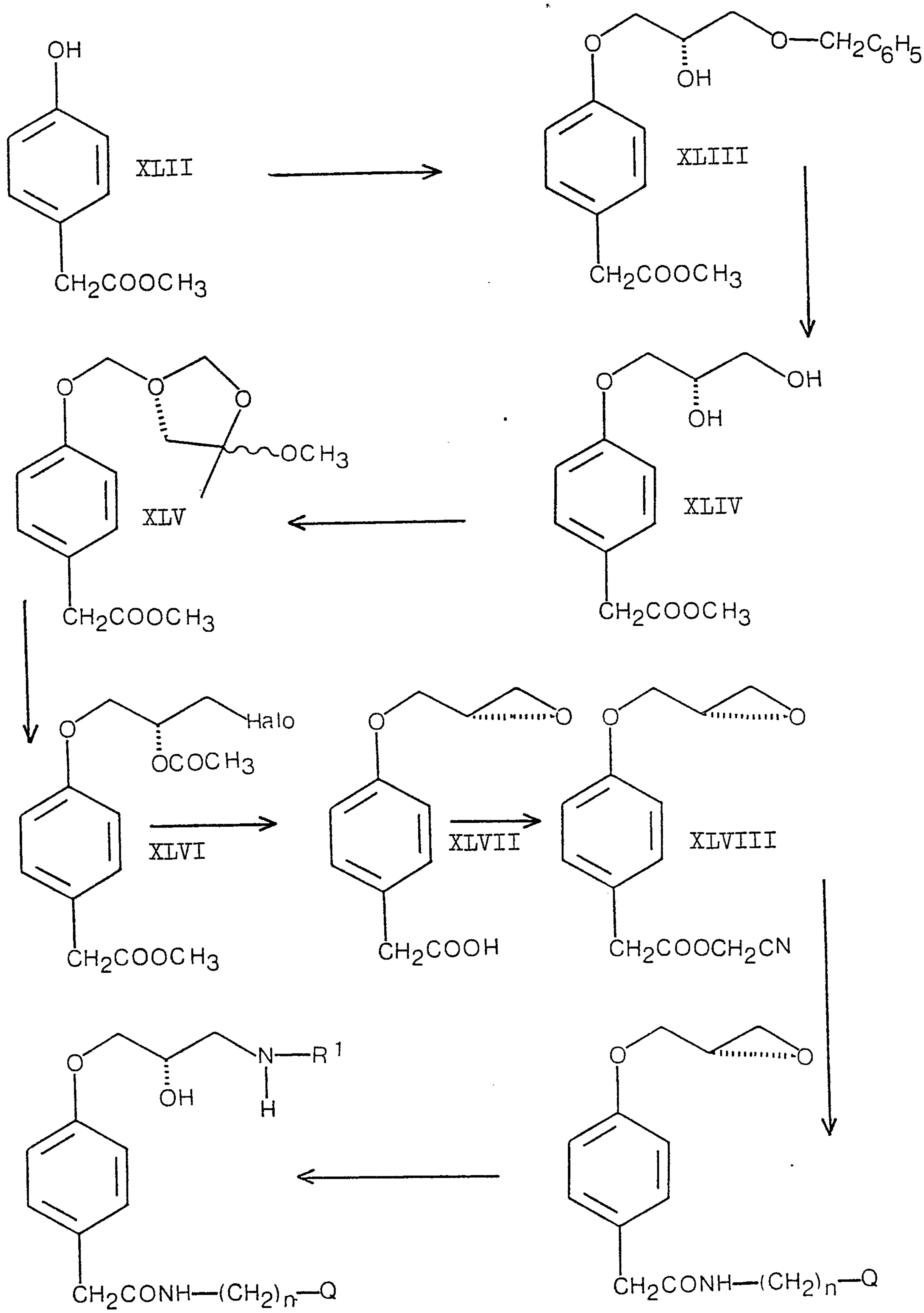

XLII

XLIII

XLIV

XLV

XLVI

XLVII

XLVIII

IIb

Ib

SCHEMA 6

(Racemische Serie )

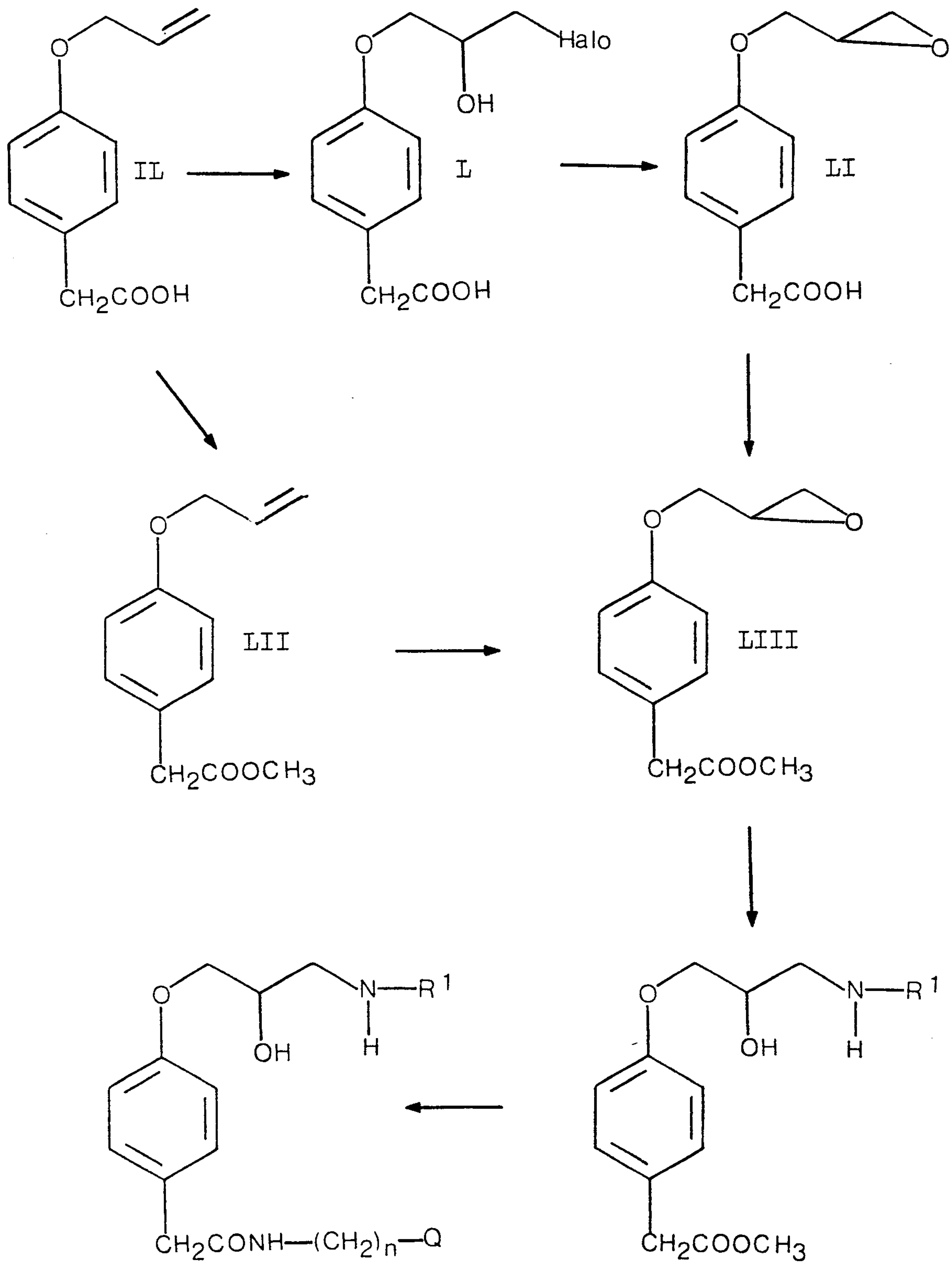

IL

L

LI

LII

LIII

Ic

VIIIa

SCHEMA 7

(Racemische Serie )

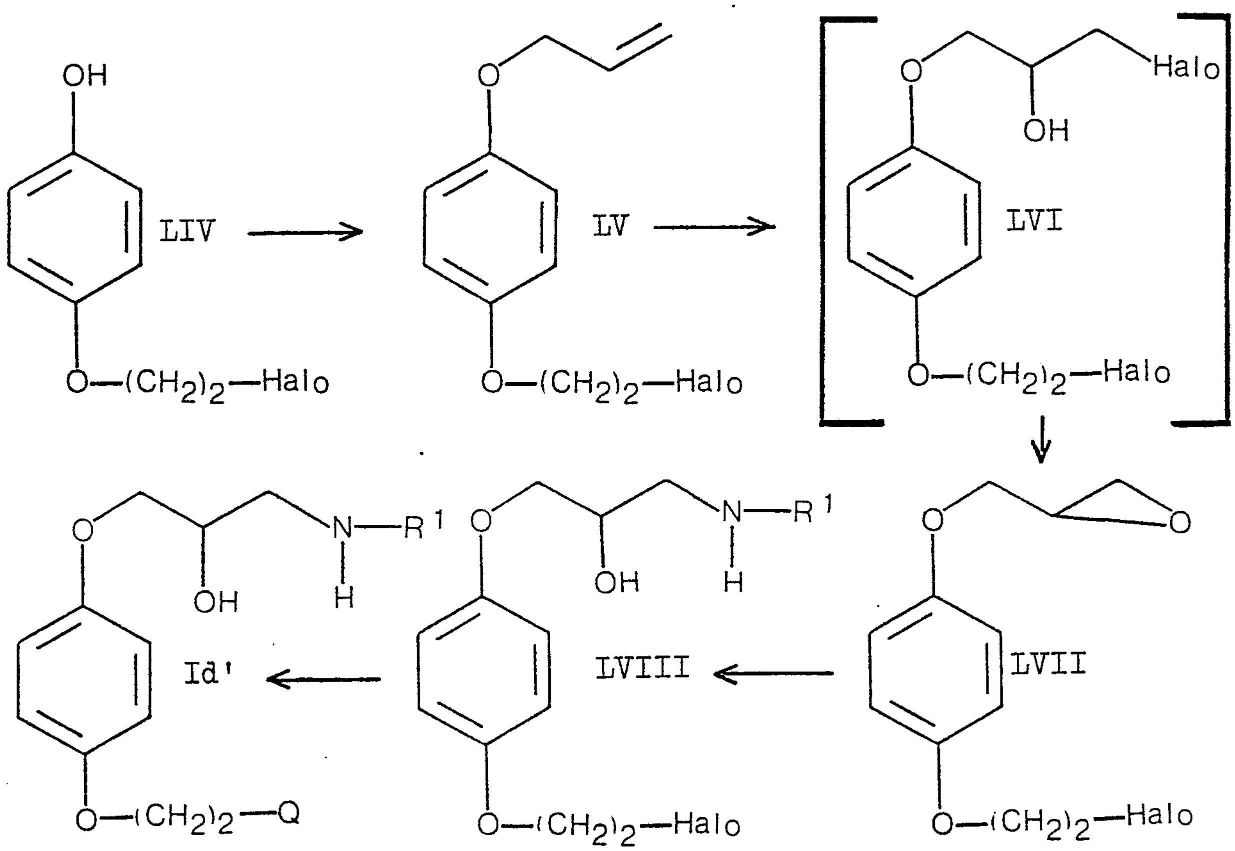

LIV LV LVI LVII LVIII Id'

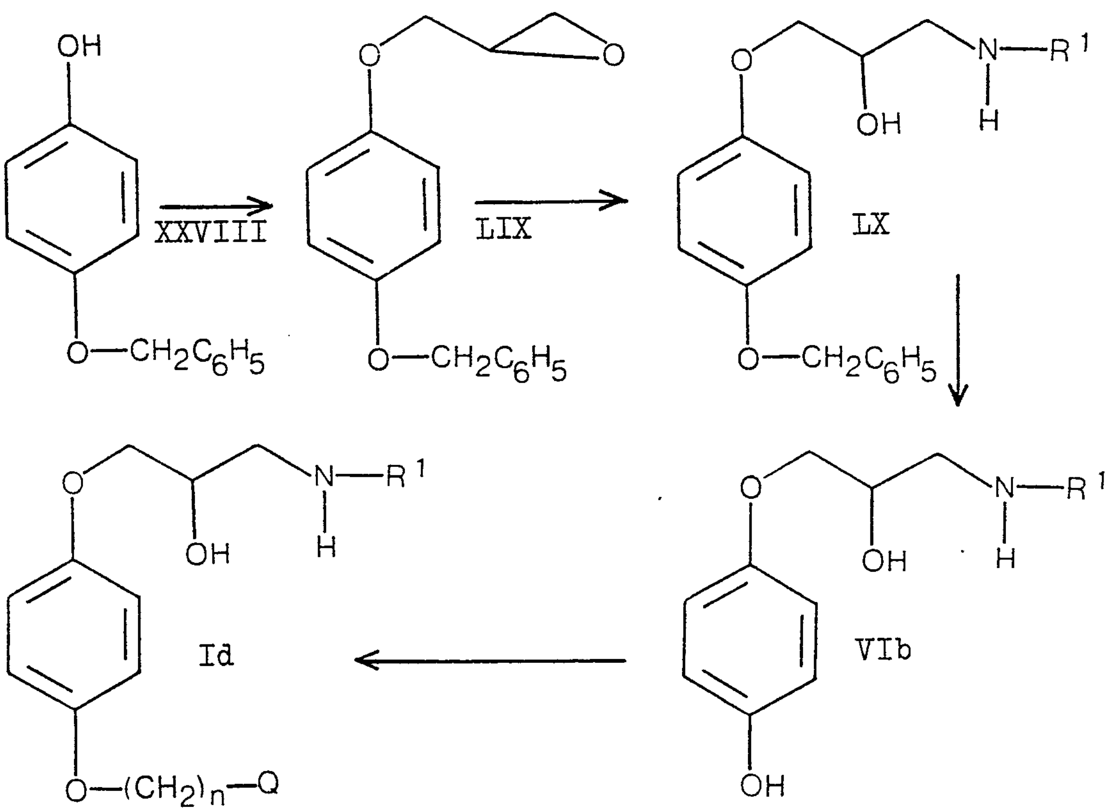

XXVIII LIX LX VIb Id

SCHEMA 8

(Racemische Serie )

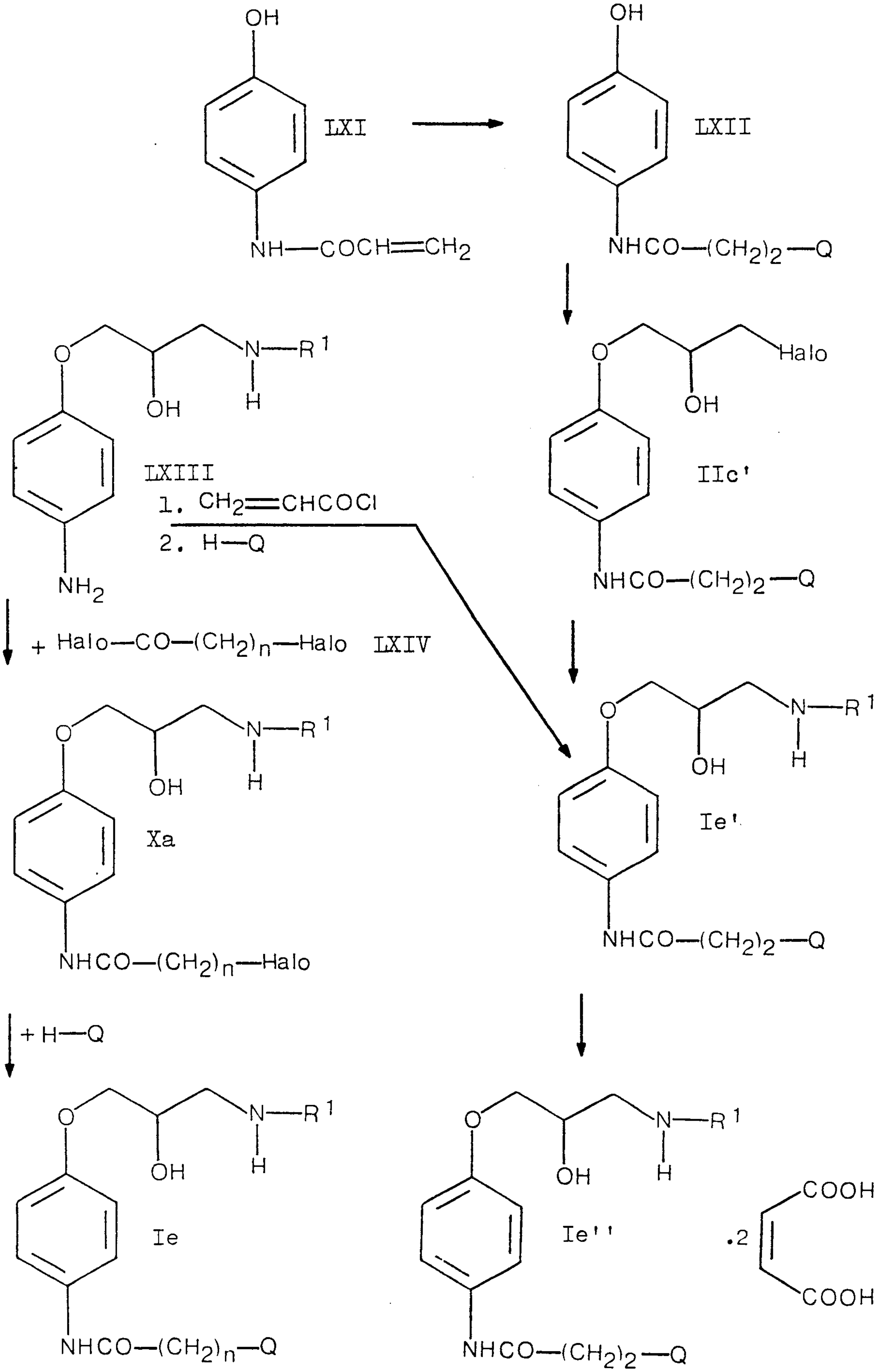


LXI, LXII, LXIII, IIc', Xa, Ie', Ie, Ie''

SCHEMA 9

(Racemische Serie )

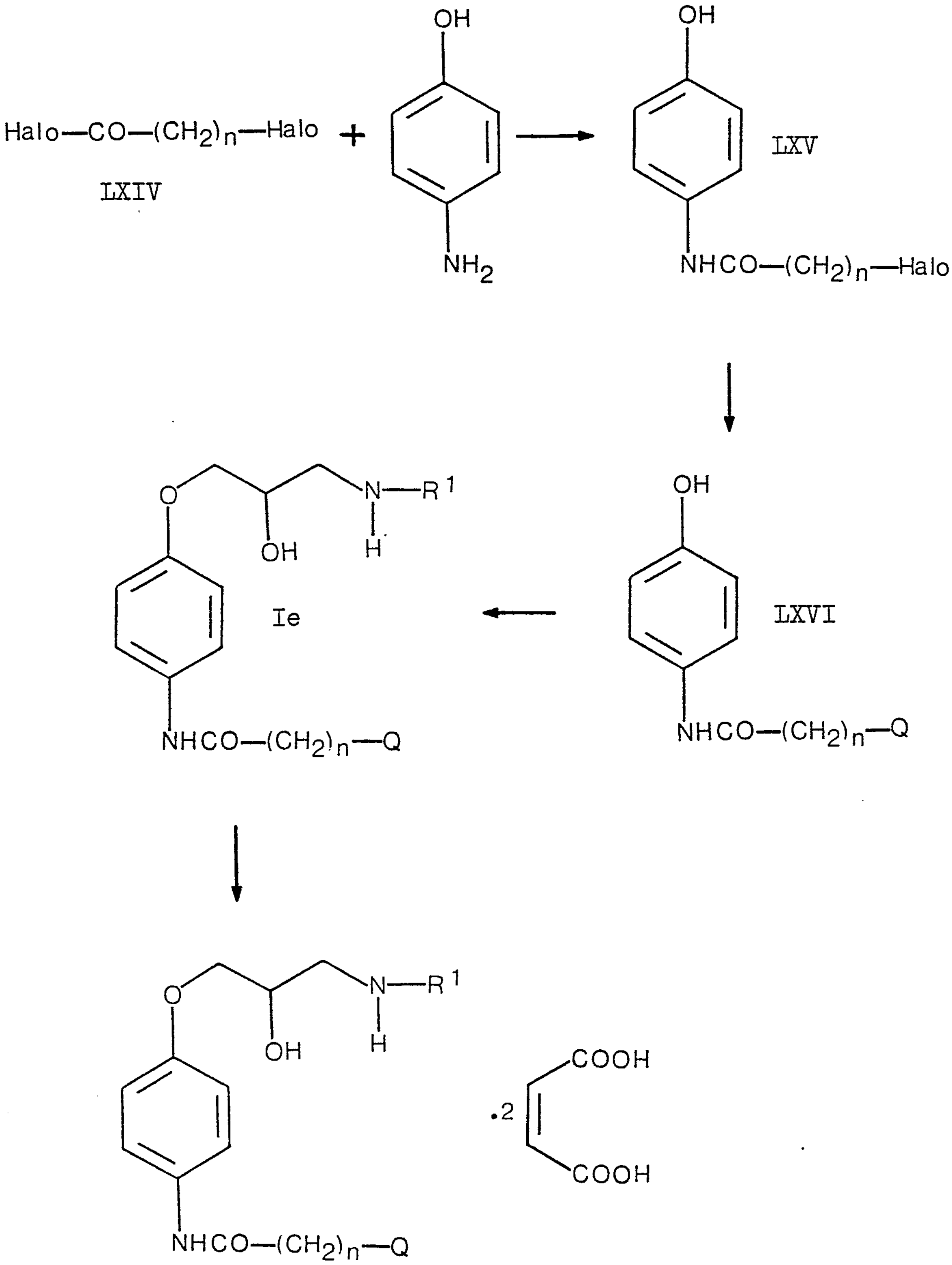

LXIV

LXV

LXVI

Ie

Ie'''

D-Mannit ⟶ XIV

Die Verbindung der Formel I, welche eine bekannte Verbindung ist, kann in einer säurekatalysierten Ketalaustauschreaktion hergestellt werden. Die Reaktion kann unter Verwendung einer starken Mineralsäure, wie Schwefelsäure oder p-Toluolsulfonsäure, oder eines Kationenaustauscherharzes ausgeführt werden. Die Reaktion kann in einem Temperaturbereich von etwa $0^{o}C$ bis $100^{o}C$ ausgeführt werden, vorzugsweise arbeitet man bei Raumtemperatur. Die Zeit bis zum Ende der Reaktion wird je nach der gewählten Reaktionstemperatur zwischen 1 und 16 Stunden schwanken.

XIV ⟶ XV ⟶ XVIII

Die Zweistufenreaktion zur Herstellung der Verbindung der Formel XVIII ist eine bekannte Reaktion, siehe z.B. J. Lecocq und C.E. Ballou, Biochemistry, 3, 976, (1964).

XVIII ⟶ XIX

Die Verbindung der Formel XIX kann durch Umsetzung des primären Alkohols XVIII mit einem Niederalkyl- oder Arylsulfonylhalogenid in Gegenwart eines tertiären Amins als Base hergestellt werden. Beispiele von Niederalkyl- oder Arylsulfonylhalogeniden, welche verwendet werden können, sind Mesyl-, Tosyl-, Brosyl- oder Benzylsulfonylchlorid oder -bromid. Beispiele von tertiären Aminbasen sind Pyridin oder Trialkylamine, z.B. Tri-n-butylamin oder Triäthylamin. Ein inertes Lösungsmittel, wie Methylenchlorid, Tetrahydrofuran oder Pyridin, kann zur Erleichterung der Reaktion verwendet werden. Das zuletzt genannte Lösungsmittel dient sowohl als Reaktionsteilnehmer als auch als Lösungsmittel. Die Reaktionstemperatur kann zwischen etwa $-25^{o}C$ und $15^{o}C$ schwanken, vorzugsweise arbeitet man bei einer Temperatur von $-10^{o}C$ bis $+15^{o}C$, wobei ein Bereich von $0^{o}C$ bis $15^{o}C$ optimal ist. Die Reaktionszeit kann

je nach der gewählten Reaktionstemperatur von einer halben bis zu einer Stunde reichen.

XIX ⟶ XVII

Die Verbindung der Formel XVII kann anschliessend durch Ersatz der Abgangsgruppe (die Alkyl- oder Arylsulfonyloxygruppe) durch ein primäres Amin, wie Methyl-, Aethyl-, Isopropyl- oder tert.Butylamin, hergestellt werden. Die Reaktion kann mit oder ohne inertes Lösungsmittel (Benzol, niedere Alkohole oder Aether können verwendet werden) bewerkstelligt werden. Im Falle von tiefsiedenden Aminen, beispielsweise Isopropylamin, sollte die Reaktion unter Druck ausgeführt werden. Die Reaktion findet im allgemeinen im Temperaturbereich von Raumtemperatur bis etwa 150°C statt, vorzugsweise arbeitet man bei etwa 100°C.

XIV ⟶ XV ⟶ XVI ⟶ XVII

Ein Vielstufenverfahren kann ebenfalls zur Herstellung einer Verbindung der Formel XVII verwendet werden; die Zwischenprodukte XV und XVI werden hierbei nicht isoliert. Die Verbindung der Formel XIV wird beispielsweise unter Verwendung von Bleitetraacetat in einem inerten aromatischen Kohlenwasserstoff als Lösungsmittel, wie Benzol, Toluol oder Xylol, zur Verbindung XV oxidiert. Die Reaktionstemperatur sollte bei Raumtemperatur oder darunter, beispielsweise 0°C, gehalten werden. Nachdem ein Nebenprodukt, d.h. Bleidiacetat, mittels Filtration entfernt worden ist, wird die in der Reaktion gebildete Essigsäure durch Zugabe eines Alkali- oder Erdalkalimetallkarbonates oder -oxides, beispielsweise Natrium- oder Kaliumcarbonat oder Bariumoxid, neutralisiert.

Der Aldehyd XV wird anschliessend zur Bildung des Imins XVI mit einem grossen Ueberschuss eines primären Amins, wie z.B. Methyl-, Aethyl-, Isopropylamin usw., umgesetzt. Die Reaktionstemperatur sollte bei etwa 25°C oder darunter liegen und es sollte ein Trocknungsmittel, wie z.B. Kaliumcarbonat, verwendet werden, um das während der Reaktion entstandene Wasser zu entfernen und die Reaktion dem Ende zuzuführen.

Anschliessend wird das Imin XVI unter Verwendung eines Katalysators, wie Edelmetalle, z.B. Platin, Palladium, Ruthenium u.s.w. auf Kohle oder Raney Nickel, unter Druck zum sekundären Amin XVII hydriert. Diese Reaktion kann bei einer Temperatur von 20°C-50°C ausgeführt werden; vorzugsweise arbeitet man bei Raumtemperatur. Die Reaktion kann je nach dem gewählten Katalysator bei 1-10 Atmosphären stattfinden.

XVII ⟶ XX

Das Amin XVII wird anschliessend mit einem Alkyl- oder Arylsulfonylhalogenid, wie Mesyl-, Tosyl-, Brosyl- oder Benzylsulfonylchlorid oder -bromid, in einem inerten aprotischen Lösungsmittel, wie hochsiedenden Aethern, beispielsweise Dioxan, Tetrahydrofuran, oder Methylenchlorid, in Gegenwart einer tertiären Aminbase, wie z.B. Triäthylamin oder Trimethylamin, umgesetzt. Die Reaktion kann in einem Temperaturbereich von etwa -50°C bis 25°C ausgeführt werden, vorzugsweise arbeitet man im Bereich von etwa -10°C bis 5°C.

XX ⟶ XXI

Die Verbindung der Formel XX wird anschliessend einer säurekatalysierten Hydrolyse der Ketalschutzgruppe unterworfen. Zur Bewerkstelligung dieser Katalyse werden starke Mineralsäuren, beispielsweise Chlorwasserstoff, Schwefelsäure oder p-Toluolsulfonsäure oder ein stark saures Ionenaustauscherharz ($H^+$-Form) verwendet. Das Lösungsmittel für die Reaktion kann Wasser und ein mischbares Lösungsmittel, wie

ein niederer Alkohol, beispielsweise Methanol, Aethanol, Propanol usw. und Aether, wie Tetrahydrofuran oder Dioxan, sein. Die Reaktionstemperatur liegt im Bereich von etwa Raumtemperatur bis 80°C, vorzugsweise arbeitet man im Bereich von etwa 60°C bis 80°C. Die Reaktionszeit kann je nach der gewählten Temperatur von einer Stunde bis zu 2 Tagen reichen.

<u>XXI ⟶ XXII</u>

Das Diol der Formel XXI wird anschliessend mit einem Alkyl- oder Arylsulfonylhalogenid (wie vorstehend in der Stufe XVIII ⟶ XIX beschrieben) in Gegenwart einer tertiären Aminbase (ebenfalls in XVIII ⟶ XIX beschrieben) umgesetzt, wobei die primäre Hydroxylgruppe selektiv in eine Alkyl- oder Arylsulfonyloxygruppe übergeführt wird. Wie bereits vorher in Stufe XVIII ⟶ XIX erwähnt, kann Pyridin als Base und Lösungsmittel dienen; es können aber auch andere vorstehend genannte Lösungsmittel verwendet werden. Der Temperaturbereich der Reaktion hängt davon ab, welche Sulfonyloxygruppe gewünscht wird; es kann im Temperaturbereich von etwa -45°C bis -50°C (für die Mesyloxygruppe) bis -5°C bis 5°C (für die Tosyloxygruppe) gearbeitet werden. Wenn $R^4$ in Formel XXII Halogen sein soll, wird sich die Reaktion von der oben beschriebenen unterscheiden. Die Verbindung der Formel XXI wird dann einer säurekatalysierten Austauschreaktion mit einem Trialkylorthoacetat, z.B. Trimethyl-, Triäthylorthoacetat usw.,unterworfen, wodurch das cyclische Orthoacetat der Formel

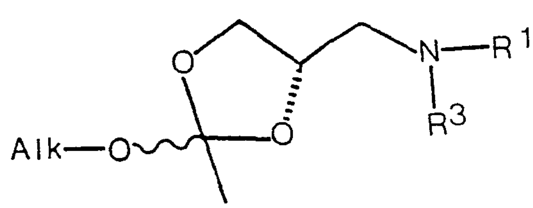

worin $R^1$ und $R^3$ die oben angegebene Bedeutung haben, und Alkanol entstehen. Um eine vollständige Reaktion zu ermöglichen, wird das entstandene Alkanol laufend aus der Reaktions-

mischung abdestilliert. Für diese Reaktion sind keine Lösungsmittel erforderlich. Man arbeitet im Temperaturbereich von etwa 60°C bis 100°C, wobei etwa 80°C bevorzugt sind. Die Reaktionszeit wird je nach Reaktionstemperatur zwischen 30 Minuten und einer Stunde liegen. Das cyclische Orthoacetat wird anschliessend mit Trimethylhalosilan in einem inerten, aprotischen Lösungsmittel, wie Methylenchlorid, umgesetzt, wodurch das Zwischenprodukt der Formel

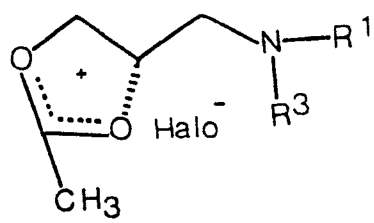

worin $R^1$ und $R^3$ die oben angegebene Bedeutung haben, entsteht; dieses Zwischenprodukt ergibt beim Angriff durch ein Halogenidion das Haloacetat. Als Lösungsmittel für diese Stufe eignen sich inerte, aprotische Lösungsmittel, wie hochsiedende Aether und halogenierte Kohlenwasserstoffe, beispielsweise Methylenchlorid, am besten. Die Reaktion kann in einem Temperaturbereich von etwa Raumtemperatur bis zur Rückflusstemperatur während etwa 30 Minuten bewerkstelligt werden. Man arbeitet vorzugsweise bei einer Reaktionstemperatur von etwa 40°C. Anschliessend wird das Haloacetat in einer säurekatalysierten Hydrolyse umgesetzt. Zur Ausführung der Reaktion wird eine Lösung des Substrates in einem zur Hydrolyse geeigneten Lösungsmittel, wie Alkohole, beispielsweise Methanol, Aethanol, Propanol usw. oder wässrige Alkoholmischungen, enthaltend eine katalytische Menge einer Mineralsäure, wie Salzsäure, Schwefelsäure oder ein saures Ionenaustauscherharz, verwendet. Die Reaktion kann bei einer Temperatur von 0°C bis zur Rückflusstemperatur (vom Lösungsmittel abhängig) während 30 Minuten bis 16 Stunden ausgeführt werden, vorzugsweise arbeitet man bei Raumtemperatur.

XIV ⟶ XV ⟶ XVIII

Dieses Verfahren wird wie vorstehend beschrieben mittels eines Verfahrens nach dem Stand der Technik, ausgeführt.

XVIII ⟶ XXIII

Die Hydroxylgruppe der Verbindung der Formel XVIII wird durch Herstellung der Verbindung der Formel XXIII geschützt, beispielsweise in Form ihres Benzyläthers. Die Reaktion besteht in der Umsetzung des Alkohols XVIII mit einem Alkalimetallhydrid, beispielsweise Natrium-, Lithium-, Kaliumhydrid; es entsteht das Alkoxid, welches anschliessend mit einem Alkylierungsmittel, beispielsweise einem Benzylhalogenid (Benzylchlorid oder -bromid) umgesetzt wird; man erhält den Benzyläther XXIII. Lösungsmittel, geeignet für eine solche Reaktion, sind z.B. wasserfreies Dimethylformamid, Dimethylsulfoxid und hochsiedende Aether, wie Tetrahydrofuran oder Dioxan. Die Reaktion kann im Temperaturbereich von etwa Raumtemperatur bis 100°C ausgeführt werden, vorzugsweise arbeitet man bei Raumtemperatur.

XXIII ⟶ XXIV

Die Verbindung der Formel XXIV kann anschliessend mittels einer säurekatalysierten Hydrolyse der Ketalschutzgruppe hergestellt werden. Die Reagenzien und Reaktionsparameter sind gleich wie in der vorstehend beschriebenen Stufe XX ⟶ XXI.

XXIV ⟶ XXV ⟶ XXVI

Das Diol der Formel XXIV kann via die Verbindung der Formel XXV und das Zwischenprodukt der Formel

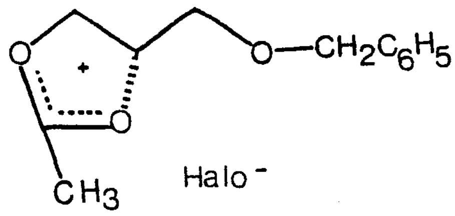

in das Haloacetat der Formel XXVI übergeführt werden, wobei man gemäss Stufe XXI → XXII arbeitet und die dort beschriebenen Reaktionsteilnehmer und -parameter verwendet. Diese Folge von Reaktionen wird ausgeführt, weil eine selektive Alkyl- oder Arylsulfonyloxylierung der primären Hydroxylgruppe in XXIV infolge der ähnlichen Reaktivität der beiden Hydroxylgruppen in XXIV schwierig ist.

XXVI → XXVII

Die Verbindung der Formel XXVI wird anschliessend einer Zweistufenreaktion unterworfen, worin das Acetat XXVI verseift wird,und man das Halohydrin -Zwischenprodukt der Formel

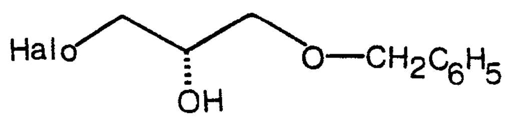

erhält, welches dann unter basischen Bedingungen in das Epoxid der Formel XXVII übergeführt wird. Die Reaktion wird unter Verwendung eines Alkalimetallhydroxides, beispielsweise Natrium- oder Kaliumhydroxid, in einer Lösungsmittelmischung, bestehend aus Wasser und einem inerten, mit Wasser mischbaren Lösungsmittel, wie einem niederen Alkohol, beispielsweise Methanol, Aethanol und Propanol usw. ausgeführt. Die Reaktionstemperatur kann zwischen etwa $-10^{o}C$ und $25^{o}C$ schwanken, vorzugsweise arbeitet man im Bereich von etwa $0^{o}C$ bis $10^{o}C$.Es ist darauf hinzuweisen, dass die obigen Reaktionen unter Erhaltung der Stereochemie des asymmetrischen Kohlenstoffatomes erfolgen.

XXVIII ⟶ XXIX

Diese Umsetzung ist ein Zweistufenverfahren, in welchem eine Verbindung der Formel XXII unter basischen Reaktionsbedingungen in das Epoxid der Formel

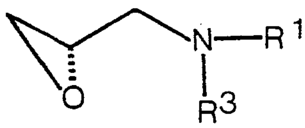

,

worin $R^1$ und $R^3$ die oben angegebene Bedeutung haben, übergeführt wird. Diese Verbindung wirkt als Alkylierungsmittel in der nachfolgenden Reaktion mit dem Phenol XXVIII unter Basenkatalysierten Bedingungen unter Bildung des Aethers XXIX. Die in der Reaktion verwendete Base ist ein Alkalimetallhydroxid, beispielsweise Natrium- oder Kaliumhydroxid; die Reaktionstemperatur liegt zwischen etwa Raumtemperatur und 100°C. Je nach der gewählten Reaktionstemperatur kann die Reaktionszeit zwischen etwa zwei Stunden und mehreren Tagen liegen. Die verwendeten Lösungsmittel können Dimethylsulfoxid, Tetrahydrofuran und niederer Alkohol/Wasser-Mischungen sein.

HYDROXYPHENOL ⟶ XXX

Diese Umsetzung ist ähnlich wie die Stufe XXVIII → XXIX, ausser dass ein grosser Ueberschuss an Hydroxyphenol verwendet wird, um möglicherweise auftretende Dialkylierung auf ein Minimum herabzusetzen. Wie in der Stufe XXVIII ⟶ XXIX ist das Epoxid das wirksame Alkylierungsmittel (siehe oben). Die Reagentien und Reaktionsparameter sind die gleichen wie oben (XXVIII ⟶ XXIX).

XXIX ⟶ XXX

Die Verbindung der Formel XXIX wird anschliessend einer Hydrolyse unter Bildung einer Verbindung der Formel XXX

unterworfen. Als Katalysatoren für diese Reaktion können Edelmetalle, wie Platin, Palladium, Rhodium oder Ruthenium auf Kohle dienen. Geeignete Lösungsmittel sind unter anderem niedere Alkohole, beispielsweise Methanol, Aethanol usw., Ester, beispielsweise Aethylacetat oder Butylacetat, sowie Aether, beispielsweise Dioxan oder Tetrahydrofuran usw. Die Reaktion kann bei einer Temperatur von etwa $0^{\circ}C$ bis $100^{\circ}C$ ausgeführt werden, vorzugsweise arbeitet man bei Raumtemperatur.

XXVIII ⟶ XXXI

Diese Reaktion besteht aus der O-Alkylierung des Phenols der Formel XXVIII mit einer Verbindung der Formel VII unter Verwendung eines Alkalimetallhydroxids (Natrium- oder Kaliumhydroxid) in einem inerten, mit Wasser mischbaren Lösungsmittel, beispielsweise einer Dimethylsulfoxid/Wasser-Mischung. Die Umsetzung kann zwischen etwa Raumtemperatur und etwa $60^{\circ}C$ erfolgen, wobei eine Reaktionstemperatur von $60^{\circ}C$ bevorzugt ist; die Reaktionszeit hängt von der Reaktionstemperatur ab und beträgt im allgemeinen 1 - 2 Stunden bis zu mehreren Tagen. Alternative O-Alkylierungssysteme, welche in Verbindung mit dem Phenylpiperazin verwendet werden können, sind unter anderem Alkalimetallalkoxide in niederen Alkoholen, beispielsweise Natriummethoxid in Methanol, oder Kaliumcarbonat in Aceton.

XXX ⟶ XXXIV

Die Verbindung der Formel XXX wird mit einer Verbindung der Formel

$$Y(CH_2)_nX$$

worin X und Y gleiche oder verschiedene Schutzgruppen bedeuten, wobei X die oben angegebene Bedeutung hat und Y aus den gleichen Schutzgruppen wie X ausgewählt ist, und n die oben angegebene Bedeutung hat,

umgesetzt.

Die Umsetzung, welche eine O-Alkylierung eines Phenols der Formel XXX mit beispielsweise einem α, ω-Dihaloalkan darstellt, wird unter Verwendung eines Alkalimetallcarbonates, wie Kalium- oder Natriumcarbonat,in vorzugsweise unter Rückfluss kochendem Aceton ausgeführt. Die Umsetzung kann zwischen Raumtemperatur und Rückflusstemperatur erfolgen, vorzugsweise arbeitet man bei Rückflusstemperatur.

<u>XXX ⟶ IVa</u>

Bei dieser Reaktion gelten die gleichen Reaktionsparameter und es werden auch die gleichen Reagentien verwendet, wie sie vorstehend in Stufe XXVIII ⟶ XXXI beschrieben worden sind.

<u>XXX ⟶ XXXII</u>

Die Verbindung der Formel XXX wird anschliessend mit einer Verbindung der Formel

$$X(CH_2)_{n-1}\overset{O}{C}-O-R$$

worin R Niederalkyl bedeutet und X und n die oben angegebene Bedeutung haben,

umgesetzt.

Diese O-Alkylierung des Phenols der Formel XXX mit beispielsweise einem Alkyl-ω-haloalkanoat, beispielsweise Aethyl-6-bromhexanoat oder Aethyl-bromacetat erfolgt unter Verwendung eines Alkalimetallalkoxides, wie Kalium-tert-butoxid, -methoxid oder - aethoxid, als Base in einem niederen Alkohol, wie z.B. Methanol, Aethanol usw. in einem Temperaturbereich von etwa 0°C bis 100°C, wobei man vorzugsweise in einem Temperaturintervall von 60°C bis 80°C arbeitet. Das Produkt wird anschliessend in bekannter Weise verseift und man erhält die Säure der Formel XXXII. Die Verseifung wird üblicherweise zwischen Raumtemperatur und 65°C während einer Zeit von 3 bis 40 Stunden bewerkstelligt.

XXX ⟶ Va

Die Verbindung der Formel XXX wird mit einem Alkylierungsmittel der Formel

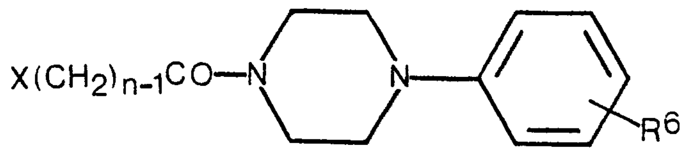

worin X, n und $R^6$ die oben angegebene Bedeutung haben, wie 1-(ω- Haloalkanoyl )-4-phenylpiperazin, in einer Alkohol/Wasser-Mischung oder Tetrahydrofuran oder Dimethylsulfoxid/Wasser-Mischung, enthaltend ein Alkalimetallhydroxid, z.B. Natrium- oder Kaliumhydroxid, umgesetzt. Die Reaktion wird zwischen Raumtemperatur und 100°C ausgeführt, wobei man vorzugsweise zwischen 75°C und 80°C arbeitet.

XXXI ⟶ XXXIII ⟶ IVa

Die Verbindung XXXI wird einer Hydrogenolyse des Benzyläther-Anteils unter Verwendung eines Edelmetallkatalysators, wie z.B. Palladium, Platin, Rhodium usw. auf Kohle unterworfen. Das Lösungsmittel für eine solche Reaktion kann ein Alkohol, beispielsweise Methanol, Aethanol usw. oder Essigsäure, enthaltend eine geringe Menge Mineralsäure, wie z.B. Chlorwasserstoff oder Schwefelsäure sein. Die Reaktion kann bei einer Temperatur von etwa 0°C bis 100°C stattfinden, wobei man vorzugsweise bei Raumtemperatur arbeitet. Die Verbindung der Formel XXXIII wird anschliessend einer O-Alkylierung unter Verwendung einer Verbindung der Formel XVI als Alkylierungsmittel in Gegenwart eines Alkalimetallhydroxides als Base unterworfen. Die Reagentien und Reaktionsparameter für diese Reaktion sind die gleichen, wie sie vorstehend für Stufe XXXVIII ⟶ XXIX beschrieben worden sind.

XXXII ⟶ Va

Die Säure der Formel XXXII wird durch Behandlung mit Aethylchlorformiat unter wasserfreien Bedingungen in einem aprotischen Lösungsmittel, beispielsweise Tetrahydrofuran oder Dioxan, bei einer tiefen Temperatur, beispielsweise etwa 0°C bis 5°C, in Gegenwart eines tertiären Amins, beispielsweise eines Trialkylamins, als Base in das aktivierte Zwischenprodukt der Formel

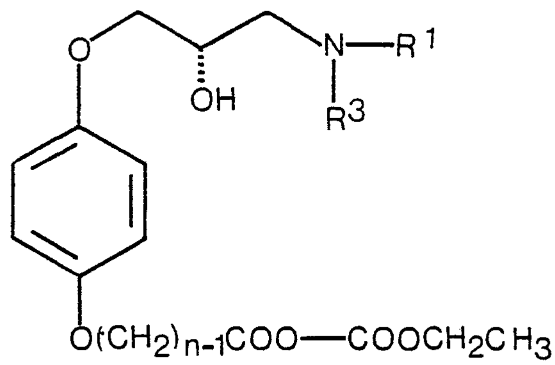

worin $R^1$, $R^3$ und n die oben angegebene Bedeutung haben, übergeführt. Dieses gemischte Anhydrid wird in situ mit einem Phenylpiperazin der allgemeinen Formel XI behandelt, wobei ein tertiäres Amid entsteht. Diese Reaktion wird bei einer Temperatur zwischen etwa 10°C und 25°C ausgeführt.

IVa ⟶ Ia

Die Verbindung der Formel IVa wird einer reduktiven Spaltung der $R^3$-Schutzgruppe unter Verwendung einer 60-70%igen Lösung von Natrium-bis-(methoxyäthoxy)-aluminiumhydrid in einem aromatischen Kohlenwasserstoff, wie Benzol oder Toluol, als inertem Lösungsmittel unterworfen. Inerte aprotische Lösungsmittel, wie Tetrahydrofuran oder Dioxan können ebenfalls verwendet werden. Die Umsetzung kann zwischen etwa Raumtemperatur und 100°C stattfinden, wobei man vorzugsweise in einem Bereich von etwa 80°C bis 100°C arbeitet.

Va ⟶ Ia

Die Verbindung der Formel Va wird einer reduktiven Spaltung der $R^3$-Schutzgruppe unterworfen, wobei gleichzeitig eine Reduktion der Amidfunktion zur Aminogruppe stattfindet. Die Reagentien und Reaktionsparameter für diese Umsetzung sind die gleichen wie in der vorher beschriebenen Stufe IVa ⟶ Ia, ausser dass eine proportional grössere Menge des Hydridreduktionsmittels verwendet wird.

XXXV ⟶ XXXVI

Das geeignet geschützte optisch aktive 2,3-Epoxypropanol XXXV wird anschliessend mit einem substituierten Phenol der Formel XXVIII in einer O-Alkylierung unter Verwendung eines Alkalimetallalkoxides, beispielsweise Natrium- oder Kaliummethoxid oder -äthoxid, als Base in einem niederen Alkohol, beispielsweise Methanol, Aethanol usw. als Lösungsmittel, umgesetzt. Brauchbar als Base in der obigen Reaktion wäre ebenfalls ein Alkalimetallhydroxid, beispielsweise Natrium- oder Kaliumhydroxid, in einem Lösungsmittel, wie z.B. wässrigem Alkohol, Tetrahydrofuran, Dioxan oder Dimethylsulfoxid. Die Reaktion kann im Bereich von Raumtemperatur bis zur Rückflusstemperatur des gewählten Lösungsmittels ausgeführt werden; vorzugsweise arbeitet man bei 60°C bis 80°C.

XXXVI ⟶ XXXVII

Die Benzyläther der Formel XXXVI werden anschliessend einer Hydrogenolyse unter Verwendung eines Edelmetallkatalysators, wie Palladium, Platin, Ruthenium usw. auf Kohle unterworfen. Geeignete Lösungsmittel sind unter anderem Alkohole, beispielsweise Methanol, Aethanol, Propanol usw. oder Essigsäure, enthaltend eine geringe Menge Mineralsäure, wie Schwefelsäure oder Chlorwasserstoff. Die Reaktion kann im Bereich von etwa 0°C bis 100°C ausgeführt werden, wobei man vorzugsweise bei Raumtemperatur arbeitet. Die

Hydrogenolyse kann ebenfalls unter einem Druck bis zu 10 Atmosphären erfolgen, falls ein Katalysator, wie Raney Nickel verwendet wird.

XXXVII ⟶ XXXVIII ⟶ XXXIX

Die Reagentien und Reaktionsparameter für diese Stufe sind bereits vorher in Stufe XXIV ⟶ XXV ⟶ XXVI beschrieben worden.

XXXIX ⟶ XL

Das Acetat XXXIX wird anschliessend einer säurekatalysierten Hydrolyse unterworfen, wobei die Umsetzung des Acetates in einem hierfür geeigneten Lösungsmittel, wie Alkohole , beispielsweise Methanol, Aethanol etc. oder Alkohol/Wasser-Mischungen, enthaltend eine katalytische Menge einer Mineralsäure, wie Schwefelsäure oder Chlorwasserstoff, oder eines sauren Ionenaustauscherharzes, erfolgt. Die Reaktion kann im Bereich von etwa 0°C bis zur Rückflusstemperatur des verwendeten Lösungsmittels stattfinden; vorzugsweise arbeitet man im Bereich von etwa Raumtemperatur bis 60°C.

XL ⟶ IIa

Das Halohydrin XL wird anschliessend mit einer Verbindung der Formel VII umgesetzt.

Diese Reaktion enthält zwei getrennte und nicht miteinander in Beziehung stehende, durch Baseninduzierte Umwandlungen, d.h. (1) die Ueberführung des Halohydrins in ein Epoxid und (2)die O-Alkylierung des Phenols mit dem ausgewählten Phenylpiperazin. Die Reaktion erfolgt unter basischen Bedingungen unter Verwendung eines Alkalimetallhydroxides, beispielsweise Natrium- oder Kaliumhydroxid,in einem wässrigen Lösungsmittel, wie Dimethylsulfoxid, Tetrahydrofuran oder Alkohol, z.B. Methanol, Aethanol usw. Die Umsetzung

tion wird im Bereich von etwa 0°C bis etwa 40°C ausgeführt; vorzugsweise arbeitet man bei höheren Temperaturen, beispielsweise bei etwa 40°C.

IIa → Ia

Das Epoxid der Formel IIa wird mit einem Monoalkylamin, wie Isopropylamin, tert.Butylamin usw., zur Herstellung des Aminoalkohols umgesetzt. Lösungsmittel für eine solche Umsetzung sind $C_1$-$C_4$-Alkohole, vorzugsweise Methanol, oder Aether, wie Tetrahydrofuran oder Dioxan. Die Umsetzung kann im Bereich von etwa 0°C bis 100°C erfolgen, vorzugsweise arbeitet man bei etwa 65°C.

XXIX → XLI

Die Verbindung der Formel XXIX wird einer reduktiven Spaltung des N-geschützten Amins zur Erzeugung des sekundären Amins unterworfen. Die Reagentien und Reaktionsparameter für diese Umsetzung sind vorstehend für Stufe IVa → Ia beschrieben werden.

XLI → VIa

Der Benzyläther der Formel XLI wird anschliessend einer katalysierten Hydrogenolyse unterworfen. Geeignete Katalysatoren sind unter anderem Edelmetalle, (wie vorstehend offenbart) auf Kohle in einem Lösungsmittel, wie niederen Alkoholen ($C_1$ bis $C_4$), z.B. Methanol oder Aethanol, in einem Temperaturbereich von etwa 0°C bis 100°C. Falls Raney Nickel als Katalysator verwendet wird, sollte die Umsetzung unter Druck, beispielsweise bis zu 10 Atmosphären, stattfinden.

VIa → Ia

Das Phenol VIa wird einer Basen-induzierten O-Alkylierung mit einer Verbindung der Formel VII in wässrigem

Dimethylsulfoxid, Tetrahydrofuran oder Dioxan als Lösungsmittel unterworfen. Geeignete Basen sind unter anderem Alkalimetallhydroxide, wie Natrium- oder Kaliumhydroxid. Die Reaktion kann im Bereich von etwa 0°C bis 100°C stattfinden, vorzugsweise arbeitet man bei etwa 60°C.

<u>XLII ⟶ XLIII</u>

Die Verbindung der Formel XLII wird mit einer Verbindung der Formel XXVII zur Herstellung der Verbindung der Formel XLIII umgesetzt. Die Reaktionsteilnehmer und Reaktionsparameter hiefür sind vorstehend in Stufe XXXV ⟶ XXXVI beschrieben worden.

<u>XLIII ⟶ XLIV</u>

Die Verbindung der Formel XLIII wird anschliessend einer Hydrogenolyse unterworfen, wobei die gleichen Reagentien und Reaktionsparameter wie in der vorher beschriebenen Stufe XXXVI ⟶ XXXVII verwendet werden.

<u>XLIV ⟶ XLV ⟶ XLVI</u>

Die Verbindung der Formel XLIV wird unter Verwendung der gleichen Reagentien und Reaktionsparameter, wie vorstehend in Stufe XXIV ⟶ XXV ⟶ XXVI beschrieben, in eine Verbindung der Formel XLVI übergeführt.

<u>XLVI ⟶ XLVII</u>

Die Verbindung der Formel XLVI wird wie folgt einer dreistufigen Reaktion unter basischen Bedingungen unterworfen:

(A) Verseifung des Methylesters
(B) Verseifung des Acetats zur Erzeugung des Halohydrins und
(C) Umwandlung des Halohydrin- -Zwischenproduktes

in das Epoxid der Formel XLVII.

Geeignete Basen sind unter anderem Alkalimetallhydroxide, z.B. Natrium- oder Kaliumhydroxid, in einem Lösungsmittel, bestehend aus Wasser und einem inerten, mit Wasser mischbaren Lösungsmittel, wie einem niederen Alkohol ($C_1$ bis $C_4$), z.B. Methanol, oder einem Aether, z.B. Tetrahydrofuran oder Dioxan usw. Die Umsetzung wird im Bereich von etwa 0°C bis 30°C ausgeführt, wobei man vorzugsweise zwischen etwa 10°C und 25°C arbeitet.

XLVII → XLVIII

Der aktivierte Ester der Formel XLVIII kann durch Behandlung einer Verbindung der Formel XLVII mit einem Ueberschuss eines Haloacetonitrils in Gegenwart einer tertiären Aminbase, wie einem Trialkylamin, z.B. Triäthylamin oder Trimethylamin, hergestellt werden. Die Reaktion kann im Bereich von etwa 0°C bis 70°C erfolgen; vorzugsweise arbeitet man im Bereich von etwa 25°C bis 70°C. Der aktivierte Ester kann Kondensationsreaktionen unterworfen werden, beispielsweise Reaktionen mit Aminen zur Bildung von Amiden, wobei wesentlich höhere Geschwindigkeiten als mit gewöhnlichem Methyl- oder Aethylester erzielbar sind.

XLVIII → IIb

Der Ester der Formel XLVIII wird anschliessend mit einem primären Amin der Formel IX unter Bildung des Amides IIb in einem inerten Lösungsmittel, wie Tetrahydrofuran oder Dioxan kondensiert. Ein Temperaturbereich von etwa 0°C bis 100°C kann verwendet werden; vorzugsweise arbeitet man bei Raumtemperatur.

IIb → Ib

Das Epoxid der Formel IIb wird anschliessend mit einem primären Amin, d.h. einem Amin der Formel III, in einem

Lösungsmittel, wie einem ($C_1$ bis $C_4$)-Alkohol,oder Aether, z.B. Tetrahydrofuran oder Dioxan, unter Bildung des Aminoalkoholes Ib umgesetzt. Die Umsetzung kann im Bereich von etwa $0^oC$ bis zur Rückflusstemperatur erfolgen, wobei man vorzugsweise im Bereich von Raumtemperatur bis $65^oC$ arbeitet.

IL ⟶ L

Die Verbindung der Formel IL* wird mit einer hypohalogenigen Säure (in situ gebildet aus einem N-Halogensuccinimid in wässrigem Aceton, enthaltend eine katalytische Menge Perchlorsäure) umgesetzt und man erhält eine Mischung von Halohydrinen, d.h. die Verbindung der Formel L und die isomere Verbindung der Formel.

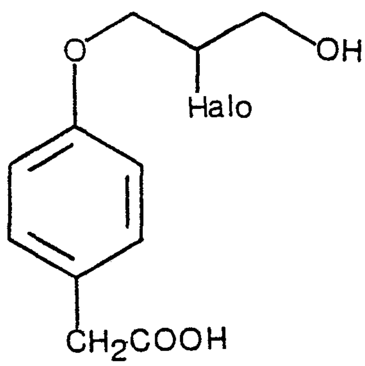

L'

L und L' ⟶ LI

Die Halohydrine werden anschliessend unter basischen Bedingungen in das Epoxid LI überführt. Als Base kann ein Alkalimetallhydroxid, z.B. Natrium- oder Kaliumhydroxid, verwendet werden. Geeignete Lösungsmittel für diese Reaktion sind unter anderem Aether, wie Dioxan oder Tetrahydrofuran, und Alkohole ($C_1$ bis $C_4$), z.B. Methanol, Aethanol usw. Die Umsetzung kann im Bereich von etwa $0^oC$ bis $40^oC$

* J.M. van der Zanden & G. de Vrier, Rec. Trav. Chim., 71, S. 879 (1952).

stattfinden, wobei man vorzugsweise bei Raumtemperatur arbeitet.

IL ⟶ LII

Die Säurefunktion der Verbindung der Formel IL wird durch Behandlung mit einem Ueberschuss an Alkylierungsmittel, wie einem Methylhalogenid, z.B. Methyljodid oder Methylbromid, in Gegenwart eines Alkalimetallcarbonates, z.B. Natrium- oder Kaliumcarbonat, in einem Lösungsmittel, wie Aceton, Dimethylformamid, Dimethylsulfoxid oder Hexamethylphosphoramid usw. verestert und man erhält den Ester der Formel LII. Die Reaktionstemperatur ist nicht kritisch, der Einfachheit halber ist jedoch Raumtemperatur bevorzugt.

LI ⟶ LIII

Die Säurefunktion des Epoxides LI kann unter Verwendung von Diazomethan in Gegenwart eines Lösungsmittels, wie Aether, z.B. Tetrahydrofuran, oder eines ($C_1$ bis $C_4$)-Alkohols ebenfalls verestert werden. Wie oben, wird diese Reaktion vorzugsweise bei Raumtemperatur durchgeführt.

LII ⟶ LIII

Die Verbindung der Formel LII wird anschliessend mit einer aromatischen oder aliphatischen Persäure, wie m-Chlorperbenzoesäure, Peressigsäure, Perameisensäure, Trifluorperessigsäure, Permaleinsäure, Perbenzoesäure, Monoperphthalsäure, o-Sulfoperbenzoesäure oder p-Nitroperbenzoesäure ,umgesetzt. In dieser Reaktion wird als Lösungsmittel ein inerter, halogenierter aliphatischer Kohlenwasserstoff, wie Methylenchlorid oder Chloroform verwendet. Die Reaktion kann im Bereich von etwa $0^oC$ bis zur Rückflusstemperatur erfolgen, wobei man vorzugsweise bei Raumtemperatur arbeitet.

LIII ⟶ VIIa

Die Epoxidfunktion der Verbindung der Formel LIII wird anschliessend mit einem primären Amin der Formel III in einem geeigneten Lösungsmittel, wie ($C_1$ bis $C_4$)-Alkohole oder Aether, wie Dioxan oder Tetrahydrofuran, umgesetzt. Die Reaktion kann im Bereich von etwa 0°C bis Raumtemperatur stattfinden, wobei man vorzugsweise bei Raumtemperatur arbeitet.

VIIIa ⟶ Ic

Die Esterfunktion der Verbindung der Formel VIIIa wird anschliessend mit einem primären Amin der Formel IX unter Bildung des Amides kondensiert. In dieser Stufe ist kein Lösungsmittel erforderlich. Die Reaktion kann im Bereich von etwa 100°C bis 150°C erfolgen, wobei man vorzugsweise im Bereich von etwa 140°C bis 145°C arbeitet.

XXVIII ⟶ LIX

Die Phenolfunktion der Verbindung der Formel XXVIII wird einer O-Alkylierung mit einem Epihalohydrin, z.B. Epichlorhydrin, unter Verwendung eines Alkalimetallhydroxides, z.B. Kalium- oder Natriumhydroxid, als Base in einer Mischung von Wasser und Dioxan, Tetrahydrofuran oder Dimethylsulfoxid, unterworfen. Die Reaktion kann im Bereich von etwa 0°C bis 100°C erfolgen; vorzugsweise arbeitet man bei etwa Raumtemperatur.

LIX ⟶ LX

Die Epoxidfunktion der Verbindung der Formel LIX wird anschliessend mit einem Amin der Formel III in einem Lösungsmittel, wie einem ($C_1$ bis $C_4$)-Alkohol oder einem Aether, wie Tetrahydrofuran oder Dioxan, umgesetzt. Die Reaktion kann im Bereich von etwa 0°C bis zur Rückflusstemperatur stattfinden, wobei man vorzugsweise im Bereich

von etwa Raumtemperatur bis 65°C arbeitet.

LX ⟶ VIb

Die Benzyloxyfunktion der Verbindung der Formel LX wird unter Verwendung der vorstehend für das Isomere beschriebenen (siehe Stufe XLI ⟶ VIa) Reagentien und Reaktionsparameter in die Phenolfunktion übergeführt.

VIb ⟶ Id

Die Phenolfunktion der Verbindung der Formel VIb wird mit einem Phenylpiperazin unter Verwendung der vorstehend in Stufe VIa ⟶ Ia beschriebenen Reagentien und Reaktionsparameter umgesetzt.

LIV* ⟶ LV

Die Phenolfunktion der Verbindung der Formel LIV wird einer O-Alkylierung mit einem Alkylierungsmittel, wie einem Allylhalogenid, z.B. Allylchlorid oder-bromid, in Gegenwart eines Alkalimetallcarbonates (Natrium- oder Kaliumcarbonat) in einem unter Rückfluss siedenden Lösungsmittel, wie Aceton, unterworfen.

LV ⟶ LVI ⟶ LVI'

Die Verbindung der Formel LV wird anschliessend unter Verwendung der in Stufe IL ⟶ L angegebenen Reagentien und Reaktionsparameter in die Halohydrine der Formeln LVI und LVI' übergeführt.

LVI und LVI' ⟶ LVII

Die Halohydrine werden anschliessend unter Verwen-

* F.S.H. Head, J. Chem. Soc (C), 871 (1971)

dung der in Stufe L ⟶ LI angegebenen Reagentien und Reaktionsparamtern in das Epoxid der Formel LVIII übergeführt.

LVII ⟶ LVIII

Das Epoxid der Formel LVII wird anschliessend mit einem primären Amin der Formel III unter Bildung des Aminoalkohols der Formel LVIII umgesetzt. Geeignete Lösungsmittel sind unter anderem ($C_1$ bis $C_4$)Alkohole, Aether, wie Tetrahydrofuran, oder Dimethylsulfoxid oder Dimethylformamid. Die Reaktion kann im Bereich von etwa Raumtemperatur bis 60°C stattfinden; vorzugsweise arbeitet man im Bereich von etwa Raumtemperatur bis 55°C.

LVIII ⟶ Id'

Das Halogenatom der Verbindung der Formel LVIII wird anschliessend durch ein Phenylpiperazin der Formel XI unter Bildung einer Verbindung der Formel Id' ersetzt. Geeignete Lösungsmittel für diese Reaktion sind unter anderem ($C_1$ bis $C_4$)-Alkohole und Aether, wie Dioxan und Tetrahydrofuran. Die Reaktion kann im Bereich von etwa 0°C bis 100°C stattfinden, wobei man vorzugsweise zwischen 80°C und 100°C arbeitet.

LXI* ⟶ LXII

Eine Verbindung der Formel LXI wird mit einem Phenylpiperazin der Formel XI in einem ($C_1$ bis $C_4$)-Alkohol umgesetzt. Die Umsetzung kann im Bereich von etwa Raumtemperatur bis zum Siedepunkt des verwendeten Alkohols stattfinden. Ein bevorzugter Alkohol ist Aethanol und man arbeitet vorzugsweise bei dessen Siedepunkt.

* T. Kunitake & S. Shinkai. J. Amer. Chem. Soc., 93, 4247 (1971)

LXII ⟶ IIc'

Die Verbindung der Formel LXII wird anschliessend durch Behandlung mit einer Base, wie einem Alkalimetall- oder Erdalkalimetallhydroxid (Natrium-, Kalium- oder Bariumhydroxid), in einem Lösungsmittel, wie Wasser, ($C_1$ bis $C_4$)-Alkoholenoder Dimethylformamid, oder einem Alkalimetallsalz von niederen Alkoholen in Lösungsmitteln, wie Dimethylformamid oder ($C_1$ bis $C_4$)-Alkoholen in eine Verbindung der Formel IIc' übergeführt werden. Zu dieser Reaktionsmischung, enthaltend das Salz der Verbindung der Formel LXII, wird das Epihalohydrin zugesetzt, wodurch das der Verbindung der Formel IIc' entsprechende Epoxid gebildet wird. Dieses Epoxid liefert bei der Behandlung mit einem Halogenwasserstoff die Verbindung der Formel IIc'.

IIc' ⟶ Ie'

Die Verbindung der Formel IIc' wird mit einem Amin der Formel III in einem geeigneten Lösungsmittel, z.B. ($C_1$ bis $C_4$)-Alkoholen behandelt. Die Umsetzung kann in einem Bereich von etwa Raumtemperatur bis zur Rückflusstemperatur ausgeführt werden, wobei man vorzugsweise bei Rückflusstemperatur arbeitet. Ein bevorzugtes Lösungsmittel ist Methanol. Falls höhere Reaktionsgeschwindigkeiten erwünscht sind, kann das Gemisch in einem Druckkessel über dessen Siedepunkt hinaus erhitzt werden.

LXIII ⟶ Ie' ⟶ Ie"

Die Verbindung der Formel Ie' kann alternativ aus der Verbindung der Formel LXIII durch Behandlung mit einem Propenoylhalogenid in einem inerten Lösungsmittel, z.B. Dioxan, Dimethylsulfoxid etc., bei einer Temperatur von etwa 0°C bis 50°C hergestellt werden. Das Zwischenprodukt wird in situ mit einem Phenylpiperazin der Formel XI behandelt. Die Reaktionsbedingungen sind gleich wie in der obigen Stufe LXI ⟶ LXII. Die Verbindung der Formel Ie" wird

durch Vermischen von Maleinsäure mit der Verbindung Ie' in einem inerten Lösungsmittel gebildet.

LXIII ⟶ Xa ⟶ Ie

Das Haloalkanoylhalogenid der Formel LXIV wird mit der Verbindung LXIII in Gegenwart eines tertiären Amins, wie Pyridin, Triäthylamin usw., in einem inerten Lösungsmittel, wie Aether, z.B. Tetrahydrofuran oder Dioxan, chloriertem Kohlenwasserstoffen, z.B. Methylenchlorid, bei einer Temperatur zwischen etwa $0^{o}C$ und $100^{o}C$, vorzugsweise $50^{o}C$, umgesetzt.

LXV ⟶ LXVI

Das Haloalkanoylhalogenid der Formel LXIV wird mit 4-Aminophenol in Gegenwart eines Ueberschusses an 4-Aminophenol oder eines Aequivalentes an tertiärem Amin, wie Pyridin, Triäthylamin usw., in einem inerten Lösungsmittel, wie einem Aether, z.B. Tetrahydrofuran oder Dioxan, oder einem polyhalogenierten Kohlenwasserstoff, z.B. Methylenchlorid, bei einer Temperatur von etwa $0^{o}C$ bis $100^{o}C$ umgesetzt. Ein bevorzugtes Reaktionssystem für diese Umwandlung ist Dioxan in Gegenwart eines Ueberschusses an 4-Aminophenol bei Raumtemperatur.

LXV ⟶ LXVI

Die Verbindung der Formel LXV wird mit einem Phenylpiperazin der Formel XI in Gegenwart eines Halogenwasserstoffbindenden Mittels, wie einem Ueberschuss an Phenylpiperazin oder einem weniger reaktiven Amin, wie Triäthylamin oder Pyridin, in einem inerten Lösungsmittel, z.B. einem ($C_1$ bis $C_4$)-Alkohol, umgesetzt. Die Reaktion kann im Bereich von etwa Raumtemperatur bis etwa $100^{o}C$ stattfinden.

LXVI ⟶ Ie ⟶ Ie'''

Die Umwandlung der Verbindung der Formel LXVI in diejenige der Formel Ie''' ist in Bezug auf die Reagentien und Reaktionsparameter ähnlich, wie oben in den Stufen LXII ⟶ IIc' ⟶ Ie' ⟶ Ie" beschrieben. Das in der ersten Stufe gebildete Epoxid wird jedoch nicht mit Halogenwasserstoff behandelt (zur Erzeugung des entsprechenden Halohydrins), sondern direkt durch Umsetzung mit dem Amin in die Verbindung der Formel Ie übergeführt.

Die Endprodukte der vorliegenden Erfindung können in ihre pharmazeutisch annehmbaren Salze übergeführt werden, welche eine vergleichbare pharmakologische Aktivität zeigen. Die Endprodukte haben drei Aminfunktionen; von diesen sind jedoch lediglich zwei genügend basisch zur Bildung von stabilen Salzen. Deshalb bilden sie Disäuresalze mit verschiedenen organischen und anorganischen Säuren. Beispiele von verwendbaren organischen oder anorganischen Säuren sind Maleinsäure, Fumarsäure, Weinsäure, Zitronensäure, Chlorwasserstoff, Bromwasserstoff und Schwefelsäure. Eine typische Herstellung von einem dieser Salze erfolgt durch Mischen einer Lösung des basischen Endproduktes, z.B. der Verbindung der Formel Ia in einem ($C_1$ bis $C_4$)-Alkohol, mit einer Lösung einer oben beschriebenen, pharmazeutisch annehmbaren Säure, ebenfalls in einem ($C_1$ bis $C_4$)-Alkohol. Das so gebildete Salz kristallisiert spontan aus der Lösung aus, ebenso bei der Zugabe eines geeigneten Lösungsmittels, z.B. Aethylacetat, Aether, Aceton oder halogenierte Kohlenwasserstoffe, wie z.B. Chloroform, 1,2-Dichloräthan etc.

Ein alternatives Verfahren, welches im Falle von Verbindungen, wie dem Endprodukt Ib, brauchbar ist, umfasst die Behandlung von zwei Teilen Base mit überschüssiger Salzsäure in Methanol. Das Lösungsmittel wird dann im Vakuum entfernt, um die überschüssige Säure zu entfernen, welche das unstabile Trihydrochloridsalz bildet.

Das Salz wird dann wieder in Methanol aufgelöst und 1 Teil freie Base der Lösung zugesetzt. Das Dihydrochloridsalz wird dann durch Zugabe eines Lösungsmittels, wie oben beschrieben, aus der Lösung ausgefällt.

Die erfindungsgemässen Verbindungen und ihre pharmazeutisch annehmbaren Salze sind wertvolle α- und β-adrenergische Blocker, insbesondere bei Verwendung in oralen Präparaten. Im Rahmen der vorliegenden Erfindung werden die neuen Endprodukte und ihre pharmazeutisch annehmbaren Salze in pharmazeutische Darreichungsformen gebracht, welche etwa 0,1 bis etwa 50 mg, vorzugsweise 1 bis 50 mg, Wirkstoff enthalten, wobei die Dosierung der Spezies und den individuellen Erfordernissen anzupassen ist. Die neuen Endprodukte und ihre pharmazeutisch annehmbaren Salze können innerlich, z.B. parenteral oder enteral, in herkömmlichen pharmazeutischen Darreichungsformen verabreicht werden. Sie können beispielsweise in konventionelle pharmazeutische Darreichungsformen gebracht werden, z.B. in herkömmliche flüssige oder feste Trägermaterialien, wie Wasser, Gelatine, Stärke, Magnesiumstearat, Talk, pflanzliche Oele usw., um Tabletten, Elixiere, Kapseln, Lösungen, Emulsionen usw. gemäss herkömmlicher pharmazeutischer Praxis zu erhalten. Obwohl weniger bevorzugt,können auch intravenöse und intramuskuläre Darreichungsformen zur Bereitstellung der neuen Verbindungen verwendet werden.

Wie bereits oben erwähnt, besitzen die erfindungsgemässen Verbindungen sowohl α- als auch β-adrenergische Blockerwirkung, was für ihre Verwendung als Antihypertensiva wesentlich ist. Sie bewirken eine konkurrenzierende und reversible Blockade der α- und β-Adrenoreceptoren und sind überraschenderweise cardioselektiv, indem sie eine niedere $\beta_2$-Blockerwirkung und eine hohe $\beta_1$-Blockerwirkung besitzen. Diese Selektivität ist wesentlich bei der Auswahl eines Antihypertensivums. Ueberdies zeigen die Verbindungen eine sekretionshemmende, d.h. spasmolytische Aktivität.

Die α- und β-adrenergischen Blockerwirkungen können gemäss den folgenden Methoden bestimmt werden:

(A) Vas deferens von Meerschweinchen (α-Blocker)

Die vasa deferentia werden aus narkotisierten Meerschweinchen von etwa 500 g Gewicht herausgeschnitten. Die Segmente werden in einem 10 ml Organbad in Tyrode-Lösung aufgehängt, welche auf 34°C thermostatisiert ist und mit 95% Sauerstoff und 5% Kohlendioxid durchperlt wird. Die Ruhespannung wird auf 0,6 g eingestellt. Die Muskelkontraktionen werden isometrisch mit einem "force -displacement-transducer" gemessen und mit einem Schreiber aufgezeichnet.

Nach zweistündigem Aequilibrieren wird eine Dosis-Wirkungs-Kurve für Noradrenalinkontraktionen erstellt. Danach wird ausgewaschen, und die Organe werden für 10 Minuten mit verschiedenen Konzentrationen eines α-adrenergischen Blockers vor der erneuten Erstellung einer Noradrenalin-Dosis-Wirkungs-Kurve inkubiert. Die Affinitätskonstanten ($pA_2$) für die Antagonisten werden, wie von H.O. Schild in Brit. J. Pharmacol. 2, 189-206 (1947) beschrieben, ermittelt.

(B) Isolierte Meerschweinchen-Trachea ($\beta_2$-Blocker)

Tracheae werden aus narkotisierten Meerschweinchen von ungefähr 500 g Gewicht herausgeschnitten und zwischen den Knorpelringen geschnitten. Die Segmente werden zur Bildung einer Kette mit einem Faden zusammengebunden, wie von Castillo und DeBeer in J. Pharmacol. Exp. Ther. 90, 104-109 (1947) beschrieben, und in einem 10 ml Organbad in Tyrode-Lösung aufgehängt, welche auf 37°C thermostatisiert ist und mit 95% Sauerstoff und 5% Kohlendioxid durchperlt wird. Die Ruhespannung wird auf 0,5 eingestellt. Die Kontraktion des Trachealmuskels

wird isometrisch unter Verwendung eines "force-displacement-transducer's" gemessen und mit einem Schreiber aufgezeichnet.

Nach zweistündigem Aequilibrieren wird die durch $3 \cdot 10^{-7}$ Mol Carbachol ausgelöste Kontraktion durch Zugabe von Isoproterenol antagonisiert. Isoproterenol wird in 5-minütigen Intervallen zugesetzt, woraus eine kumulative Dosis-Wirkungs-Kurve des Agonisten gemäss der Methode von Van Rossum, Arch. Int. Pharmacol. Therapy 143, 299-330 (1963) abgeleitet wird. Die Wirkungen werden in Prozenten der maximalen Isoproterenol-Wirkung ausgedrückt.

Die β-adrenergische Blockerwirkung wird studiert, indem man verschiedene Konzentrationen des Antagonisten während 10 Minuten auf das Gewebe einwirken lässt, bevor erneut eine kumulative Dosis-Wirkungs-Kurve, wie oben erwähnt, erstellt wird. Der log $ED_{50}$-Wert in Gegenwart des β-Blockers wird vom log $ED_{50}$-Wert ohne β-Blocker subtrahiert. Diese in Logarithmen umgewandelten Werte werden als Funktion der β-Blocker-Konzentration aufgetragen. Die Affinität des Antagonisten ($pA_2$) ist der negative Logarithmus der molaren Konzentration des Antagonisten, welche eine Verschiebung um einen Faktor 2 hervorruft.

(C) Atria von Meerschweinchen ($\beta_1$-Blocker

Die Herzen von narkotisierten Meerschweinchen von ungefähr 500 g Gewicht werden herausgeschnitten und sofort in eine Ringer-Locke-Lösung gebracht. Die rechten, spontan schlagenden Atria werden sorgfältig freipräpariert, damit der Sinusknoten nicht verletzt wird. Es werden an die Spitze eines jeden Atriums ein Faden gebunden. Das Präparat wird in einem 10 ml Organbad in Ringer-Locke-Lösung aufgehängt, welche auf 30°C thermostatisiert ist und mit 95% Sauerstoff und 5% Kohlendioxid durchperlt wird. Die Kontraktionsfrequenz des Herzens wird mit einem "force-displacement-

transducer's" gemessen und mit einem Schreiber aufgezeichnet.

Nach einstündiger Aequilibrierung wird die Ruheherzfrequenz der Atria gemessen. Eine zehn Punkte umfassende kumulative Dosis-Wirkungs-Kurve für Isoproterenol ($1 \cdot 10^{-9}$ Mol bis $3 \cdot 10^{-5}$ Mol) wird erstellt und das Gewebe danach ausgewaschen. Nach einer 90minütigen Erholungsphase werden die Atria während 30 Minuten mit den β-Blockern inkubiert, bevor die Erstellung der kumulativen Dosis-Wirkungs-Kurve für Isoproterenol wiederholt wird.

Die Blockeraffinitätskonstanten ($pA_2$) werden, wie oben beschrieben, graphisch ermittelt.

Die pharmakologischen Daten für die Verbindungen der Formel I und deren Racemate sind in der folgenden Tabelle zusammengestellt.

Tabelle

| Verbindungen | Affinitätskonstanten $pA_2$ (molar) A) | B) | C) |
|---|---|---|---|
| Phentolamin | 6,7 | - | - |
| Propanolol | - | 6,2 | 6,7 |
| Practolol | - | < 5,0 | 4,9 |
| (R,S)-1-[2-(4-(2-Hydroxy-3-isoproproylaminopropoxy)-phenoxy)-äthyl]-4-phenylpiperazin | 5,2 | < 5,0 | 4,1 |
| (S)-1-[6-(4-(2-Hydroxy-3-isopropylaminopropoxy)-phenoxy)-hexyl]-4-phenylpiperazin | 5,2 | < 5,0 | 4,3 |
| 4-[(2-Hydroxy-3-isopropylaminopropoxy)phenyl]-N-[2-(4-phenyl-1-piperazinyl)-äthyl]acetamid | 5,6 | < 5,0 | 4,3 |
| 4-[(2-Hydroxy-3-isopropylaminopropoxy)phenyl]-N-[6-(4-phenyl-1-piperazinyl)-hexyl]acetamid | 6,0 | < 5,0 | 4,7 |

Die erfindungsgemässen Verbindungen gehören der Klasse der 1-Aryloxy-3-alkylaminopropan-2-ole an; bei vielen Beispielen dieser Klasse von Verbindungen konnte gezeigt werden, dass sie β-adrenergische Blockerwirkung aufweisen. Da diese Klasse von Verbindungen ein asymmetrisches Zentrum besitzt, existieren diese Verbindungen in zwei enantiomeren Formen. Es wurde gefunden, dass die β-Blockerwirkung solcher Verbindungen zu einem grossen Teil in den Isomeren mit der absoluten Konfiguration S gefunden wird, d.h. demjenigen Isomeren, welche dem natürlich vorkommenden β-Agonisten (R)-(-) Noradrenalin stereochemisch äquivalent sind, während die racemischen

Formen ungefähr eine etwa halb so grosse Aktivität zeigen, vergleiche z.B.

(I) R. Howe & B.S. Rao, J. Med. Chem. 11, 1118, (1968)
(2) B. Ablad et al, Acta. Pharmacol Toxicol. 25, 85 (1967)
(3) L. Almirante & W. Murmann, J. Med. Chem. 9 650 (1966)
(4) M. Dukes & L.H. Smith, J. Med. Chem. 14 326 (1971)
(5) J.C. Danilewiez & J.E.G. Kemp, J. Med. Chem. 16 168 (1973)

Das gewünschte S-Isomere der erfindungsgemässen Verbindungen ist auf zwei Wegen erhältlich:

(a) durch Trennung des racemischen Materials über eine fraktionierte Kristallisation seiner mit einer optisch aktiven Säure, wie Weinsäure, gebildeten, diastereoisomeren Salze.

(b) durch asymmetrische Synthese unter Verwendung eines optisch aktiven Synthons der passenden absoluten Konfiguration. Zwei solche Synthone, XXII und XXVII, welche leicht aus D-Mannit, einem natürlich vorkommenden Zucker, erhältlich sind, wurden zum Aufbau der Oxypropanolamin-Seitenkette in den erfindungsgemässen Verbindungen verwendet. Synthon XXII kann nur für die Synthese von solchen α-, β-Blockern verwendet werden, deren Struktur die im Verlaufe der Synthese notwendige Abspaltung der Schutzgruppe $R^3$, d.h. die reduktive Spaltung unter Verwendung eines gemischten Metallhydrides, erlaubt. Ist eine solche reduktive Abspaltung im Hinblick auf die Struktur des Endproduktes nicht möglich, z.B. in Verbindung Ib wegen der vorhandenen Amidgruppe, wird das Synthon XXVII zur Einführung der Oxypropanolamin-Seitenkette verwendet.

Die Angabe von verschiedenen Publikationen und US-Patentschriften erfolgte im Hinblick auf eine vollständige Offenbarung.

Die folgenden Beispiele illustrieren die Erfindung, beschränken diese jedoch nicht. Alle Temperaturangaben beziehen sich auf °C, sofern nicht anders angegeben.

Beispiel 1

Eine Mischung aus 546 g (3,0 Mol) pulverisiertem D-Mannit, 3,0 g p-Toluolsulfonsäure und 780 g (7,5 Mol) Dimethoxypropan in 900 ml trockenem Dimethylsulfoxid wird bei Raumtemperatur unter wasserfreien Bedingungen gerührt. Innerhalb von 30 Minuten bis zu einer Stunde löst sich das gesamte Mannit auf. Nach 16 Stunden wird die Reaktionsmischung in 900 ml einer gesättigten Natriumbicarbonatlösung gegossen und dann weiter mit 2 Litern Wasser verdünnt. Die Mischung wird mit Aethylacetat (1 x 4,5 Liter; 3 x 3 Liter) extrahiert; die Extrakte werden ihrerseits mit Wasser (3 x 1,5 Liter) gewaschen. Die vereinigten Aethylacetatextrakte werden über Natriumcarbonat getrocknet und im Vakuum bei einer Badtemperatur von etwa 45° eingeengt, bis der Rückstand erstarrt ist. Der Rückstand wird dann auf Rückflusstemperatur erhitzt, um den Feststoff wieder aufzulösen; die Lösung wird mit 8 Litern heissem Hexan verdünnt. Man lässt die Mischung über Nacht langsam abkühlen; das gebildete kristalline Material wird abfiltriert und mit einer Aether/Hexan-Mischung (1:3; 4 x 500 ml) gewaschen. Man erhält (2R,3R,4R,5R)-Mannit-1,2,5,6-diacetonid, Schmelzpunkt 115-119°.

Die Mutterlaugen werden zur Trockene eingeengt. Eine Lösung des entstandenen Rückstandes in Aether (300 ml) wird mit etwa 1,6 Litern Hexan verdünnt. Dies ergibt weiteres Diacetonid, Schmelzpunkt 119-120°.

Beispiel 2

263 g (0,59 Mol) Bleitetraacetat werden unter Argon in 1500 ml trockenem Benzol dispergiert. Zur schnell gerührten Mischung werden innerhalb von 15 Minuten in 5-10 g Portionen 140 g (2R,3R,4R,5R)-Mannit -1,2,5,6-diacetonid zugesetzt; anschliessend werden 10 weitere 1 g Portionen (2R,3R,4R,5R)-Mannit -1,2,5,6-diacetonid zugegeben, bis die Mischung einen negativen Test für ein Oxidans (Kaliumjodid/Stärke-Papier) zeigt. Die Mischung wird über Celit filtriert; der Filterkuchen wird mit zwei 100 ml Portionen trockenem Benzol gewaschen. Das Filtrat wird zur Neutralisation der Essigsäure, welche in der Oxidationsreaktion entstanden ist, mit 300 g wasserfreiem Kaliumcarbonat während 30 Minuten gerührt. Nach einer zweiten Filtration über Celit wird die Lösung mit 450 ml Isopropylamin und 300 g Kaliumcarbonat behandelt. Nach einem halbstündigen Rühren wird die Mischung filtriert; das Filtrat wird über 15 g 10% Palladium auf Kohle bei Atmosphärendruck und einer Temperatur von 23$^{o}$ hydriert. Die Reaktion ist nach der Aufnahme von 26,4 Litern Wasserstoff im wesentlichen beendet. Der Katalysator wird durch Filtration entfernt; die Einengung des Filtrates ergibt (2S)-3-Isopropylamino-1,2-propandiolacetonid.

## Beispiel 3

90 ml (1,2 Mol) Mesylchlorid werden unter Rühren zu einer vorher auf -10$^{o}$ gekühlten Lösung von 188 g (1,087 Mol) (2S)-3-Isopropylamino-1,2-propandiolacetonid und 288 ml (1,63 Mol) Triäthylamin in trockenem Tetrahydrofuran mit einer solchen Geschwindigkeit zugegeben, dass die Reaktionstemperatur 5$^{o}$ nicht übersteigt. Die Mischung wird dann während 30 Minuten bei 10-15$^{o}$ gerührt, worauf sie mit 1,5 Litern gesättigter Kochsalzlösung verdünnt wird. Die Schichten werden voneinander getrennt; die wässrigen Schichten werden mit Aether (3 x 500 ml) extrahiert. Die organischen Schichten werden ihrerseits mit gesättigter Kochsalzlösung (2 x 500 ml) gewaschen und dann vereinigt, über Natriumsulfat getrocknet und eingedampft, wodurch man (2S)-3-N-Mesylisopropylamino-1,2-propandiolacetonid als ein Oel erhält.

Eine kleine Portion wird aus Hexan umkristallisiert (3 x ), wodurch man analytisch reines Material erhält, Schmelzpunkt 33-34$^{o}$; $[\alpha]_{D}^{25}$ -14,76$^{o}$ (c = 1,0% in Chloroform).

## Beispiel 4

200 ml mit Wasser und Methanol vorgewaschenes Dowex 50W-3X Ionenaustauscherharz ($H^+$-Form) werden zu einer Lösung von 264 g rohem (2S)-3-N-Mesylisopropylamino-1,2-propandiolacetonid in einem Liter Methanol und 325 ml Wasser gegeben. Man rührt die Mischung 90 Minuten lang unter Rückfluss. Die gekühlte Mischung wird filtriert; das Filtrat wird in Vakuum eingeengt. Der Rückstand wird mehrere Male aus Benzol/Aethanol-Mischungen eingedampft, um letzte Spuren von Wasser zu entfernen. Der entstandene Feststoff wird mit 2,5 Litern Aether zerrieben und liefert (2S)-3-N-Mesylisopropylamino-1,2-propandiol, Schmelzpunkt 67-70°. Engt man den Aether ein, so erhält man eine weitere Menge an Produkt, Schmelzpunkt 64-66°. Die Kristallisation aus Aethylacetat/Hexan liefert analytisch reines Produkt, Schmelzpunkt 73-74°; $[\alpha]_D^{25}$ -15,94° ( c = 1,0% in Wasser).

## Beispiel 5

Man löst 19,1 g (90,5 mMol) (2S)-3-N-Mesylisopropylamino-1,2-propandiol in 150 ml wasserfreiem Pyridin unter Argon auf und kühlt auf -45° ab. 7.0 ml (40,0 mMol) Mesylchlorid werden innerhalb von 5 Minuten tropfenweise zugesetzt; die Mischung wird während 5 Stunden bei -45° gerührt. Die kalte Mischung wird dann mit 100 ml Wasser und anschliessend mit 200 ml 6N Salzsäure verdünnt und 3 x mit Aethylacetat extrahiert. Die Aethylacetatschichten werden ihrerseits mit 250 ml 3N Salzsäure, gesättigter Kochsalzlösung und Natriumbicarbonatlösung gewaschen. Die vereinigten Extrakte werden eingedampft und man erhält (S)-1-Mesyloxy-2-hydroxy-3-N-mesylisopropylaminopropan als ein Oel, welches beim Stehen erstarrt.

Dieses Material ist mit etwa 2% (S)-1,2-Dimesyloxy-3-N-mesylisopropylaminopropan verunreinigt. Eine kleine Probe wird wie folgt gereinigt:

500 mg werden in 10 ml Wasser aufgelöst und durch Filtration von nicht aufgelöstem Material getrennt. Das Filtrat wird 2 x mit Aether und dann 2 x mit Aethylacetat extrahiert. Man vereinigt die Aethylacetatschichten, trocknet über Natriumsulfat und dampft ein. Die Kristallisation des Rückstandes aus Aether liefert eine analysenreine Probe, Schmelzpunkt 51-52°, $[\alpha]_D^{25}$ -1,21° (c = 1,0% in Wasser).

## Beispiel 6

Eine gerührte Lösung von 63,3 g (0,3 Mol) (2S)-3-N-Mesylisopropylamino-1,2-propandiol in 450 ml Pyridin wird auf -20° abgekühlt; es werden 85,5 g (0,45 Mol) Tosylchlorid zugegeben. Man rührt die Reaktionsmischung 2 Stunden lang in einem Eiswasserbad, worauf 5-10 g Eis der Mischung zugesetzt werden; das Rühren wird während weiteren 15 Minuten fortgesetzt, bevor man auf eine Mischung aus 1 kg Eis und 500 ml konzentrierter Salzsäure giesst und mit Methylenchlorid extrahiert (1 x 1 Liter; 2 x 500 ml). Die Extrakte werden vereinigt und ihrerseits mit 500 ml gesättigter Kochsalzlösung und 5%iger Natriumbicarbonatlösung gewaschen. Man trocknet die vereinigten Extrakte über Natriumsulfat und engt im Vakuum ein, wodurch (S)-1-Tosyloxy-2-hydroxy-3-N-mesylisopropylaminopropan als ein Oel erhalten wird. Eine kleine Probe wird zu Analysenzwecken chromatographiert; $[\alpha]_D^{25}$ +5,65° (c = 1,0% in Chloroform).

## Beispiel 7

Eine Mischung aus 10,55 g (50 mMol) (2S)-3-N-Mesylisopropylamino-1,2-propandiol, 9,0 g (75 mMol) Trimethylorthoacetat und 0,4 g (3,27 mMol) Benzoesäure wird unter Rühren während 45 Minuten auf 80° erhitzt. Die viscose Reaktionsmischung wird abgekühlt und zwischen Methylenchlorid und einer gesättigten Natriumbicarbonatlösung verteilt. Die Methylenchloridschicht wird über Natriumsulfat getrocknet und eingeengt, wodurch rohes cyclisches

Orthoacetat erhalten wird, welches dann in 75 ml Methylenchlorid aufgelöst, mit 12 ml (95 mMol) Trimethylchlorsilan behandelt und während 60 Minuten unter Rückfluss erhitzt wird. Man entfernt das Lösungsmittel im Vakuum; der Rückstand wird in 100 ml 0,3N methanolischer Salzsäure aufgelöst; die Mischung lässt man während etwa 65 Stunden bei Raumtemperatur stehen. Das Lösungsmittel wird dann unter reduziertem Druck entfernt und das übrigbleibende Oel über 50 g Silicagel chromatographiert, wodurch man reines (2S)-1-Chlor-2-hydroxy-3-N-mesylisopropylaminopropan als ein Oel erhält, $[\alpha]_D^{25}$ -12,7° (c = 1,0% in Methanol).

## Beispiel 8

44 g einer 50%igen Dispersion von Natriumhydrid in Oel werden in einen Kolben gebracht und mit trockenem Hexan gewaschen. Dieser Kolben wird dann mit einer Lösung von 120 g Benzylchlorid in 1500 ml trockenem Dimethylformamid beschickt. Zu dieser gerührten Mischung gibt man innerhalb von 45 Minuten eine Lösung von (2S)-Glycerin-2,3-acetonid (120 g) in trockenem Dimethylformamid (500 ml) bei Raumtemperatur zu (die Reaktionstemperatur beträgt am Anfang 17° und steigt dann während der Zugabe bis zu einem Maximum von 27° an). Nach beendeter Zugabe wird das Rühren eine Stunde lang fortgesetzt; dann gibt man etwa 30 ml Methanol tropfenweise zu, um überschüssiges Hydrid zu vernichten. Die Apparatur wird dann mit einer Destillationsvorrichtung ausgerüstet; das Dimethylformamid wird im Vakuum abdestilliert (55-60°). Man verdünnt den Rückstand mit gesättigter Kochsalzlösung (2 Liter) und extrahiert mit Methylenchlorid (3 x 1 Liter). Die organischen Schichten werden ihrerseits mit gesättigter Kochsalzlösung gewaschen und dann vereinigt, über Magnesiumsulfat getrocknet und eingedampft, wodurch man ein Oel erhält.

Das Oel wird im Vakuum destilliert und man erhält (2S)-3-Benzyloxypropandiol-1,2-acetonid, Sdp. 78-80°/

0,05 mm Hg.

## Beispiel 9

Eine Suspension von 75 ml Dowex 50W-3X ($H^+$-Form) in einer gerührten Lösung von 254,6 g (1,15 Mol) (2S)-3-Benzyloxypropandiol-1,2-acetonid in 800 ml Methanol und 200 ml Wasser wird eine Stunde lang unter Rückfluss erhitzt. Das Harz wird abfiltriert; das Filtrat wird im Vakuum eingeengt. Man befreit das (2R)-3-Benzyloxy-1,2-propandiol vom restlichen Wasser, indem man mehrere Male aus Aethanol/Benzol-Mischungen eindampft. Auf diese Weise erhält man ein farbloses Oel.

## Beispiel 10

Eine gerührte Lösung von 207 g (1,13 Mol) (2R)-3-Benzyloxy-1,2-propandiol in 200 g (1,66 Mol) Trimethylorthoacetat, welche 3 g (0,024 Mol) Benzoesäure enthält, wird in einem Oelbad in einem für eine Abwärtsdestillation ausgerüsteten Kolben auf 75$^O$ erwärmt. Das entstandene Methanol wird laufend aus der Reaktionsmischung entfernt. Nach 40 Minuten wird die Reaktionsmischung abgekühlt und zwischen Methylenchlorid (800 ml) und einer 1N Natriumhydroxidlösung (200 ml) verteilt. Die wässrige Phase wird weiter mit Methylenchlorid (2 x 250 ml) extrahiert; dann werden die organischen Schichten ihrerseits mit 200 ml 0,5N Natriumhydroxidlösung gewaschen. Man trocknet die vereinigten Methylenchloridschichten über Natriumsulfat und engt zur Trockene ein, wodurch das cyclische Orthoacetat 2(R,S),4(S)-2-Methoxy-2-methyl-4-[(benzyloxy)methyl)]-1,3-dioxolan als ein farbloses Oel erhalten wird.

Eine Analyse des NMR-Spektrums ergibt eine 4:3-Mischung von Diastereoisomeren. Eine kleine Probe wird destilliert (ungefähr 165$^O$/0,1 mm) und liefert eine analysenreine Probe $[\alpha]_D^{25}$ +7,4$^O$ (c = 1,0% in Methanol).

## Beispiel 11

155 ml (1,21 Mol) Trimethylchlorsilan werden zu einer Lösung von 270 g (1,13 Mol) rohem 2(R,S),4(S)-2-Methoxy-2-methyl-4-[(benzyloxy)methyl)]-1,3-dioxolan in 600 ml trockenem Methylenchlorid zugegeben; die Mischung wird 30 Minuten in einer inerten Atmosphäre unter Rückfluss erhitzt. Das Lösungsmittel und das überschüssige Reagens werden unter vermindertem Druck entfernt, wodurch man (2S)-3-Benzyloxy-2-acetoxy-1-chlorpropan als ein bewegliches Oel erhält. Dieses Material wird ohne weitere Reinigung verwendet.

Eine kleine Probe wird destilliert (ungefähr 165°/0,1 mm) und liefert eine analysenreine Probe $[\alpha]_D^{25}$ +10,8° (c = 1,0% in Methanol).

## Beispiel 12

Eine gekühlte Lösung von 115 g Natriumhydroxid (2,875 Mol) in 600 ml Wasser wird tropfenweise während 20 Minuten einer gekühlten Lösung von (2S)-3-Benzyloxy-2-acetoxy-1-chlorpropan (theoretisch 1,13 Mol) in Methanol (800 ml) unter Rühren zugesetzt. Die Reaktionstemperatur wird während der Zugabe unterhalb 12° gehalten. Nachdem eine weitere Stunde bei etwa 12° gerührt worden ist, wird die Reaktionsmischung auf 5° abgekühlt und dann unter Verwendung von verdünnter Schwefelsäure auf pH 7,0 neutralisiert. Die Reaktionsmischung wird dann im Vakuum eingeengt (Badtemperatur ungefähr 25°), um das Methanol zu entfernen; dann wird sie mit 250 ml gesättigter Kochsalzlösung verdünnt und mit Methylenchlorid extrahiert (1 x 750 ml; 1 x 400 ml). Die Methylenchloridextrakte werden vereinigt, über Natriumsulfat getrocknet und zur Trockene eingedampft. Das resultierende Oel wird im Vakuum destilliert; man erhält (2S)-1,2-Epoxy-3-benzyloxypropan, Sdp. 84-86°/0,45 mm Hg; $[\alpha]_D^{25}$ -10,64° (c = 1,0% in Methanol).

Beispiel 13

Eine Lösung von (S)-1-Tosyloxy-2-hydroxy-3-N-mesylisopropylaminopropan (103 g, 0,282 Mol) und p-Benzyloxyphenol (79 g, 0,395 Mol) in 500 ml Dimethylsulfoxid wird mit 93,7 ml 4N Natriumhydroxidlösung (0,375 Mol) behandelt und bei 100° unter Argon während 2-2 1/2 Stunden gerührt. Man kühlt die Lösung ab und gibt langsam 300 ml 1N Natriumhydroxidlösung zur kräftig gerührten Lösung. Anschliessend erfolgt die Zugabe von 600 ml Wasser. Der entstandene Feststoff wird durch Filtration entfernt und mit Wasser gewaschen. Nach einem teilweisen Trocknen an Luft wird der Feststoff in Methylenchlorid aufgelöst; die Lösung wird über Magnesiumsulfat getrocknet. Die entfärbte Lösung (Aktivkohle) wird zur Trockene eingedampft und der entstandene feste Rückstand mit heissem Aether (1,5 Liter) zerrieben. Die Filtration des farblosen Feststoffes ergibt (S)-1-(4-Benzyloxyphenoxy)-2-hydroxy-3-N-mesylisopropylaminopropan, Schmelzpunkt 91-93°.

Die Umkristallisation aus Methylenchlorid/Aether ergibt eine reine Probe, Schmelzpunkt 94-95°; $[\alpha]_D^{25}$ -0,93° (c = 1,0% in Chloroform).

Beispiel 14

Eine Lösung von 8,3 g (36,1 mMol) (2S)-1-Chlor-2-hydroxy-3-N-mesylisopropylaminopropan und 8,69 g (43,4 mMol) p-Benzyloxyphenol in 75 ml Dimethylsulfoxid wird mit 10,86 ml einer 4N Natriumhydroxidlösung (43,4 mMol) behandelt; man erwärmt die Mischung 5 Stunden lang auf 100°. Zur abgekühlten Reaktionsmischung gibt man unter Rühren 40 ml einer 1N Natriumhydroxidlösung und 60 ml Wasser; der entstandene Feststoff wird durch Filtration gesammelt und gut mit Wasser gewaschen. Das getrocknete rohe Produkt wird aus Aethylacetat/Hexan umkristallisiert und liefert (S)-1-(4-Benzyloxyphenoxy)-2-hydroxy-3-N-mesylisopropylaminopropan als weisse Nadeln, Schmelzpunkt 93-94°.

Beispiel 15

Ein Brei von 10% Palladium auf Kohle in 30 ml Aethylacetat wird zu einer Lösung von 38,4 g (S)-1-(4-Benzyloxyphenoxy)-2-hydroxy-3-N-mesylisopropylamino-propan in 850 ml Methanol gegeben; die Mischung wird hydriert (760 mm Hg; 20°). Innerhalb einer Stunde ist die Aufnahme von Wasserstoff beendet (insgesamt 2,6 Liter) und der Katalysator wird mittels Filtration über Celit entfernt. Das Filtrat wird unter vermindertem Druck eingeengt und der Rückstand aus Methylenchlorid/Aether kristallisiert, wodurch man (S)-1-(4-Hydroxyphenoxy)-2-hydroxy-3-N-mesyl-isopropylaminopropan erhält, Schmelzpunkt 91-93°. Eine Umkristallisation einer Probe aus Aethylacetat/Hexan ergibt analytisch reines Material, Schmelzpunkt 92-94°; $[\alpha]_D^{25}$ -1,93° (c = 1,0% in Chloroform).

Beispiel 16

Zu einer gerührten Lösung von 24 g (0,08 Mol) (S)-1-(4-Hydroxyphenoxy)-2-hydroxy-3-N-mesylisopropylaminopropan in 240 ml absolutem Aethanol (Argonatmosphähre) werden bei Raumtemperatur 9,04 g (0,08 Mol) Kalium-t-butoxid gegeben. Nach 15minütigem Rühren gibt man 13,36 g (0,08 Mol) Aethylbromacetat zu; die Reaktionsmischung wird über Nacht unter Rückfluss erhitzt. Sie wird dann abgekühlt und im Vakuum eingeengt. Der Rückstand wird mit 1N Salzsäure auf pH 2 angesäuert; dann wird er in Aethylacetat aufgelöst. Man wäscht die organische Schicht 2 x mit 1N Natriumhydroxidlösung, 1 x mit Wasser und trocknet über Natriumsulfat. Die Verdampfung des Lösungsmittels liefert den rohen Ester als ein Oel, welcher beim Stehenlassen erstarrt.

Zu einer Lösung von 29 g (0,75 Mol) des rohen Esters in 290 ml Methanol gibt man 40 ml 4N Natriumhydroxidlösung. Die Reaktionsmischung wird während 10 Minuten unter Rückfluss erhitzt; dann lässt man während 1 Stunde auf Raumtemperatur abkühlen. Der grösste Teil des Lösungsmittels wird im Vakuum abgedampft; man behandelt den Rückstand mit 50 ml 6N Salzsäurelösung. Der resultierende Feststoff wird durch Filtration gesammelt und 2 x aus Aceton/Hexan kristallisiert; man erhält (S)-4-(2-Hydroxy-3-N-mesylisopropylamino-propoxy)phenoxy-essigsäure, Schmelzpunkt 128-129°. Die Kristallisation aus Aceton/Hexan ergibt eine analytisch reine Probe, Schmelzpunkt 129-130°; $[\alpha]_D^{25}$ +1,5° (c = 1,0% in Methanol).

## Beispiel 17

Zu einer gerührten Lösung von N-Phenylpiperazin (95% rein; 300 g; 1,85 Mol) in 1 Liter Methanol, welches vorher auf -20° abgekühlt worden ist, wird in einer Portion eine gekühlte Lösung von Aethylenoxid (150 ml) in Methanol (250 ml) zugegeben. Die Mischung wird über Nacht in einem Eiswasserbad gerührt. Nach dem Entfernen von Lösungsmittel und überschüssigem Reagens im Vakuum wird der Rückstand in Toluol aufgenommen und zur Entfernung von restlichem Methanol wieder eingedampft.

Das so erhaltene rohe 1-(2-Hydroxyäthyl)-4-phenyl-piperazin (ungefähr 400 g) wird in trockenem Methylenchlorid (3,5 Liter), welches Triäthylamin (400 ml; 2,9 Mol) enthält, aufgelöst; die Lösung wird auf -10° abgekühlt. Eine Lösung von 250 g (2,18 Mol) Mesylchlorid in 300 ml Methylenchlorid wird innerhalb von 30 Minuten der gerührten Mischung zugesetzt; dann lässt man die Reaktionsmischung bis auf Raumtemperatur erwärmen. Sie wird dann bei Raumtemperatur gerührt, bis die Umwandlung des Mesylat-Zwischenproduktes zur Chlorverbindung vollständig abgelaufen ist (16-40 Stunden). Man gibt Wasser zu (1 Liter) und trennt die Schichten. Die wässrige Schicht wird mit Methylenchlorid (1 x 300 ml) gewaschen; dann werden die vereinigten Methylenchloridphasen über Kaliumcarbonat getrocknet und eingedampft. Der Rückstand wird mit heissem Hexan (1 x 1 Liter; 4 x 200 ml) zerrieben; die vereinigten Extrakte werden entfärbt (Aktivkohle) und dann auf 0-5° abgekühlt. Die Filtration der entstandenen farblosen Kristalle ergibt 1-(2-Chloräthyl)-4-phenylpiperazin, Schmelzpunkt 56-58°. Einengen der Mutterlauge auf etwa 400 ml liefert zusätzliches Produkt, Schmelzpunkt 55-57°.

Eine Umkristallisation einer kleinen Probe aus Hexan liefert analytisch reines Material, Schmelzpunkt 59-60°.

## Beispiel 18

In analoger Weise wie in Beispiel 17 führt man 50 mMol N-(2-Methoxyphenyl)piperazin in 1-(2-Chloräthyl)-4-(2-methoxyphenyl)piperazin, Schmelzpunkt 36,5-37°, über.

## Beispiel 19

26,25 ml einer 4N Natriumhydroxidlösung (0,105 Mol) werden zu einer gerührten Mischung von 21,0 g (0,105 Mol) 4-Benzyloxyphenol und 22,5 g (0,1 Mol) 1-(2-Chloräthyl)-4-phenylpiperazin in 250 ml Dimethylsulfoxid zugegeben; die Reaktionsmischung wird eine Stunde lang auf 60° erwärmt. Nach Abkühlen wird die gerührte Mischung mit 50 ml einer 1N Natriumhydroxidlösung verdünnt; der kristalline Niederschlag wird durch Filtration gesammelt und mit Wasser gewaschen. Nach dem Trocknen im Vakuum erhält man im wesentlichen reines 1-[2-(4-Benzyloxyphenoxy)äthyl]-4-phenylpiperazin, Schmelzpunkt 116-119°.

Eine kleine Probe wird zur Analyse aus Aethylacetat umkristallisiert, Schmelzpunkt 120-121°.

## Beispiel 20

35,75 g 1-[2-(4-Benzyloxyphenoxy)äthyl]-4-phenylpiperazin werden rasch unter Rühren zu 75 ml konzentrierter Salzsäure gegeben; die Mischung wird 15 Minuten lang auf einem Dampfbad erwärmt. Während dieser Zeit löst sich das

Ausgangsmaterial; dann beginnt sich ein weisser kristalliner Feststoff zu bilden und schliesslich erstarrt die Reaktionsmischung beinahe. Die Mischung wird auf etwa 5° abgekühlt; dann wird mit 100 ml Aethanol verdünnt. Die Feststoffe werden abfiltriert und mit Aethanol und Aether gewaschen; man erhält 1-[2-(4-Hydroxyphenoxy)äthyl]-4-phenylpiperazin als Monohydrochlorid. Das Salz wird in 150 ml heissem Methanol und 50 ml Wasser aufgelöst und dann mit 25 ml Triäthylamin behandelt. Man gibt dann zur unter Rückfluss kochenden Mischung bis zur beginnenden Trübung Wasser zu, worauf das Produkt aus der Lösung auszukristallisieren beginnt. Man kühlt die Mischung ab und sammelt die Feststoffe durch Filtration und erhält die freie Base, Schmelzpunkt 142-143°.

Eine Umkristallisation aus Aethylacetat liefert analytisch reines Material, Schmelzpunkt 143-144°.

## Beispiel 21

1-[2-(4-Benzyloxyphenoxy)äthyl]-4-phenylpiperazin (211,2 g; 0,544 Mol) in 1 Liter Essigsäure wird über 20 g 10% Palladium auf Kohle hydriert (21°; 1 Atmosphäre). Die Absorption des Wasserstoffs ist nach 3 Stunden im wesentlichen beendet (gesamte Aufnahme 15,1 Liter). Man filtriert den Katalysator über Celit ab und engt das Filtrat im Vakuum zur Trockene ein. Das rohe Material wird in 750 ml heissem Methanol aufgelöst. Man gibt 250 ml heisses Wasser (ungefähr 70°) und anschliessend 120 ml Triäthylamin zu. Wasser wird anschliessend bis zur beginnenden Trübung zugesetzt, worauf das Produkt rasch aus der Lösung auszukristallisieren beginnt. Man kühlt die Mischung auf etwa 5° ab, entfernt das kristalline Material mittels Filtration und wäscht mit Methanol/Wasser (1:1), wodurch man 1-[2-(4-Hydroxyphenoxy)äthyl]-4-phenylpiperazin, Schmelzpunkt 143-144° erhält.

Beispiel 22

11,0 ml einer 4N Natriumhydroxidlösung (44 mMol) werden zu einer gerührten Lösung von 13,3 g (43,9 mMol) (S)-1-(4-Hydroxyphenoxy)-2-hydroxy-3-N-mesylisopropylaminopropan und 10,0 g (44,4 mMol) 1-(2-Chloräthyl)-4-phenylpiperazin in 100 ml Dimethylsulfoxid zugegeben. Die Mischung wird unter Argon auf 60° erwärmt, anschliessend abgekühlt und mit 200 ml Wasser verdünnt. Der entstandene Feststoff wird durch Filtration gesammelt, mit Wasser gewaschen und dann in Methylenchlorid aufgelöst. Die Methylenchloridlösung wird mit 5%iger Natriumcarbonatlösung gewaschen, über Kaliumcarbonat getrocknet und anschliessend im Vakuum zu einem weissen Feststoff eingedampft. Die Kristallisation des Produktes aus Aethylacetat liefert (S)-1-[2-(4-(2-Hydroxy-3-N-mesylisopropylaminopropoxy)phenoxy)äthyl]-4-phenylpiperazin in zwei Fraktionen: Schmelzpunkt 104-106°; Schmelzpunkt 103-105°.

Eine kleine Probe der zweiten Fraktion wird aus Aethylacetat umkristallisiert, wodurch man analytisch reines Material erhält, Schmelzpunkt 104-106°; $[\alpha]_D^{25}$ -0,5° (c = 1% in Chloroform).

Beispiel 23

300 ml einer 4N Natriumhydroxidlösung (1,2 Mol) werden zu einer gerührten Mischung von 362 g (0,99 Mol) (S)-1-Tosyloxy-2-hydroxy-3-N-mesylisopropylaminopropan und 298,4 g (1 Mol) 1-[2-(4-Hydroxyphenoxy)äthyl]-4-phenylpiperazin in 2,4 Liter Dimethylsulfoxid zugegeben; die Reaktionsmischung wird während 12 Stunden auf 95-100° erwärmt. Die gekühlte Mischung wird mit einem Liter 1N Natriumhydroxidlösung und 7 Liter Wasser verdünnt; man extrahiert unter Verwendung von Benzol (1 x 12 Liter; 1 x 2 Liter). Man wäscht die organischen Extrakte ihrerseits mit Wasser (2 x 2 Liter), trocknet sie anschliessend über Kaliumcarbonat und dampft zur Trockene ein. Eine Lösung des Rückstandes in heissem Aethylacetat wird mit Aktivkohle behandelt und nach einer Einengung auf etwa 2,5 Liter über Nacht bei 0-5° stehen gelassen, wodurch man (S)-1-[2-(4-(2-Hydroxy-3-N-mesylisopropylaminopropoxy)-phenoxy)äthyl]-4-phenylpiperazin erhält. Man engt die Mutterlaugen auf etwa 500 ml ein und verdünnt mit 500 ml Hexan und erhält so weiteres Produkt. Die beiden Fraktionen werden vereinigt und aus Aceton/Hexan umkristallisiert, worauf man im wesentlichen reines Material erhält, Schmelzpunkt 95-100°.

Beispiel 24

4,5 g (0,04 Mol) Kalium-t-butoxid werden in eine Lösung von 66,3 g (0,404 Mol) (2S)-1,2-Epoxy-3-benzyloxypropan und 97,0 g (0,484 Mol) p-Benzyloxyphenol in 240 ml Methanol gegeben; die Mischung wird über Nacht unter Rückfluss erhitzt. Man kühlt die Mischung ab und verdünnt anschliessend unter langsamer Zugabe von 1 Liter einer 1N Natriumhydroxidlösung. Der entstandene Feststoff wird durch Filtration gesammelt, mit 0,5N Natriumhydroxidlösung und Wasser gewaschen und luftgetrocknet. Man löst das Material in 800 ml Methylenchlorid auf und wäscht die Lösung mit Wasser (3 x 150 ml). Die Wasserschichten werden ihrerseits mit 200 ml Methylenchlorid zurückgewaschen; Einengen der vereinigten, getrockneten (Natriumsulfat) Methylenchloridschichten liefert im wesentlichen reines (S)-1-(4-Benzyloxyphenoxy)-3-benzyloxy-2-propanol.

Eine kleine Probe wird aus Methylenchlorid/Hexan kristallisiert (2 x) ,und man erhält analytisch reines Material, Schmelzpunkt 62-63,5°; $[\alpha]_D^{25}$ +4,4° (c = 1,0% in Methanol).

Beispiel 25

Eine Lösung von 138 g (0,378 Mol) (S)-1-(4-Benzyloxyphenoxy)-3-benzyloxy-2-propanol in 1,7 Liter Essigsäure, enthaltend 36 ml konz. Salzsäure, wird bei Normaltemperatur und Atmosphärendruck über 14 g 10% Palladium auf Kohle hydriert. Die Aufnahme von Wasserstoff ist nach 70 Minuten im wesentlichen beendet (ungefähr 17,7 Liter Wasserstoff werden gebraucht). Man entfernt den Katalysator mittels Filtration über Celit und engt das Filtrat zur Trockene ein, was ein Oel ergibt, welches anschliessend in 800 ml 0,5N methanolischer Salzsäure aufgelöst wird. Zur Hydrolyse der Acetate, welche während der Hydrierung gebildet worden sind, lässt man über Nacht bei Raumtemperatur stehen. Man engt die Mischung zur Trockene ein und dampft den Rückstand mehrere Male aus Methylenchlorid ein, um restliche Salzsäure zu entfernen. Man zerreibt den entstandenen Feststoff mit Aether und erhält im wesentlichen reines (S)-3-(4-Hydroxyphenoxy)-1,2-propandiol.

Eine Probe von 0,5 g wird aus Methanol/Chloroform kristallisiert. Man erhält analytisch reines Material, Schmelzpunkt 149,5-151$^{o}$; $[\alpha]_D^{25}$ +8,01$^{o}$ (c = 1,0% in Methanol).

Beispiel 26

Eine gerührte Mischung von 62,15 g (0,337 Mol) (S)-3-(4-Hydroxyphenoxy)-1,2-propandiol und 0,025 Mol Benzoesäure in 60 g Trimethylorthoacetat wird in einem Oelbad auf 80$^{o}$ erwärmt. Das entstandene Methanol wird laufend abdestilliert. Nach 30 Minuten kühlt man die Reaktionsmischung ab und verteilt sie zwischen 750 ml Benzol und 250 ml einer gesättigten Natriumbicarbonatlösung. Die Benzolschicht wird mit einer weiteren 250 ml Portion Natriumbicarbonatlösung gewaschen, worauf

die wässerigen Schichten mit 250 ml Benzol zurückgewaschen werden. Die vereinigten organischen Extrakte werden über Natriumsulfat getrocknet und im Vakuum eingedampft, wodurch man das cyclische Orthoacetat als ein Oel erhält. Das gesamte rohe Material wird dann in 400 ml trockenem Methylenchlorid aufgelöst und mit 65 ml Trimethylchlorsilan behandelt. Nach einem 30-minütigen Erhitzen unter Rückfluss engt man die Lösung unter vermindertem Druck zur Trockene ein. Zur Entfernung von überschüssigem Reagens wird der Rückstand aus Toluol eingedampft und man erhält rohes (R)-3-(4-Hydroxyphenoxy)-2-acetoxy-1-chlorpropan als ein Oel. Das Acetat wird durch Erwärmen einer Lösung des Rohproduktes in 250 ml 0,5N methanolischer Salzsäure bei 55° während 30 Minuten hydrolysiert. Man engt das Lösungsmittel im Vakuum ein und verdünnt den Rückstand mit Wasser; die Extraktion mit Aethylacetat liefert rohes (R)-1-(4-Hydroxyphenoxy)-3-chlor-2-propanol. Das rohe Material wird in 120 ml Methylenchlorid aufgelöst und auf eine Silicagelkolonne (800 g, präpariert in Methylenchlorid) gebracht. Es werden 3 Liter des Eluierungsmittels Methylenchlorid gesammelt und verworfen; das Produkt wird unter Verwendung von 3 Litern Methylenchlorid/Aethylacetat (1:1) eluiert und man erhält nach Verdampfung des Lösungsmittels das Endprodukt als ein Oel.

## Beispiel 27

29,5 ml einer 4N Natriumhydroxidlösung (0,118 Mol) werden rasch tropfenweise zu einer gerührten Lösung von 12 g (59 mMol) (R)-1-(4-Hydroxyphenoxy)-3-chlor-2-propanol in 210 ml Dimethylsulfoxid zugegeben. Während der Zugabe wird die Temperatur der Mischung mittels eines Kühlbades unterhalb 25° gehalten. Nachdem die Mischung während 10 Minuten bei etwa 20° gerührt worden ist, gibt man 12,6 g (56,3 mMol) 1-(2-Chloräthyl)-4-phenylpiperazin in einer Portion zu;

die Mischung wird während 2 bis 2 1/2 Stunden auf 40° erwärmt (nach 20 Minuten beginntdie Kristallisation eines festen Materials aus der Lösung). Man kühlt die Mischung ab und verdünnt sie mit 30 ml Wasser. Der kristalline Niederschlag wird durch Filtration gesammelt und mit 100 ml Dimethylsulfoxid/Wasser (3:1) und mit Wasser gewaschen. Nach dem Trocknen an der Luft werden die Feststoffe in Benzol (600 ml) aufgelöst und mit Wasser (3 x 150 ml) gewaschen. Die wässerigen Schichten werden mit Benzol (1 x 200 ml) zurückgewaschen. Die vereinigten Benzolextrakte werden über Kaliumcarbonat getrocknet und eingedampft, wodurch man (S)-1-[2-(4-(2,3-Epoxypropoxy)-phenoxy)äthyl]-4-phenylpiperazin als einen weissen Feststoff erhält.

Eine kleine Probe aus einem vorangegangenen Versuch wird durch eine kurze Silicagelkolonne filtriert und aus Aceton kristallisiert, wodurch man analytisch reines Material erhält, Schmelzpunkt 118-119,5°; $[\alpha]_D^{25}$ -10,1° (c = 1,0% in Methanol).

## Beispiel 28

Unter einem Argonfluss werden 318 ml einer 70%igen Lösung von Natrium-bis-(2-methoxyäthoxy)aluminiumhydrid innerhalb von 15 Minuten zu einer gerührten Lösung von 36,5 g (S)-1-(4-Benzyloxyphenoxy)-2-hydroxy-3-N-mesylisopropylaminopropan in 800 ml trockenem Benzol zugegeben. Nach dem Ende der Zugabe wird die Mischung 3 Stunden lang unter Rühren unter Rückfluss erhitzt. Die Mischung wird unter Verwendung eines Eiswasser-Bades gekühlt; 30 ml einer 1N Natriumhydroxidlösung werden zur Vernichtung des Hydridüberschusses tropfenweise zugesetzt; anschliessend gibt man 800 ml Natriumhydroxidlösung zu. Die Phasen werden getrennt und die organische Schicht mit 1N Natriumhydroxidlösung und mit Wasser gewaschen. Die wässerige Phase und

wässerigen Waschflüssigkeiten werden mit Benzol (1 x 500 ml) zurückextrahiert. Die vereinigten Benzolextrakte werden über Kaliumcarbonat getrocknet und zu einem Feststoff eingedampft. Man löst das rohe Material in 1500 ml Aether auf und engt die Lösung auf etwa 750 ml ein. Das entstandene kristalline Produkt wird abfiltriert,und man erhält reines (S)-1-(4-Benzyloxyphenoxy)-2-hydroxy-3-isopropylaminopropan, Schmelzpunkt 93-95°; $[\alpha]_D^{25}$ -6,26° (c = 1,0% in Chloroform).

Die Einengung der Mutterlaugen ergibt zwei weitere Fraktionen an Produkt: Erste Fraktion, Schmelzpunkt 91-93°, zweite Fraktion Schmelzpunkt 89-93°.

## Beispiel 29

Ein Brei von 5,4 g 10% Palladium auf Kohle in 50 ml Benzol wird zu einer Lösung von 54,1 g (S)-1-(4-Benzyloxyphenoxy)-2-hydroxy-3-isopropylaminopropan in 600 ml Methanol zugegeben. Ein Hydrierungsversuch mit dieser Mischung zeigt, dass die Aufnahme von Wasserstoff infolge Vergiftung des Katalysators durch geringe Mengen schwefelhaltiger Verunreinigungen im Ausgangsmaterial sehr langsam verläuft. Der vergiftete Katalysator wird durch 5,4 g frischen Katalysator ersetzt, worauf 4,2 Liter Wasserstoff innerhalb von 40 Minuten aufgenommen werden. Man entfernt den Katalysator mittels Filtration und engt das Filtrat im Vakuum ein. Die Kristallisation des Rückstandes aus Aceton liefert (S)-1-(4-Hydroxyphenoxy)-2-hydroxy-3-isopropylaminopropan, Schmelzpunkt 125-127°; $[\alpha]_D^{25}$ -22,1° (c = 1,0% in 0,1N Salzsäure). Eine analytisch reine Probe, Schmelzpunkt 125-127°, wird durch Kristallisation aus Aceton erhalten.

Beispiel 30

Eine Lösung von 50 g (0,247 Mol) (R)-1-(4-Hydroxyphenoxy)-3-chlor-2-propanol in 300 ml Methanol, welche 100 ml Isopropylamin enthält, wird über Nacht unter Rückfluss erhitzt. Das Lösungsmittel wird im Vakuum entfernt und der Rückstand zweimal aus Methanol verdampft, um restliches Isopropylamin zu beseitigen. Man löst das rohe Produkt in 250 ml 1N Salzsäure auf und extrahiert die Lösung zur Entfernung nicht-basischer Verunreinigungen mit Aether (2 x). Die wässerige Schicht wird in einem Eiswasserbad abgekühlt und mit 13,5 g Natriumcarbonat (0,255 Aequivalente) behandelt. Nach 5 Minuten beginnt das Phenolamin aus der Lösung auszukristallisieren. Die Mischung wird während 30 Minuten in einem Eiswasserbad gerührt und anschliessend eine Stunde lang bei $0^{o}$ stehengelassen. Man sammelt das Produkt mittels Filtration, wäscht mit Wasser und erhält so (S)-1-(4-Hydroxyphenoxy)-2-hydroxy-3-isopropylaminopropan, Schmelzpunkt 124-126°. Man gibt 60 g Natriumchlorid zum mit den Waschflüssigkeiten vereinigten Filtrat zu und extrahiert die resultierende Lösung mit 6 x 200 ml Aethylacetat. Die Extrakte werden über Kaliumcarbonat getrocknet und eingedampft, wodurch man eine zusätzliche Menge an Produkt erhält.

Die Kristallisation des gesamten Produktes aus Aceton (Aktivkohle) liefert reines Produkt, Schmelzpunkt 124-126°; $[\alpha]_D^{25}$ -22,0° (c = 1,0% in 0,1N Salzsäure).

Beispiel 31

Methode A

Eine Lösung von 15,35 g (42,5 mMol) (S)-4-(2-Hydroxy-3-N-mesylisopropylaminopropoxy)phenoxyessigsäure in 170 ml trockenem Tetrahydrofuran wird auf $0^{o}$ gekühlt. Zu dieser Lösung (Argonatmosphäre) gibt man 5,16 g (51 mMol) Triäthylamin und anschliessend tropfenweise 4,61 g (42,5 mMol) Aethylchlorformiat. Man lässt die Reaktionsmischung während einer Stunde bei 0° rühren und filtriert dann vom entstandenen Niederschlag ab. Das Filtrat wird dann mit 10,35 g (63,8 mMol) N-Phenylpiperazin behandelt; man lässt die Reaktionsmischung während 90 Minuten bei Raumtemperatur unter Argon rühren. Man dampft das Lösungsmittel im Vakuum ein , löst den Niederschlag in Methylenchlorid auf und behandelt ihn mit 100 ml 5N Salzsäure. Die wässerige Schicht wird zweimal mit Methylenchlorid extrahiert. Die organischen Schichten werden dann zweimal mit 5N Salzsäure und einmal mit 5%iger Natriumbicarbonatlösung gewaschen. Man trocknet die vereinigten organischen Schichten über Natriumsulfat und dampft ein, wodurch man ein Oel erhält, welches in Benzol aufgelöst und an Silicagel (400 g) chromatographiert wird. Die Kolonne wird mit Benzol/Aethylacetat-Mischungen eluiert, wobei das Produkt mit Benzol/Aethylacetat (3:1) und reinem Aethylacetat eluiert wird. Nach Eindampfen dieser Lösungsmittelfraktionen erhält man das Amidzwischenprodukt 1-[(S)-4-(2-Hydroxy-3-N-mesylisopropylaminopropoxy)phenoxyacetyl]-4-phenylpiperazin als ein Oel.

Zu einer Portion dieses Materials (18 g; 35,7 mMol) in 600 ml trockenem Benzol gibt man tropfenweise (innert 25 Minuten) 113 ml einer 70%igen Lösung von Natrium-bis-(2-methoxyäthoxy)aluminiumhydrid in Benzol. Man erhitzt die Reaktionsmischung während 30 Minuten unter Rückfluss, kühlt dann ab und behandelt tropfenweise mit 120 ml einer 1N Natriumhydroxidlösung und anschliessend mit einem Liter Wasser. Die resultierende Mischung wird zweimal mit 1500 ml Benzol extrahiert; die organischen Schichten werden zweimal mit Wasser gewaschen. Die getrockneten (Natriumsulfat) organischen Schichten werden im Vakuum eingeengt,und man erhält einen farblosen Feststoff, Schmelzpunkt 88-95°. Die Kristallisation aus Methylenchlorid/Aether ergibt (S)-1-[2-(4-(2-Hydroxy-3-isopropylaminopropoxy)phenoxy)äthyl]-4-phenylpiperazin, Schmelz-

punkt 99-102°. Eine weitere Kristallisation aus dem gleichen Lösungsmittelsystem liefert eine analytisch reine Probe, Schmelzpunkt 102-104°; $[\alpha]_D^{25}$ -0,3° (c = 1,0% in Methanol).

Methode B

18,75 ml (75 mMol) einer 4N Natriumhydroxidlösung werden zu einer Lösung von 16,0 g (71,4 mMol) 1-(2-Chloräthyl)-4-phenylpiperazin und 16,8 g (75 mMol) (S)-1-(4-Hydroxyphenoxy)-2-hydroxy-3-isopropylaminopropan in 150 ml Dimethylsulfoxid gegeben; die Mischung wird während 3 Stunden bei 60° gerührt. Zur abgekühlten Lösung gibt man langsam 100 ml 1N Natriumhydroxidlösung und anschliessend 100 ml Wasser; der entstandene Feststoff wird durch Filtration gesammelt und mit Wasser gut gewaschen. Man löst den rohen Feststoff in Methylenchlorid (600 ml) auf, trocknet die Lösung über Kaliumcarbonat und dampft ein, wodurch man das rohe Produkt erhält. Dieses Material wird in 400 ml heissem Aethylacetat aufgelöst, mit 400 ml Hexan verdünnt und über Nacht bei 0-5° stehengelassen. Man sammelt den entstandenen Feststoff mittels Filtration und wäscht ihn mit 200 ml Aethylacetat/Hexan (1:1) und erhält so (S)-1-[2-(4-(2-Hydroxy-3-isopropylaminopropoxy)phenoxy)äthyl]-4-phenylpiperazin, Schmelzpunkt 102-104°.

23,3 g (56,4 mMol) (S)-1-[2-(4-(2-Hydroxy-3-isopropylaminopropoxy)phenoxy)äthyl]-4-phenylpiperazin werden zu einer heissen Lösung von Maleinsäure (13,34 g; 115 mMol) in 300 ml Methanol zugegeben. Heisses Aethylacetat wird portionenweise der unter Rückfluss kochenden Lösung so zugesetzt, das Volumen bei etwa 500 ml verbleibt, bis das Salz aus der Lösung auszukristallisieren beginnt. Man kühlt die Mischung ab und erhält mittels Filtration (S)-1-[2-(4-(2-Hydroxy-3-isopropylaminopropoxy)phenoxy)äthyl]-4-phenylpiperazin-bis-maleat, Schmelzpunkt 151-153°.

Eine Umkristallisation aus dem gleichen Lösungsmittel erhöht den Schmelzpunkt auf 153-154°; $[\alpha]_D^{25}$ -10,8° (c = 1,0% in Methanol).

Methode C

Eine Lösung von 7,8 g (22 mMol) (S)-1-[2-(4-(2,3-Epoxypropoxy)phenoxy)äthyl]-4-phenylpiperazin in 80 ml Methanol, welches 20 ml Isopropylamin enthält, wird während 2 Stunden auf 75° erwärmt. Man entfernt das Lösungsmittel im Vakuum und löst den Rückstand in 100 ml Methanol, welches 5,1 g (44 mMol) Maleinsäure enthält, auf. Die leicht trübe Lösung wird durch Celit filtriert und anschliessend auf etwa 60 ml eingeengt, worauf heisses Aethylacetat zugesetzt wird, bis die Kristallisation einsetzt. Das entstandene Salz wird mittels Filtration gesammelt und mit Aethylacetat gewaschen. Man erhält (S)-1-[2-(4-(2-Hydroxy-3-isopropylaminopropoxy)-phenoxy)äthyl]-4-phenylpiperazin-bis-maleat, Schmelzpunkt 150-151,5°.

Methode D

In einer inerten Atmosphäre werden 82 ml einer 70%igen Lösung von Natrium-bis-(2-methoxyäthoxy)aluminiumhydrid in Benzol tropfenweise zu einer gerührten Lösung von 19,2 g (39 mmol) (S)-1-[2-(4-(2-Hydroxy-3-N-mesyliso-propylaminopropoxy)phenoxy)äthyl]-4-phenylpiperazin in 100 ml Benzol zugegeben. Nachdem 3 bis 3 1/2 Stunden auf Rückflusstemperatur erhitzt worden ist, wird die Mischung in einem Eiswasserbad abgekühlt und das überschüssige Reagens durch vorsichtige tropfenweise Zugabe von Wasser zerstört. Die Mischung wird dann mit 2N Natriumhydroxidlösung und Methylenchlorid verdünnt, was zu einer Ausfällung von etwas unlöslichem anorganischem Feststoff führt. Die Feststoffe werden mittels Filtration durch Celit entfernt; das Filtrat wird mit Methylenchlorid (3 x) extrahiert. Die organischen Schichten werden ihrerseits mit 2N Natriumhydroxidlösung (2 x) und gesättigter Kochsalzlösung (1 x) gewaschen und dann vereinigt, über Kaliumcarbonat getrocknet und eingedampft, wodurch man (S)-1-[2-(4-(2-Hydroxy-3-isopropylaminopropoxy)phenoxy)-äthyl]-4-phenylpiperazin als kristallinen Feststoff erhält. Eine Lösung des Feststoffes in 125 ml heissem Aethylacetat wird mit warmem Hexan (125 ml) verdünnt; man lässt das Produkt langsam aus der Lösung kristallisieren. Die Filtration liefert (S)-1-[2-(4-(2-Hydroxy-3-isopropylaminopropoxy)phenoxy)äthyl]-4-phenylpiperazin, Schmelzpunkt 100-104$^{o}$.

13,9 g (33,6 mMol) (S)-1-[2-(4-(2-Hydroxy-3-isopropylaminopropoxy)phenoxy)äthyl]-4-phenylpiperazin werden zu einer heissen Lösung von 7,96 g (68,6 mMol) Maleinsäure in 100 ml Methanol zugegeben. Heisses Aethylacetat wird in Portionen zur unter Rückfluss kochenden Lösung gegeben, bis die Kristallisation des Salzes einsetzt. Nach dem Abkühlen wird die Mischung filtriert, und man erhält (S)-1-[2-(4-(2-Hydroxy-3-isopropylaminopropoxy)phenoxy)äthyl]-4-phenylpiperazin-bis-maleat, Schmelzpunkt 152-154$^{o}$.

## Beispiel 32

Eine Lösung von 7,8 g (22 mMol) (S)-1-[2-(4-(2,3-Epoxypropoxy)phenoxy)äthyl]-4-phenylpiperazin in 80 ml Methanol, welches 25 ml tert.-Butylamin enthält, wird 2 Stunden lang unter Rückfluss erhitzt. Das Lösungsmittel wird unter vermindertem Druck entfernt, wodurch ein öliger Rückstand entsteht, welcher beim Stehen lassen kristallisiert. Das rohe Produkt wird in 60 ml heissem Methanol, welches 5,1 g (44 mMol) Maleinsäure enthält, aufgelöst. Beinahe augenblicklich beginnt sich ein kristalliner Feststoff zu bilden. Die Mischung wird abgekühlt und filtriert, und man erhält (S)-1-[2-(4-(2-Hydroxy-3-tert.-butylaminopropoxy)phenoxy)äthyl]-4-phenylpiperazin-bis-maleat, Schmelzpunkt 180-182$^{o}$.

Eine zweite Fraktion, Schmelzpunkt 172-175$^{O}$, erhalten aus den Mutterlaugen, wird aus Methanol umkristallisiert, Schmelzpunkt 179-181$^{O}$.

Die erste Fraktion wird aus Methanol umkristallisiert, wodurch man ein analytisch reines Salz erhält, Schmelzpunkt 182-184$^{O}$; $[\alpha]_D^{25}$ -6,03$^{O}$ (c = 0,58% in Wasser).

1,3 g der zweiten Fraktion werden in 60 ml warmem Wasser aufgelöst und mit 5 ml 2N Natriumhydroxidlösung basisch gestellt. Der entstandene Feststoff wird mittels Filtration gesammelt, mit Wasser gewaschen und in Methylenchlorid aufgelöst. Die Methylenchloridlösung wird über Kaliumcarbonat getrocknet und eingedampft, wodurch man die freie Base als weissen Feststoff erhält. Die Kristallisation aus Aethylacetat liefert die reine Base als Monohydrat, Schmelzpunkt 82-84$^{O}$.

Beispiel 33

9,33 g 4N Natriumhydroxidlösung (37,3 mMol) werden zu einer Lösung von 8,4 g (37,3 mMol) (S)-1-(4-Hydroxyphenoxy)-2-hydroxy-3-isopropylaminopropan und 9,0 g (35,4 mMol) 1-(2-Chloräthyl)-4-(2-methoxyphenyl)piperazin in 75 ml Dimethylsulfoxid zugegeben und die Mischung wird 90 Minuten lang auf 60$^{O}$ erwärmt. Nach dem Abkühlen wird die Mischung mit 200 ml 0,5N Natriumhydroxidlösung verdünnt und unter Verwendung von Benzol (3 x 200 ml) extrahiert. Die organischen Extrakte werden ihrerseits mit Wasser (2 x 100 ml) gewaschen und dann vereinigt, über Kaliumcarbonat getrocknet und eingedampft.

8,6 g (74 mMol) Maleinsäure werden zu einer Lösung des Rückstandes in 40 ml Methanol zugegeben; Aethylacetat wird bis zur beginnenden Trübung zugesetzt. Die Verbindung kristallisiert nicht aus, weshalb die Lösungsmittel im Vakuum entfernt werden. Das rohe Salz wird in Aethanol (40 ml) aufgelöst; Aethylacetat wird wieder gerade bis

zur beginnenden Trübung zugesetzt. Der entstandene rohe Feststoff wird aus Aethanol/Aethylacetat umkristallisiert, wobei man im wesentlichen reines (S)-1-[2-(4-(2-Hydroxy-3-isopropylaminopropoxy)phenoxy)ethyl]-4-(2-methoxyphenyl)piperazin bis-maleat, Schmelzpunkt 105-110° erhält. Analytisch reines Salz [Schmelzpunkt 115-117°; $[\alpha]_D^{25}$ -10,4° (c = 1,0% in Methanol)] wird aus den gleichen Lösungsmitteln erhalten.

0,7 g des bis-Maleatsalzes werden in 5 ml Wasser aufgelöst und mit 3 ml einer 1N Natriumhydroxidlösung basisch gestellt. Die entstandene rohe freie Base wird abfiltriert und getrocknet. Die Kristallisation aus Aether ergibt analytisch reines Material, Schmelzpunkt 89-90,5°.

## Beispiel 34

Zu einer Lösung von 45,0 g 4-(2-Bromäthoxy)phenol (0,207 Mol) in 310 ml Aceton werden 54 ml Allylbromid (0,619 Mol) und 45,0 g wasserfreies Kaliumcarbonat (0,326 Mol) zugegeben. Man rührt die Reaktionsmischung 11 Stunden lang unter Rückfluss, kühlt sie ab und giesst sie in 2500 ml Wasser. Die Mischung wird zweimal mit Aether/Methylenchlorid (3:1) extrahiert; die organischen Schichten werden zweimal mit 1N Natriumhydroxidlösung und einmal mit Wasser gewaschen, über Natriumsulfat getrocknet und unter vermindertem Druck zur Trockene eingeengt, wobei man 1-(2-Bromäthoxy)-4-(2-propenyloxy)benzol als ein farbloses Oel erhält. Eine analytisch reine Probe von farblosen Kristallen erhält man durch Kristallisation aus Hexan, Schmelzpunkt 27-28°.

## Beispiel 35

Zu einer Lösung von 48,0 g 1-(2-Bromäthoxy)-4-(2-propenyloxy)benzol (0,186 Mol) in 960 ml Aceton und 125 ml Wasser werden 2,0 ml 70%ige Perchlorsäure zugegeben. Dieser gerührten Reaktionsmischung werden portionenweise

über eine Zeit von 12 Minuten 38,4 g (0,278 Mol) N-Bromacetamid zugesetzt. Die gerührte Reaktionsmischung wird während 2 bis 2 1/2 Stunden bei 22° gehalten und anschliessend mit festem Natriumbisulfit behandelt, bis eine negative Stärke/KJ-Reaktion erzielt wird. Als nächstes wird das Aceton im Vakuum entfernt und der Rückstand mit einem Liter Wasser verdünnt. Man extrahiert die Mischung zweimal mit Methylenchlorid und wäscht die organischen Schichten mit Wasser, trocknet über Natriumsulfat und engt unter vermindertem Druck zur Trockene ein, wodurch man das Bromhydrin-Zwischenprodukt als ein rotes Oel erhält.

Das rohe Oel wird in 1300 ml Methanol wieder aufgelöst und mit 375,0 ml 1N Natriumhydroxidlösung behandelt. Man lässt die Reaktionsmischung während 2 Stunden bei Raumtemperatur unter Argon stehen, worauf das Methanol im Vakuum entfernt und der Rückstand in einem Liter Wasser aufgelöst wird. Der entstandene Niederschlag wird filtriert und mit Wasser gut gewaschen. Er wird in Methylenchlorid wieder aufgelöst, über Natriumsulfat getrocknet und unter vermindertem Druck zur Trockene eingeengt, wodurch man einen schwach gelben Feststoff erhält. Der rohe Feststoff wird auf einer Kolonne von Florisil unter Verwendung von Benzol/Methylenchlorid (1:1) als Eluierungsmittel chromatographiert. Die eluierten Fraktionen werden vereinigt und zur Trockene eingeengt, wodurch man einen gelben Feststoff, Schmelzpunkt 50-61,5°, erhält. Der Feststoff wird dann durch eine Kolonne von Florisil in Benzol chromatographiert und wieder mit Benzol/Methylenchlorid (1:1) eluiert, wodurch man (R,S)-1-(2-Bromäthoxy)-4-(2,3-epoxypropoxy)benzol als einen farblosen Feststoff, Schmelzpunkt 56-62°, erhält. Die Kristallisation aus Aether/Hexan ergibt das Endproduckt in Form von farblosen Kristallen, Schmelzpunkt 59-62°. Eine zweite Fraktion kann aus den Mutterlaugen erhalten werden, Schmelzpunkt 58,5-61,5°. Die Kristallisation aus Aether/Hexan ergibt eine analytisch reine Probe, Schmelzpunkt 62-64°.

Beispiel 36

Zu einer gerührten Suspension von 15,0 g (R,S)-1-(2-Bromäthoxy)-4-(2,3-epoxypropoxy)benzol (0,055 Mol) in 150 ml Methanol werden 10,2 ml Isopropylamin (0,122 Mol) zugegeben. Die Reaktionsmischung wird während 3 Stunden in einer Argonatmosphäre gerührt und auf 55° erhitzt; anschliessend wird sie auf Raumtemperatur abgekühlt. Das Lösungsmittel wird im Vakuum entfernt, wodurch man ein schwach gelbes Oel erhält, welches beim Stehenlassen kristallisiert. Das Produkt wird zweimal aus Methylenchlorid/Hexan kristallisiert, wodurch man (R,S)-1-(2-Bromäthoxy)-4-[2-hydroxy-3-isopropylaminopropoxy]benzol in Form von farblosen Kristallen erhält, Schmelzpunkt 83-87,5°.

Die Kristallisation aus Methylenchlorid/Hexan liefert eine analytisch reine Probe, Schmelzpunkt 87-89,5°.

Beispiel 37

Zu einer Lösung von 200 g (1 Mol) p-Benzyloxyphenol in 2,5 l Dimethylsulfoxid werden 375 ml 4N Natriumhydroxidlösung und anschliessend 216 ml Epichlorhydrin zugegeben; die entstandene Mischung wird während 3 Stunden bei Raumtemperatur gerührt. Die Mischung wird in 6 Liter einer Eis/Wasser-Mischung gegossen und mit Methylenchlorid (3 x 1 Liter) extrahiert. Die organischen Extrakte werden ihrerseits mit Wasser (3 x 500 ml) gewaschen und anschliessend vereinigt, über Natriumsulfat getrocknet und zur Trockene eingedampft. Der entstandene rohe Feststoff wird aus Aether kristallisiert, wodurch man (R,S)-1-(4-Benzyloxyphenoxy)-2,3-epoxypropan in zwei Fraktionen erhält. Eine Lösung von 135 g (0,527 Mol) des obigen Epoxides in 1 Liter Methanol, enthaltend 135 ml Isopropylamin, wird 90 Minuten lang unter Rückfluss erhitzt. Man entfernt dann die Lösungsmittel unter vermindertem Druck und zerreibt den restlichen Feststoff mit Aether und erhält so (R,S)-1-(4-Benzyloxyphenoxy)-2-hydroxy-3-isopropylaminopropan, Schmelzpunkt

99-100°. Durch Einengen der Aetherextrakte erhält man eine zweite Fraktion, Schmelzpunkt 98-100°.

Eine kleine Probe (1 g ) der zweiten Fraktion wird aus Aether umkristallisiert und liefert eine analytisch reine Probe, Schmelzpunkt 100-101°.

Beispiel 38

(R,S)-1-(4-Benzyloxyphenoxy)-2-hydroxy-3-isopropylaminopropan (124 g ) in Methanol (1 Liter), enthaltend 5 g 10% Palladium auf Kohle, wird hydriert (21°; Atmosphärendruck). Nach einer Stunde ist die Aufnahme von Wasserstoff (insgesamt 9,6 Liter) im wesentlichen beendet. Man entfernt den Katalysator mittels Filtration (Celit) und engt das Filtrat im Vakuum zur Trockene ein. Der entstandene feste Niederschlag wird aus Aethanol kristallisiert und ergibt (R,S)-1-(4-Hydroxyphenoxy)-2-hydroxy-3-isopropylaminopropan, Schmelzpunkt 158-159°. Die Einengung der Mutterlauge liefert eine zweite Fraktion an Produkt.

Beispiel 39

Eine gerührte Mischung von 9,0 g (R,S)-1-(2-Bromäthoxy)-4-[2-hydroxy-3-isopropylaminopropoxy]benzol (0,027 Mol) und 9,0 g (0,055 Mol) N-Phenylpiperazin in 135 ml Aethanol wird 7 Stunden lang unter Argon unter Rückfluss erhitzt. Man kühlt anschliessend die Reaktionsmischung auf Raumtemperatur ab und entfernt das Lösungsmittel im Vakuum. Man löst den Rückstand in Benzol auf und wäscht zweimal mit 1N Natriumhydroxidlösung und einmal mit Wasser. Die getrocknete (Natriumsulfat) organische Schicht wird unter vermindertem Druck zur Trockene eingeengt und liefert einen rohen Feststoff, welcher zweimal aus Aether/Hexan kristallisiert wird, wobei man (R,S)-1-[2-(4-(2-Hydroxy-3-isopropylaminopropoxyphenoxy)ethyl]-4-phenylpiperazin als farblose Kristalle erhält, Schmelzpunkt 182,5-186,5°. Eine Kristallisation aus Methylenchlorid/Aether ergibt eine analytisch

reine Probe, Schmelzpunkt 87-89,5°.

Eine Lösung von 7,70 g (18,6 mMol) (R,S)-1-[2-(4-(2-Hydroxy-3-isopropylaminopropoxyphenoxy)ethyl]-4-phenylpiperazin in 150 ml absolutem Aethanol wird mit einer Lösung von 5,10 g (43,9 mMol) Maleinsäure in 80 ml absolutem Aethanol behandelt. Man erwärmt die Reaktionsmischung während 2 Minuten auf dem Dampfbad und lässt dann langsam auf Raumtemperatur abkühlen. Das Lösungsmittel wird im Vakuum entfernt, wodurch man einen schwach gelben Feststoff erhält, der zweimal aus Methanol/Aethylacetat kristallisiert wird und (R,S)-1-[2-(4-(2-Hydroxy-3-isopropylaminopropoxyphenoxy)ethyl]-4-phenylpiperazin-bis-maleat als schwach gelbe Kristalle ergibt, Schmelzpunkt 141,5-144°. Eine Kristallisation aus Methanol/Aethylacetat liefert eine analytisch reine Probe in Form von farblosen Kristallen, Schmelzpunkt 142-144°.

## Beispiel 40

Zu einer gerührten Mischung von (R,S)-1-(4-Hydroxyphenoxy)-2-hydroxy-3-isopropylaminopropan (4,5 g; 20 mMol) und 1-(2-Chloräthyl)-4-phenylpiperazin (4,6 g; 20,5 mMol) in 35 ml Dimethylsulfoxid werden 5,0 ml einer 4N Natriumhydroxidlösung (20 mMol) zugegeben. Nach dreistündigem Rühren bei 60° wird die Reaktionsmischung abgekühlt und zwischen Methylenchlorid und verdünnter Natriumhydroxidlösung verteilt. Man trennt die Schichten voneinander und extrahiert die wässrige Schicht mit Methylenchlorid (2 x). Die organischen Schichten werden ihrerseits anschliessend mit Wasser (2 x) gewaschen und vereinigt, über Kaliumcarbonat getrocknet und im Vakuum eingedampft.

Man löst das so erhaltene rohe (R,S)-1-[2-(4-(2-Hydroxy-3-isopropylaminopropoxyphenoxy)ethyl]-4-phenylpiperazin in 75 ml Methanol, enthaltend 4,6 g (40 mMol) Maleinsäure, und gibt portionenweise bis zur beginnenden Trübung Aethylacetat zur siedenden Lösung. Nach dem Abkühlen

der Lösung wird der entstandene Feststoff mittels Filtration gesammelt, wodurch man (R,S)-1-[2-(4-(2-Hydroxy-3-isopropylaminopropoxyphenoxy)ethyl]-4-phenylpiperazin-bis-maleat erhält. Eine Umkristallisation aus Methanol/Aethylacetat liefert ein reines Salz, Schmelzpunkt 143,5-144°.

## Beispiel 41

Eine Lösung von 25 g (0,128 Mol) 6-Bromhexansäure und 12,7 g (0,125 Mol) Triäthylamin in 150 ml Aether wird auf 0° abgekühlt und tropfenweise mit 28,5 g (0,263 Mol) Aethylchlorformiat behandelt. Die Reaktionsmischung wird eine Stunde lang gerührt und anschliessend filtriert. Zum gekühlten (0°) Filtrat wird eine Lösung von 21 g (0,129 Mol) N-Phenylpiperazin in 150 ml Aether zugegeben. Man lässt die Reaktionsmischung anschliessend während 30 Minuten sich auf Raumtemperatur erwärmen und wäscht dann einmal mit 1N Natriumhydroxidlösung und einmal mit Wasser. Die getrockneten (Natriumsulfat) organischen Schichten werden im Vakuum eingeengt und liefern ein gelbes Oel, welches in Benzol aufgelöst und an Silicagel (500 g) chromatographiert wird. Die Kolonne wird mit Benzol/Aethylacetat-Mischungen eluiert, wobei das Produkt mit den Benzol/Aethylacetat-Mischungen (8,5:1,5) eluiert wird. Man dampft diese vereinigten Mischungen ein und erhält 1-(6-Bromhexanoyl)-4-phenylpiperazin.

Dieses Material (4,55 g, 13,2 mMol) wird zu einer Lösung von 4,0 g (13,2 mMol) (S)-1-(4-Hydroxyphenoxy)-2-hydroxy-3-N-mesylisopropylaminopropan in 30 ml Dimethylsulfoxid zugegeben. Man gibt 3,33 ml 4N Natriumhydroxidlösung zu dieser gerührten Lösung zu und erhitzt die Reaktionsmischung während 30 Minuten auf 70°. Sie wird dann abgekühlt, mit Wasser verdünnt und 3 x mit Aethylacetat extrahiert. Die organischen Schichten werden einmal mit 1N Natriumhydroxidlösung und zweimal mit Wasser gewaschen, über Natriumsulfat getrocknet und eingedampft, wodurch man das Amidzwischenprodukt 1-[(S)-4-(2-Hydroxy-3-N-mesylisopropylaminopropoxy)phenoxyhexanoyl]-4-phenylpiperazin

als ein Oel erhält.

Zu diesem Material (7,4 g, 13,2 mMol) in 62 ml trockenem Benzol werden tropfenweise 42 ml 70%iges Natrium-bis-(2-methoxyethoxy)aluminiumhydrid in Benzol zugegeben. Man erhitzt die Mischung 80 Minuten lang unter Rückfluss, kühlt sie dann ab und behandelt sie mit 40 ml 2N Natriumhydroxidlösung und anschliessend mit 100 ml Wasser. Man extrahiert die Mischung 3 x mit Benzol und wäscht die organischen Schichten zweimal mit Wasser, trocknet über Natriumsulfat und dampft ein. Der Rückstand wird aus Methylenchlorid/Aether kristallisiert und liefert (S)-1-[6-(4-(2-Hydroxy-3-isopropylaminopropoxyphenoxy)hexyl]-4-phenylpiperazin, Schmelzpunkt 75-76°. Eine Kristallisation aus Aceton/Hexan ergibt eine analysenreine Probe, Schmelzpunkt 78-79°; $[\alpha]_D^{25}$ -3,52° (c = 1,0% in Chloroform).

## Beispiel 42

Eine Lösung von 4,265 g (9 mMol) (S)-1-[6-(4-(2-Hydroxy-3-isopropylaminopropoxyphenoxy)hexyl]-4-phenylpiperazin in 60 ml Aethanol wird mit 3,5 ml 5,13N äthanolischem Chlorwasserstoff behandelt. Der entstandene Feststoff wird durch Filtration gesammelt und mit Aethanol gewaschen, und man erhält (S)-1-[6-(4-(2-Hydroxy-3-isopropylaminopropoxyphenoxy)-hexyl]-4-phenylpiperazin-dihydrochlorid, Schmelzpunkt 183-185°. Eine Kristallisation aus Aethanol ergibt eine analysenreine Probe, Schmelzpunkt 183-184°; $[\alpha]_D^{25}$ -11,4° (c = 0,5% in Methanol).

## Beispiel 43

Zu einer gerührten Lösung von 13,26 g 11-Bromundecansäure (50 mMol) in 75 ml Aether werden 6,9 ml (50 mMol) Triäthylamin zugegeben. Man kühlt die gerührte Lösung auf 0° ab und gibt tropfenweise 4,80 ml Aethylchlorformiat (50 mMol) zu. Die Mischung wird eine Stunde lang bei 0° gerührt; der entstandene Niederschlag wird abfiltriert und

mit Aether gewaschen.

Man kühlt das Filtrat auf 0° ab und gibt eine Lösung von 8,10 g (50 mMol) N-Phenylpiperazin in 25,0 ml Aether unter Rühren zu. Ein Niederschlag wird gebildet, und man entfernt das Eisbad. Das Rühren wird während weiteren 25 Minuten bei Raumtemperatur fortgesetzt; dann wird das Material mittels Filtration gesammelt und mit Aether gut gewaschen, wodurch man farblose Kristalle, Schmelzpunkt 70-74°, erhält. Eine Kristallisation aus Methylenchlorid/Aether ergibt 1-(11-Bromundecanoyl)-4-phenylpiperazin als farblose Kristalle, Schmelzpunkt 73-75,5°. Eine Kristallisation aus Methylenchlorid/Aether liefert eine analysenreine Probe, Schmelzpunkt 73,5-75,5°.

Zu einer gerührten Lösung von 3,03 g (10 mMol) (S)-1-(4-Hydroxyphenoxy)-2-hydroxy-3-N-mesylisopropylaminopropan und 4,09 g 1-(11-Bromundecanoyl)-4-phenylpiperazin (10 mMol) in 20 ml Dimethylsulfoxid werden 3,0 ml (12 mMol) 4N Natriumhydroxidlösung zugegeben. Die Reaktionsmischung wird gerührt und unter Argon während 35 Minuten auf 75-78° erwärmt; anschliessend wird sie auf Raumtemperatur abgekühlt und mit 100 ml kaltem Wasser verdünnt. Die Mischung wird zweimal mit Aethylacetat extrahiert; die organischen Schichten werden einmal mit 1N Natriumhydroxidlösung und zweimal mit Wasser gewaschen und über Natriumsulfat getrocknet. Das Lösungsmittel wird unter vermindertem Druck zur Trockene eingeengt und man erhält ein farbloses Oel, welches aus 1-[(S)-4-(2-Hydroxy-3-N-mesylisopropylamino-propoxyphenoxy)undecanoyl]-4-phenylpiperazin besteht.

Zu einer gerührten Lösung von 6,50 g dieses Oels (10,4 mMol) in 55,0 ml Benzol werden 36,6 ml 70%iges Natrium-bis-(2-methoxyäthoxy)aluminiumhydrid in Benzol zugegeben. Die Reaktionsmischung wird während 2 bis 2 1/4 Stunden unter Rückfluss (Argonatmosphäre) erhitzt; dann wird sie auf Raumtemperatur abgekühlt und mit 35 ml 2N Natriumhydroxidlösung und 100 ml Wasser behandelt. Man extrahiert

die Mischung zweimal mit Benzol, wäscht die organischen Schichten zweimal mit Wasser, trocknet über Kaliumcarbonat und engt unter vermindertem Druck zur Trockene ein, wodurch man einen farblosen Feststoff erhält. Das Material wird aus Aceton kristallisiert, und man erhält (S)-1-[11-(4-(2-Hydroxy-3-isopropylaminopropoxyphenoxy)undecanyl]-4-phenylpiperazin in Form von farblosen Kristallen, Schmelzpunkt 99-103°. Eine Kristallisation aus Aceton ergibt eine analysenreine Probe, Schmelzpunkt 101,5-104°; $[\alpha]_D^{25}$ -2,82° (c = 1,13% in Chloroform).

## Beispiel 44

Zu einer warmen (40°) Lösung von 2,83 g (5,2 mMol) (S)-1-[11-(4-(2-Hydroxy-3-isopropylaminopropoxyphenoxy)-undecanyl]-4-phenylpiperazin in 95 ml absolutem Aethanol werden unter Rühren rasch 2,03 ml (10,4 mMol) 5,13N äthanolische Chlorwasserstofflösung zugetropft. Die Mischung wird dann auf 0° abgekühlt; die entstehenden farblosen Kristalle werden durch Filtration gesammelt, und man erhält (S)-1-[11-(4-(2-Hydroxy-3-isopropylaminopropoxyphenoxy)undecanyl]-4-phenylpiperazin-dihydrochlorid, Schmelzpunkt 193-196°. Eine Kristallisation aus Aethanol ergibt eine analysenreine Probe, Schmelzpunkt 193,5-196°; $[\alpha]_D^{25}$ -7,52° (c = 0,5% in Methanol).

## Beispiel 45

39.9 g (0,362 Mol) Brenzkatechin werden zu einer Lösung von 14,5 g Natriumhydroxid (362 mMol) in 45 ml Wasser zugegeben; die Mischung wird unter Argon gerührt. Die pastenförmige Mischung wird mit 100 ml Dimethylsulfoxid verdünnt; nach 10 Minuten wird eine Lösung von 52,3 g (0,181 Mol) (S)-1-Mesyloxy-2-hydroxy-3-N-mesylisopropylaminopropan in 100 ml Dimethylsulfoxid zugesetzt. Die Lösung wird während 2 bis 2 1/2 Stunden bei 80° unter Argon gerührt; dann wird sie abgekühlt und mit 400 ml 1N

Natriumhydroxidlösung verdünnt. Man extrahiert die Lösung mit Methylenchlorid (3 x 250 ml) und wäscht die organischen Extrakte zurück (1 x ) mit verdünnter Natriumhydroxidlösung. Die vereinigten basischen wässrigen Extrakte werden unter Verwendung von 70 ml konzentrierter Salzsäure sauer gestellt und mit Methylenchlorid (2 x 500 ml) extrahiert. Die organischen Extrakte werden anschliessend ihrerseits mit Wasser (5 x 500 ml) gewaschen, dann vereinigt, über Natriumsulfat getrocknet und eingedampft, wodurch man im wesentlichen reines (S)-1-(2-Hydroxyphenoxy)-2-hydroxy-3-N-mesylisopropylaminopropan als ein Oel erhält.

## Beispiel 46

Eine Mischung aus 15 g (49,5 mMol) (S)-1-(2-Hydroxyphenoxy)-2-hydroxy-3-N-mesylisopropylaminopropan, 30,6 g (150 mMol) 1,3-Dibrompropan und 10,6 g Kaliumcarbonat in 60 ml Aceton wird über Nacht unter Rückfluss gerührt. Die Mischung wird mit Wasser verdünnt und mit Methylenchlorid extrahiert (4 x). Die Methylenchloridextrakte werden ihrerseits mit 1N Natriumhydroxidlösung gewaschen, dann vereinigt, über Natriumsulfat getrocknet und im Vakuum zur Trockene eingeengt, wobei man 18,5 g eines Oels erhält. Das Oel wird an 200 g Silicagel, präpariert in Hexan, chromatographiert. Die Kolonne wird der Reihe nach mit Hexan, Benzol, Benzol/Aether (19:1), Benzol/Aether (9:1) und Benzol/Aether (3:1) eluiert. Die mit Benzol/Aether (9:1) und (3:1) eluierten Fraktionen werden vereinigt und zur Trockene eingedampft, und man erhält chromatographisch reines (S)-1-[2-(3-Brompropoxy)-phenoxy]-2-hydroxy-3-N-mesylisopropylaminopropan, Schmelzpunkt 95-96°. Eine Kristallisation aus Aethylacetat/Hexan liefert die reine Verbindung, Schmelzpunkt 98-99°.

Eine analysenreine Probe, Schmelzpunkt 98-100°; $[\alpha]_D^{25}$ -4,99° (c = 1,0% in Chloroform) wird aus einem kleineren Versuch unter Verwendung des gleichen Lösungsmittelsystems erhalten.

## Beispiel 47

Eine Mischung aus 10,5 g (24,7 mMol) (S)-1-[2-(3-Brompropoxy)phenoxy]-2-hydroxy-3-N-mesylisopropylaminopropan und N-Phenylpiperazin (4,42 g; 27,3 mMol) in 50 ml Aethanol wird während 3 Tagen bei Raumtemperatur stehengelassen. Das Dünnschichtchromatogramm zeigt, dass die Reaktion zu etwa 50% abgelaufen ist; das Verhältnis von Ausgangsmaterial und Produkt ändert sich auch nicht nach 4 Stunden Erhitzen unter Rückfluss. 4,0 ml 4N Natriumhydroxidlösung werden zugesetzt; die Mischung wird 2 Stunden lang erhitzt; dann werden weitere 1,5 ml einer 4N Natriumhydroxidlösung [insgesamt werden 22 Aequivalente eingesetzt (95% der Theorie)] zugegeben. Nach einem weiteren 60-minütigem Erhitzen unter Rückfluss wird die Mischung abgekühlt und eingedampft. Der Rückstand wird mit Wasser verdünnt und mit Aethylacetat extrahiert. Der Aethylacetatextrakt wird über Kaliumcarbonat getrocknet und eingedampft, wodurch man ein Oel erhält. Das Oel wird über 200 g Silicagel chromatographiert. Die Kolonne wird mit Benzol und Benzol/Aethylacetat-Mischungen (19:1; 9:1; 3:1 und 1:1) eluiert; anschliessend wird das Produkt aus der Kolonne unter Verwendung von Aethylacetat eluiert, und man erhält (S)-1-[3-(2-(2-Hydroxy-3-N-mesylisopropylaminopropoxy-phenoxy)-propyl]-4-phenylpiperazin. Eine Kristallisation aus Aether liefert das Produkt in zwei Fraktionen, Schmelzpunkt 84-85$^{o}$ bzw. 82-84$^{o}$.

Eine Umkristallisation einer geringen Menge aus Aether (2 x ) liefert analysenreines Material, Schmelzpunkt 85-86$^{o}$; $[\alpha]_D^{25}$ -1,0$^{o}$ (c = 1,0% in Chloroform).

## Beispiel 48

22 ml 60%iges Natrium-bis-(2-methoxyäthoxy)aluminiumhydrid in Benzol werden zuerst tropfenweise und dann nach dem Ende der Blasenentwicklung rascher zu einer gerührten Lösung von 6,2 g (12,25 mMol) (S)-1-[3-(2-(2-Hydroxy-3-

N-mesylisopropylaminopropoxyphenoxy)propyl]-4-phenylpiperazin in 100 ml trockenem Benzol zugegeben. Die Mischung wird 5 Stunden lang unter Argon unter Rückfluss erhitzt; dann lässt man sie über Nacht bei Raumtemperatur stehen. Der Reagensüberschuss wird durch tropfenweise Zugabe von 25 ml 2N Natriumhydroxidlösung zersetzt; anschliessend wird mit Wasser (25 ml) verdünnt. Man trennt die Benzolschicht ab und extrahiert die wässrige Schicht mit Benzol (2 x). Die Benzolschichten werden ihrerseits mit 2N Natriumhydroxidlösung (1 x) und gesättigter Kochsalzlösung (2 x) gewaschen. Die vereinigten Extrakte werden über Kaliumcarbonat getrocknet und unter vermindertem Druck eingedampft,und man erhält das rohe Produkt. Die Kristallisation aus Aceton/Hexan ergibt Feststoffe in 2 Portionen, welche beim Umkristallisieren aus dem gleichen Lösungsmittelsystem reines (S)-1-[3-(2-(2-Hydroxy-3-isopropylaminopropoxyphenoxy)-propyl]-4-phenylpiperazin, Schmelzpunkt 87-88$^{O}$ liefern.

3,5 g (S)-1-[3-(2-(2-Hydroxy-3-isopropylaminopropoxyphenoxy)propyl]-4-phenylpiperazin in 150 ml absolutem Aethanol werden mit 2,2 Mol Aequivalenten ätherischem Chlorwasserstoff behandelt; anschliessend wird die Lösung mit 400 ml Aether verdünnt. Der entstandene kristalline Feststoff wird mittels Filtration gesammelt,und man erhält (S)-1-[3-(2-(2-Hydroxy-3-isopropylaminopropoxyphenoxy)-propyl]-4-phenylpiperazin-dihydrochlorid, Schmelzpunkt 192-193$^{O}$.

Durch Umkristallisation aus Aethanol erhält man analysenreines Material, Schmelzpunkt 192-193$^{O}$; $[\alpha]_D^{25}$ -5,39$^{O}$ (c = 1,0% in Wasser).

Beispiel 49

8,0 ml 4N Natriumhydroxidlösung (32 mMol) werden einer Lösung von 9,5 g (31,35 mMol) (S)-1-(2-Hydroxyphenoxy)-2-hydroxy-3-N-mesylisopropylaminopropan und 10,9 g (32 mMol) 1-(6-Bromhexanoyl)-4-phenylpiperazin in 70 ml

Dimethylsulfoxid zugesetzt; die Mischung wird während 50 Minuten unter Argon auf 80° erhitzt. Man kühlt die Mischung ab und giesst in 500 ml Wasser; es wird mit Benzol extrahiert (2 x 250 ml). Die Benzolextrakte werden ihrerseits mit 1N Natriumhydroxidlösung (2 x) und Wasser (1 x) gewaschen und dann vereinigt, über Kaliumcarbonat getrocknet und eingedampft, wodurch man ein Oel erhält. Das Oel wird an 200 g Silicagel, präpariert in Benzol, chromatographiert. Die mit Aethylacetat/Benzol (1:1 and 3:2) eluierten Fraktionen werden vereinigt und eingedampft, wodurch man im wesentlichen reines (S)-1-[6-(2-(2-Hydroxy-3-N-mesylisopropylaminopropoxy)phenoxy)hexanoyl]-4-phenylpiperazin als ein Oel erhält.

Eine kleine Probe wird zur Analyse mittels Dünnschichtchromatographie gereinigt.

## Beispiel 50

In einer inerten Atmosphäre werden 70 ml 70%iges Natrium-bis-(2-methoxyäthoxy)aluminiumhydrid in Benzol zuerst vorsichtig und dann schneller zu einer gerührten Lösung von 11,4 g (20,28 mMol) (S)-1-[6-(2-(2-Hydroxy-3-N-mesylisopropylaminopropoxy)phenoxy)hexanoyl]-4-phenylpiperazin in 100 ml trockenem Benzol zugegeben. Anschliessend wird die Reaktionsmischung erwärmt und während 90 Minuten unter Rückfluss gerührt. Zur gekühlten Reaktionsmischung gibt man dann vorsichtig 50 ml 2N Natriumhydroxidlösung und anschliessend 100 ml Wasser und 300 ml Benzol zu. Die wässrige Schicht wird abgetrennt und mit Benzol (2 x 300 ml) extrahiert. Die Benzolschichten werden dann mit Wasser (2 x 100 ml) zurückgewaschen. Die vereinigten organischen Extrakte werden über Kaliumcarbonat getrocknet und eingeengt; man erhält ein Oel, welches beim Verdampfen im Vakuum aus Aceton erstarrt. Der Rückstand wird aus Aceton/Hexan kristallisiert und ergibt reines (S)-1-[6-(2-(2-Hydroxy-3-isopropylaminoaminopropoxy)phenoxy)-hexyl]-4-phenylpiperazin, Schmelzpunkt 69-71°. Eine Um-

kristallisation aus Aceton/Hexan ergibt eine analysenreine Probe, Schmelzpunkt 69-71$^{\circ}$; $[\alpha]_D^{25}$+3,5$^{\circ}$ (c = 1,0% in Chloroform).

4,0 g (8,55 mMol) (S)-1-[6-(2-(2-Hydroxy-3-isopropylaminopropoxy)phenoxy)hexyl]-4-phenylpiperazin und 0,99 g (8,55 mMol) Fumarsäure werden in 25 ml Methanol bei Raumtemperatur aufgelöst; dann wird die Lösung mit 50 ml Aethylacetat verdünnt. Der entstandene Niederschlag, welcher langsam aus der Lösung kristallisiert, wird mittels Filtration gesammelt und mit Aethylacetat gewaschen; man erhält (S)-1-[6-(2-(2-Hydroxy-3-isopropylaminopropoxy)phenoxy)-hexyl]-4-phenylpiperazin-fumarat, Schmelzpunkt 163-165$^{\circ}$.

Eine Umkristallisation aus Methanol/Aethylacetat liefert das analysenreine Salz, Schmelzpunkt 164-165$^{\circ}$.

## Beispiel 51

Eine Lösung von 5,00 g (0,0260 Mol) 4-Allyloxyphenylessigsäure in 25 ml Aceton und 8 ml Wasser wird in einem Eisbad gekühlt und mit 5,00 g (0,0362 Mol) N-Bromsuccinimid behandelt. Man lässt die erhaltene Mischung sich langsam auf Raumtemperatur erwärmen; nach 2 1/2 Stunden werden weitere 0,5 g N-Bromsuccinimid zugesetzt. Nach einer Gesamtzeit von 3 1/2 Stunden wird die Reaktionsmischung mit 150 ml Wasser verdünnt; eine geringe Menge Zitronensäure wird zugesetzt. Das Produkt wird mit Methylenchlorid extrahiert; nach der Verdampfung des Lösungsmittels erhält man einen rohen Feststoff. Die Umkristallisation aus Aethylacetat/Hexan liefert eine Mischung der Bromhydrine der 4-Allyloxyphenylessigsäure, Schmelzpunkt 85-99$^{\circ}$. Mehrere Kristallisationen aus Aethylacetat/Hexan ergeben eine analysenreine Probe von einem der Isomeren, Schmelzpunkt 128-133$^{\circ}$.

## Beispiel 52

### Methode A

Eine Lösung von 1,66g (5,74 mMol) der Mischung der Bromhydrine der 4-Allyloxyphenylessigsäure, erhalten in Beispiel 51,in 100 ml Methanol und 5 ml 4N Natriumhydroxidlösung wird während 2 Stunden bei Raumtemperatur gerührt. Die entstandene Mischung wird mit 1M Zitronensäure auf pH 3 verdünnt und mit Methylenchlorid extrahiert. Die Kristallisation des rohen Produktes aus Methanol/Wasser ergibt (R,S)-4-(2,3-Epoxypropoxy)phenylessigsäure. Eine Umkristallisation aus Methanol/Aether liefert eine analysenreine Probe, Schmelzpunkt 73-77$^{O}$.

### Methode B

Eine Lösung von 2,00 g (10,4 mMol) 4-Allyloxyphenylessigsäure und 1,6 g N-Bromsuccinimid in 10 ml Aceton und 4 ml Wasser wird bei 0$^{O}$ während 30 Minuten gerührt; anschliessend lässt man auf Raumtemperatur erwärmen. Nach einer Stunde werden 0,2 g N-Bromsuccinimid zugesetzt; nach einer Gesamtzeit von 2 1/2 Stunden kühlt man die Mischung in einem Eisbad ab und gibt 5,5 ml 4N Natriumhydroxidlösung zu. Die Mischung wird auf Raumtemperatur erwärmt, eine Stunde lang gerührt und mit Wasser, Eis und 10%iger Zitronensäurelösung verdünnt. Die Lösung wird mit Methylenchlorid extrahiert; die Methylenchloridschichten ergeben (R,S)-4-(2,3-Epoxypropoxy)phenylessigsäure als einen leicht beigen Feststoff, Schmelzpunkt 72-76$^{O}$.

## Beispiel 53

Eine mechanisch gerührte Suspension von 101,54 g (0,528 Mol) 4-Allyloxyphenylessigsäure, 70 ml (1,12 Mol) Methyljodid und 100 g (0,756 Mol) Kaliumcarbonat in 210 ml Hexamethylphosphoramid lässt man über Nacht bei Raumtemperatur stehen. Man verdünnt die erhaltene Mischung mit einem Liter Aether und wäscht die ätherische Lösung mit 3 x 300 ml

Wasser und einmal 300 ml gesättigter Kochsalzlösung. Man engt die ätherische Lösung ein und destilliert den Rückstand, wodurch Methyl-4-allyloxyphenylacetat als farbloses Oel, Sdp. 120-135°/0,05 mmHg, erhalten wird.

## Beispiel 54

### Methode A

Zu einer eiskalten Lösung von 4,50 g (0,0216 Mol) (R,S)-4-(2,3-Epoxypropoxy)phenylessigsäure in 20 ml trockenem Methanol wird ein Ueberschuss an ätherischem Diazomethan zugesetzt. Die Verdampfung der entstandenen Lösung zur Trockene ergibt (R,S)-Methyl-4-(2,3-epoxypropoxy)phenylacetat.

### Methode B

Eine Lösung von 25,25 g (0,122 Mol) Methyl-4-allyloxyphenylacetat und 62 g (0,359 Mol) m-Chlorperbenzoesäure in 200 ml Methylenchlorid wird bei Raumtemperatur gerührt. Nach 24 Stunden wird die Reaktionsmischung in 200 ml Wasser gegossen und mit 45%iger Natriumhydroxidlösung basisch gestellt. Man extrahiert die wässrige Schicht mit 2 x 300 ml Methylenchlorid und wäscht die vereinigten organischen Schichten mit 2 x 200 ml Wasser und 1 x 200 ml gesättigter Kochsalzlösung. Das Rohprodukt wird unter Vakuum destilliert und liefert (R,S)-Methyl-4-(2,3-epoxypropoxy)phenylacetat, Sdp. 180-185°/0,1 mmHg. Eine Probe wird mittels präparativer Schichtchromatographie gereinigt und destilliert.

## Beispiel 55

Eine Lösung von 55,29 g (0,249 Mol) (R,S)-Methyl-4-(2,3-epoxypropoxy)phenylacetat und 27,0 ml (0,334 Mol) Isopropylamin in 200 ml Methanol wird über Nacht bei Raumtemperatur gerührt. Man engt die Mischung ein und löst den Rückstand in einem Ueberschuss an 1M Zitronensäure auf.

Die wässrige Lösung wird mit 3 x 100 ml Methylenchlorid gewaschen und durch Zugabe von 45%iger Natriumhydroxidlösung basisch gestellt,worauf man mit 4 x 100 ml Methylenchlorid extrahiert. Die vereinigten Extrakte werden mit gesättigter Kochsalzlösung gewaschen und über Kaliumcarbonat getrocknet. Die Verdampfung liefert Methyl 4-(2-hydroxy-3-isopropylaminopropoxy)phenylacetat, Schmelzpünkt 72-74°. Eine Umkristallisation aus Aether/Hexan ergibt eine analytisch reine Probe, Schmelzpunkt 72-74°.

## Beispiel 56

Eine Suspension von 9,85 g (0,060 Mol) Phenylpiperazin, 11,6 g (0,060 Mol) 6-Bromhexanamid und 6,35 g (0,060 Mol) Natriumcarbonat in 90 ml Toluol wird über Nacht unter Rückfluss erhitzt. Beim Abkühlen wird die Mischung filtriert und mit Wasser gewaschen. Die Verdampfung und Kristallisation des rohen Produktes aus Methylenchlorid/Hexan liefert analytisch reines 6-(4-Phenyl-1-piperazinyl)hexanamid, Schmelzpunkt 129-130°.

## Beispiel 57

Eine Suspension von 13,1 g (0,0795 Mol) Phenylpiperazin, 20,86 g (0,0795 Mol) 11-Bromundecanamid und 8,45 g (0,0795 Mol) Natriumcarbonat in 120 ml Toluol wird über Nacht unter Rückfluss erhitzt. Beim Abkühlen scheidet sich ein Feststoff ab; er wird gesammelt und mit Wasser zerrieben,und man erhält 11-(4-Phenyl-1-piperazinyl)undecanamid, Schmelzpunkt 128-129°. Zwei Umkristallisationen aus Aethanol/Aethylacetat/Hexan liefern eine analysenreine Probe, Schmelzpunkt 129-130°.

## Beispiel 58

Eine Lösung von 38,3 g (0,139 Mol) 6-(4-Phenyl-1-piperazinyl)hexanamid in 400 ml Tetrahydrofuran wird mit 500 ml 1M Boran in Tetrahydrofuran behandelt. Die Lösung

wird während 8 Stunden unter Rückfluss erhitzt und dann in einem Eisbad gekühlt; 42 ml 12N Salzsäure werden vorsichtig dazugegeben. Man verdampft das überschüssige Tetrahydrofuran, stelltdie wässrige Lösung mit Kaliumcarbonat basisch und extrahiert mit Methylenchlorid. Man dampft die organischen Schichten ein und erhält ein aus 1-(6-Aminohexyl)-4-phenylpiperazin bestehendes Oel, welches einen einzelnen Fleck auf dem Silicagel-Dünnschichtchromatogramm ergibt und ohne weitere Reinigung in der nächsten Stufe verwendet wird. Eine Portion wird zusätzlich in Form des Dihydrochloridsalzes charakterisiert; es kristallisiert aus Aethanol, Schmelzpunkt 251-253°.

## Beispiel 59

1-(11-Aminoundecyl)-4-phenylpiperazin wird in der gleichen Weise wie in Beispiel 58 beschrieben,hergestellt; man geht jedoch von 11,2 g (0,0322 Mol) 11-(4-Phenyl-1-piperazinyl)undecanamid aus und erhält ein Oel. Das Dihydrochloridsalz kristallisiert aus Aethanol, Schmelzpunkt 197-202°.

## Beispiel 60

Eine gut durchgerührte Mischung aus 5,00 g (0,0178 Mol) Methyl-4-(2-hydroxy-3-isopropylaminopropoxy)phenylacetat und 4,00 g (0,0190 Mol) 1-(2-Aminoäthyl)-4-phenylpiperazin wird über Nacht auf eine Badtemperatur von 140-145° erhitzt. Der entstandene Feststoff wird mit Aether/Benzol zerrieben,und man erhält 4-[(2-Hydroxy-3-isopropylaminopropoxy)phenyl]-N-[2-(4-phenyl-1-piperazinyl)äthyl]acetamid als einen gelbbraunen Feststoff, Schmelzpunkt 100-106°. Die Umwandlung in das Hydrochloridsalz ergibt 4-[(2-Hydroxy-3-isopropylaminopropoxy)phenyl]-N-[2-(4-phenyl-1-piperazinyl)äthyl] acetamid-dihydrochlorid, Schmelzpunkt 173-177°. Zwei Umkristallisationen aus Aethanol/Aethylacetat ergeben eine analysenreine Probe, Schmelzpunkt 178-181°.

Beispiel 61

Eine gut durchgerührte Mischung aus 10,0 g (0,0384 Mol) 1-(6-Aminohexyl)-4-phenylpiperazin und 10,0 g (0,0355 Mol) Methyl-4-(2-hydroxy-3-isopropylaminopropoxy)phenyl acetat wird über Nacht auf eine Badtemperatur von 140° erhitzt. Man löst den rohen braunen Feststoff in Methylenchlorid; Aether wird zur Ausfällung eines wachsartigen Feststoffes zugesetzt. Dieses Material wird durch 50 g basisches Aluminiumoxid, Aktivität III geleitet, wobei man mit 1% Methanol/Aethylacetat eluiert und einen farblosen Wachs erhält, welches einen einzelnen Fleck auf dem Dünnschichtchromatogramm zeigt. Zur Umwandlung in das Hydrochloridsalz werden 9,1 g des oben erhaltenen 4-[(2-Hydroxy-3-isopropylaminopropoxy)phenyl]-N-[6-(4-phenyl-1-piperazinyl)hexyl]acetamides in Portionen von 6 g und 3,1 g geteilt; die 6 g Portion wird mit einem Ueberschuss an Salzsäure behandelt und unter Hochvakuum zur Trockene eingedampft. Man nimmt dieses Oel in Alkohol auf und gibt die 3,1 g Portion zu. Die entstandene Lösung wird eingeengt und der Rückstand aus Aethanol/Aether kristallisiert. Man erhält 4-[(2-Hydroxy-3-isopropylaminopropoxy)phenyl]-N-[6-(4-phenyl-1-piperazinyl)hexyl]acetamid-dihydrochlorid, Schmelzpunkt 124-126° (Zers.). Eine analysenreine Probe wird aus Aethanol/Aether erhalten, Schmelzpunkt 123-126° (Zers.).

Beispiel 62

Eine gut durchgerührte Mischung aus 9,4 g (0,030 Mol) 1-(11-Aminoundecyl)-4-phenylpiperazin und 8,45 g (0,030 Mol) Methyl-4-(2-hydroxy-3-isopropylaminopropoxy)-phenylacetat wird über Nacht auf eine Badtemperatur von 140° erhitzt. Der entstandene braune Feststoff wird aus Methylenchlorid / Aether kristallisiert, und man erhält 4-[(2-Hydroxy-3-isopropylaminopropoxy)phenyl]-N-[11-(4-phenyl-1-piperazinyl) undecyl]acetamid, Schmelzpunkt 107-109°. Eine zweite Fraktion, Schmelzpunkt 101-105°,

wird aus dem Filtrat erhalten. Die beiden Fraktionen werden vereinigt und mit Salzsäure sauer gestellt; man erhält einen Feststoff, welcher aus Isopropanol umkristallisiert wird und 4-[(2-Hydroxy-3-isopropylaminopropoxy)phenyl]-N-[11-(4-phenyl-1-piperazinyl)undecanyl]acetamid-dihydrochlorid, Schmelzpunkt 146-150°, liefert.

## Beispiel 63

Natrium (2,3 g; 0,1 Mol) wird in 500 ml wasserfreiem Methanol aufgelöst. Man gibt dazu eine Lösung von 194,3 g (1,17 Mol) Methyl-4-hydroxyphenylacetat in 250 ml Methanol und anschliessend eine Lösung von 167,3 g (1,018 Mol) (S)-1,2-Epoxy-3-benzyloxypropan in 250 ml Methanol. Die Lösung wird 12 Stunden lang unter Rückfluss gerührt. Die abgekühlte Lösung wird mit 8,3 ml (0,1 Mol) konzentrierter Salzsäure behandelt; das Methanol wird im Vakuum entfernt. Das Rohprodukt wird in Benzol aufgelöst und mit zwei 200 ml Portionen kalter 1N Natriumhydroxidlösung und mit Wasser (1 x 300 ml) gewaschen. Die wässrigen Schichten werden mit Benzol (2 x 500 ml) zurückgewaschen. Die vereinigten organischen Schichten werden über Natriumsulfat getrocknet und im Vakuum eingedampft, wodurch man rohes (S)-Methyl-4-(3-benzyloxy-2-hydroxypropoxy)phenylacetat erhält.

Der rohe Ester wird anschliessend in einem Liter Essigsäure, enthaltend 20 ml konzentrierte Salzsäure, aufgelöst; die Mischung wird über 10 g 10% Palladium auf Kohle bei Normaldruck und Raumtemperatur hydriert. Nach etwa 100 Minuten tritt das Ende der Wasserstoffabsorption schlagartig ein (gesamte Aufnahme etwa 23,3 Liter). Der Katalysator wird mittels Filtration durch Celit entfernt; das Filtrat wird unter vermindertem Druck zur Trockene eingedampft. Da das Produkt während der Hydrierung einer teilweisen Acetylierung unterworfen worden ist, wird es in einem Liter 1N methanolischer Salzsäure aufgelöst und eine Stunde bei Raumtemperatur stehengelassen. Man dampft das Lösungsmittel ein und erhält (S)-Methyl-4-(2-dihydroxy-

propoxy)phenylacetat als weissen Feststoff.

Eine Kristallisation einer kleinen Probe aus Aether/Hexan liefert analysenreines Material, Schmelzpunkt 64-66°; $[\alpha]_D^{25}$ +6,55° (c = 1,0% in Aethanol).

## Beispiel 64

Eine gerührte Mischung aus 223,3 g (0,916 Mol) (S)-Methyl-4-(2-dihydroxypropoxy)phenylacetat, 3,0 g Benzoesäure (0,024 Mol) und 165 g (1,375 Mol) Trimethylorthoacetat wird in einem Oelbad auf 80° erwärmt. Das entstandene Methanol wird laufend aus der Reaktionsmischung destilliert. Nach 30 Minuten wird die Reaktionsmischung gekühlt und das Produkt zwischen einem Liter Methylenchlorid und 300 ml einer kalten gesättigten Natriumcarbonatlösung verteilt. Der organische Extrakt wird mit weiteren 200 ml Natriumbicarbonatlösung gewaschen; anschliessend werden die wässrigen Schichten mit Methylenchlorid (1 x 200 ml) zurückgewaschen. Die vereinigten Methylenchloridextrakte werden getrocknet und eingedampft, und man erhält das cyclische Orthoacetat.

Ohne weitere Reinigung wird dieses Material (0,895 Mol) in 750 ml trockenem Methylenchlorid aufgelöst, worauf 151 ml (1,16 Mol) Trimethylchlorsilan zugesetzt werden. Die erhaltene Lösung wird während etwa 45 Minuten unter Rückfluss erhitzt, worauf das Lösungsmittel und das überschüssige Reagens in Vakuum entfernt werden, und man (R)-Methyl-4-(2-acetoxy-3-chlorpropoxy)phenylacetat als ein Oel erhält.

Zur Analyse wird eine kleine Portion des Produktes (1 g) mittels Chromatographie an Silicagel gereinigt und man erhält 950 mg reines Material, $[\alpha]_D^{25}$ -23,86° (c = 1,0% in Methanol).

Beispiel 65

Eine kalte Lösung von 63 g (1,575 Mol) Natriumhydroxid in 200 ml Wasser wird unter Rühren während etwa 15 Minuten zu einer gekühlten Lösung ($0^{o}$) von 135 g (0,45 Mol) rohem (R)-Methyl-4-(2-acetoxy-3-chlorpropoxy)phenylacetat in 500 ml Methanol zugegeben. Die Reaktionstemperatur übersteigt während der Zugabe $10^{o}$ nicht; sie wird dann während 50 Minuten bei 0-$5^{o}$ und während weiteren 20 Minuten bei 10-$15^{o}$ gehalten. Die Mischung wird dann wieder abgekühlt und unter Verwendung einer Lösung von 32 ml konzentrierter Schwefelsäure (1,15 Aequivalente) in 200 ml Wasser sauer gestellt. Etwas Methanol (etwa 250 ml) wird durch Einengen der Reaktionsmischung im Vakuum (Badtemperatur etwa $25^{o}$) entfernt; dann wird die Lösung mit Benzol (4 x 1 Liter) extrahiert. Man wäscht die Benzolschichten ihrerseits mit gesättigter Kochsalzlösung (1 x 250 ml) und vereinigt sie, trocknet über Natriumsulfat und engt im Vakuum auf 500 ml ein. Die Benzollösung wird auf etwa $40^{o}$ erwärmt und mit warmem Hexan bis zur beginnenden Trübung verdünnt; dann wird sie mehrere Stunden bei $0^{o}$ stehengelassen. Das entstandene kristalline Material wird mittels Filtration gesammelt, und man erhält (R)-4-(2,3-Epoxypropoxy)phenylessigsäure, Schmelzpunkt 70-$72^{o}$; $[\alpha]_D^{25}$ +10,$6^{o}$ (c = 1,0% in Methanol). Eine Einengung der Mutterlaugen ergibt weiteres Produkt.

Beispiel 66

20,8 g (0,1 Mol) (R)-4-(2,3-Epoxypropoxy)phenylessigsäure werden in 30 ml Chloracetonitril suspendiert und in einem Eiswasserbad auf etwa $10^{o}$ abgekühlt. Triäthylamin (16 ml) wird rasch zur gerührten Mischung zugetropft. Das Kühlbad wird entfernt; nach 30 Minuten hat die Temperatur $30^{o}$ erreicht. Die Temperatur wird durch zeitweilige Verwendung des Kühlbades bei 25-$30^{o}$ gehalten. Nach 90 Minuten wird die Mischung zwischen Toluol und Wasser verteilt. Man wäscht die organische Phase mit gesättigter Natriumbicarbonatlösung und

mit Wasser und wäscht anschliessend die wässrigen Schichten mit Toluol zurück. Die Toluolextrakte werden vereinigt, über Magnesiumsulfat getrocknet und eingedampft,und man erhält (R)-4-(2,3-Epoxypropoxy)phenylessigsäure-cyanomethylester als ein Oel. Dieses Material wird ohne weitere Reinigung verwendet.

Beispiel 67

Eine Mischung von 10,7 g (52,1 mMol) 1-(2-Aminoäthyl)-4-phenylpiperazin und 13,5 g (54,6 mMol) (R)-4-(2,3-Epoxypropoxy)phenylessigsäure-cyanomethylester in 125 ml trockenem Tetrahydrofuran wird während 40 Stunden bei Raumtemperatur gerührt. Das Lösungsmittel wird im Vakuum entfernt, und der Rückstand mit Aether zerrieben, wodurch man das rohe Amid als einen weissen Feststoff erhält. Der Feststoff wird in 35 ml Methylenchlorid aufgelöst und auf eine Kolonne von 170 g basischem Aluminiumoxid (Woelm, Aktivität 3), präpariert in Methylenchlorid, gebracht. Man eluiert mit einem Liter Methylenchlorid und erhält im wesentlichen reines Material. Eine Kristallisation aus Aceton ergibt (R)-4-(2,3-Epoxypropoxy)phenyl-N-[2-(4-phenyl-1-piperazinyl)äthyl]acetamid, Schmelzpunkt 124-125°. Man erhält eine zweite Fraktion aus den Mutterlaugen, Schmelzpunkt 121-123°.

Durch Umkristallisation aus Methanol/Wasser erhält man aus dem obigen Material eine analysenreine Probe, Schmelzpunkt 123-124°;
$[\alpha]_D^{25}$ +5,64° (c = 1,0% in Methanol).

Beispiel 68

Eine Mischung aus 9,4 g (38 mMol) (R)-4-(2,3-Epoxypropoxy)phenylessigsäure-cyanomethylester und 9,4 g (36 mMol) 1-(6-Aminohexyl)-4-phenylpiperazin in 75 ml Tetrahydrofuran wird während 60 Stunden bei Raumtemperatur gerührt. Man entfernt das Lösungsmittel im Vakuum und zerreibt den Rückstand mit warmem Aether und erhält so das rohe Amid. 12,4 g des rohen Amides in 25 ml Methylenchlorid werden auf eine Kolonne von 125 g basischem Aluminiumoxid (Woelm, Aktivität 3) gebracht. Eluieren mit 750 ml Methylenchlorid liefert homogenes (DC) Material; Dieses Material wird aus Aceton kristallisiert, und man erhält reines (R)-4-(2,3-Epoxypropoxy)phenyl-N-[6-(4-phenyl-1-piperazinyl)-hexyl]acetamid, Schmelzpunkt 108-110°.

Eine analysenreine Probe, Schmelzpunkt 108-110° wird durch Kristallisation aus Methanol/Wasser wie im vorangegangenen Versuch hergestellt.

## Beispiel 69

Eine Lösung von 10,86 g (27,5 mMol) (R)-4-(2,3-Epoxypropoxy)phenyl-N-[2-(4-phenyl-1-piperazinyl)äthyl]acetamid in 200 ml Methanol, enthaltend 100 ml Isopropylamin, wird 90 Minuten lang unter Rückfluss erhitzt. Die Lösung wird zur Trockene eingedampft und mit heissem Aether zerrieben; man erhält (S)-4-(2-Hydroxy-3-isopropylaminopropoxy)phenyl-N-[2-(4-phenyl-1-piperazinyl)äthyl]acetamid, Schmelzpunkt 121-123°.

Eine Kristallisation einer kleinen Probe aus Aethanol/Aethylacetat liefert analysenreines Material, Schmelzpunkt 123-124°; $[\alpha]_D^{25}$ -0,8° (c = 1,0% in Methanol).

Eine Portion (S)-4-(2-Hydroxy-3-isopropylaminopropoxy)phenyl-N-[2-(4-phenyl-1-piperazinyl)äthyl]acetamid (8 g ) wird in 50 ml Aethanol aufgelöst und mit 35 ml 1,73N methanolischer Salzsäure (60 mMol) behandelt und zur Trockene eingedampft. Der Rückstand wird zur Entfernung des Salzsäureüberschusses zweimal mit Aethanol behandelt. Man löst anschliessend den Rückstand wieder in 50 ml Aethanol auf und gibt die restliche Portion (S)-4-(2-Hydroxy-3-isopropylaminopropoxy)phenyl-N-[2-(4-phenyl-1-piperazinyl)äthyl]acetamid (4 g) zu. Bei

der Zugabe von 50 ml Aethylacetat beginnen sich Kristalle zu bilden; nach Abkühlen erhält man reines (S)-4-(2-Hydroxy-3-isopropylaminopropoxy)phenyl-N-[2-(4-phenyl-1-piperazinyl)äthyl]acetamid-dihydrochlorid, Schmelzpunkt 160-162°; $[\alpha]_D^{25}$ -12,75°. Eine zweite Fraktion, Schmelzpunkt 159-161°, wird aus den Mutterlaugen erhalten.

Beispiel 70

Eine Lösung von 7,8 g (R)-4-(2,3-Epoxypropoxy)-phenyl-N-[6-(4-phenyl-1-piperazinyl)hexyl]acetamid in 175 ml Methanol, enthaltend 75 ml Isopropylamin, wird 75 Minuten lang unter Rückfluss erhitzt. Die Lösung wird zur Trockene eingedampft und der Rückstand mit Aether zerrieben; man erhält (S)-4-(2-Hydroxy-3-isopropylaminopropoxy)phenyl-N-[6-(4-phenyl-1-piperazinyl)hexyl]acetamid. Eine Kristallisation des Produktes aus Methanol/Aethylacetat liefert reines Material, Schmelzpunkt 103-104°.

Eine analysenreine Probe, Schmelzpunkt 103-104°; $[\alpha]_D^{25}$ -0,68° (c = 1,0% in Methanol) wird aus der gleichen Lösungsmittelmischung erhalten.

4,4 g (8,615 mMol) (S)-4-(2-Hydroxy-3-isopropylamino-propoxy)phenyl-N-[6-(4-phenyl-1-piperazinyl)hexyl]acetamid werden in Aceton (100 ml), enthaltend 1,0 g (8,615 mMol) Maleinsäure, aufgelöst. Man engt die Lösung auf etwa 75 ml ein und kühlt anschliessend auf 0°ab. Das entstandene kristalline Salz wird mittels Filtration gewonnen, und man erhält (S)-4-(2-Hydroxy-3-isopropylaminopropoxy)phenyl-N-[6-(4-phenyl-1-piperazinyl)hexyl]acetamid-maleat, Schmelzpunkt 107-108°: Eine Umkristallisation aus Aceton ergibt das reine Salz, Schmelzpunkt 107-108°; $[\alpha]_D^{25}$ -10,19° (c = 1% in Methanol).

Beispiel 71

Eine Lösung von 42,0 g (0,259 Mol) 1-Phenylpiperazin und 40,0 g (0,245 Mol) N-(4-Hydroxyphenyl)-2-propenamid in 600 ml Aethanol wird über Nacht unter Rückfluss erhitzt und anschliessend abgekühlt. Der entstandene Niederschlag wird abfiltriert und aus Methanol umkristallisiert; man erhält N-(4-Hydroxyphenyl)-4-phenyl-1-piperazinpropanamid als farblose Kristalle, Schmelzpunkt 172-174°. Durch Umkristallisation aus Aethanol erhält man eine analysenreine Probe, Schmelzpunkt 174-176°.

Beispiel 72

Zu einer Lösung von 33,0 g (0,102 Mol) N-(4-Hydroxyphenyl)-4-phenyl-1-piperazinpropanamid in 120 ml 1N Natriumhydroxidlösung werden 50 ml Epichlorhydrin zugesetzt. Ueber Nacht rührt man die Mischung bei Raumtemperatur und extrahiert anschliessend 3 x mit Chloroform. Das Lösungsmittel wird unter Vakuum entfernt und der kristalline Rückstand in 100 ml konzentrierter Salzsäure aufgelöst. Nach einem Stehenlassen bei Raumtemperatur während 15 Minuten wird die Lösung unter Vakuum zur Trockene eingeengt. Der Rückstand wird aus Methanol unter Verwendung einer Soxhlet-Apparatur umkristallisiert, und man erhält (R,S)-N-[4-(3-Chlor-2-hydroxypropoxy)phenyl]-4-phenyl-1-piperazinpropanamid-hydrochlorid in Form von farblosen Kristallen, Schmelzpunkt 242-244°. Eine weitere Umkristallisation ergibt eine analysenreine Probe, Schmelzpunkt 243-245°.

Beispiel 73

Eine Lösung von 2,00 g (0,0044 Mol) (R,S)-N-[4-(3-Chlor-2-hydroxypropoxy)phenyl]-4-phenyl-1-piperazinpropanamid-hydrochlorid und 20 ml Isopropylamin in 20 ml Methanol wird drei Tage unter Rückfluss erhitzt und anschliessend unter Vakuum eingeengt. Man mischt den Rückstand mit verdünnter Salzsäure und wäscht die entstandene Lösung mehrere

Male mit Chloroform; anschliessend wird mit Natriumbicarbonat basisch gestellt. Die Mischung wird mehrere Male mit Chloroform extrahiert; die Extrakte werden getrocknet und eingeengt, und man erhält (R,S)-N-[4-(2-Hydroxy-3-isopropylaminopropoxy)phenyl]-4-phenyl-1-piperazinpropanamid als einen amorphen Feststoff. Zu einer methanolischen Lösung von 0,80 g (0,0018 Mol) dieses Feststoffes werden 0,42 g (0,0036 Mol) Maleinsäure zugesetzt. Das Methanol wird unter Vakuum entfernt, und der Rückstand mehrere Male aus Acetonitril umkristallisiert; man erhält (R,S)-N-[4-(2-Hydroxy-3-isopropylaminopropoxy)phenyl]-4-phenyl-1-piperazinpropanamid- bis-maleat in Form von farblosen Kristallen, Schmelzpunkt 159,5-161,5$^{o}$.

## Beispiel 74

Eine Mischung von 39,0 g (0,20 Mol) 6-Bromhexansäure und 39,0 ml Thionylchlorid wird mittels eines Oelbades von 90$^{o}$ erwärmt, bis die Temperatur der Mischung 80$^{o}$ erreicht hat und die anfängliche Gasentwicklung im wesentlichen beendet ist. Der Ueberschuss an Thionylchlorid wird unter Vakuum von der Reaktionsmischung abdestilliert. Man gibt Toluol (25 ml) zu und destilliert dieses ebenfalls unter Vakuum ab. Die gekühlte Reaktionsmischung wird dann zu einem Brei von 43,6 g (0,40 Mol) 4-Aminophenol in 500 ml Dioxan zugegeben. Nach einem 30-minütigem Rühren wird die Mischung filtriert; das Filtrat wird unter Vakuum eingeengt. Der Rückstand wird aus Chloroform unter Verwendung einer Soxhlet-Vorrichtung umkristallisiert, und man erhält 6-Brom-N-(4-hydroxyphenyl)hexanamid als braungelbe Kristalle, Schmelzpunkt 127-129$^{o}$. Eine analysenreine Probe wird aus Methylenchlorid mit Aktivkohle in Form von farblosen Kristallen, Schmelzpunkt 126-128$^{o}$, erhalten.

## Beispiel 75

Eine Lösung von 45,7 g (0,16 Mol) 6-Brom-N-(4-hydroxyphenyl)hexanamid, 26,0 g (0,16 Mol) 1-Phenylpiperazin und

22,4 ml (16,3 g, 0,16 Mol) Triäthylamin in 400 ml Aethanol wird über Nacht unter Rückfluss erhitzt und dann unter Vakuum eingeengt. Der Rückstand wird mit Natriumbicarbonatlösung und Chloroform vermischt. Die entstandene Suspension wird filtriert. Der Feststoff wird aus Aethanol/Wasser und anschliessend aus Acetonitril umkristallisiert, und man erhält N-(4-Hydroxyphenyl)-4-phenyl-1-piperazinhexanamid als braungelbe Kristalle, Schmelzpunkt 157-159°.

Beispiel 76

Eine Mischung von 38,1 g (0,104 Mol) N-(4-Hydroxyphenyl)-4-phenyl-1-piperazinhexanamid und 500 ml 3N Natriumhydroxidlösung wird während 45 Minuten bei Raumtemperatur gerührt. Zur erhaltenen Lösung gibt man 76 ml Epichlorhydrin und setzt das Rühren über Nacht fort. Die heterogene Reaktionsmischung wird mit Methylenchlorid extrahiert; die Extrakte werden getrocknet und eingeengt, wodurch man 45,0 g eines Oeles erhält. Dieses wird auf eine Kolonne mit 500 g Silicagel gebracht. Eluieren mit 7% und 10% Aethylacetat in Methylenchlorid ergibt einen farblosen Feststoff. Eine Lösung von 8,8 g dieses farblosen Feststoffes und 50 ml Isopropylamin in 50 ml Methanol wird während 4 Stunden unter Rückfluss erhitzt und unter Vakuum eingeengt. Der Rückstand wird mit Methylenchlorid und Natriumbicarbonatlösung vermischt; die organische Schicht wird getrocknet und eingeengt. Der Rückstand wird mehrere Male aus Methylenchlorid/Aether ausgefällt, und man erhält (R,S)-N-[4-(2-Hydroxy-3-isopropylaminopropoxy)phenyl]-4-phenyl-1-piperazinhexanamid als farblosen, amorphen Feststoff, Schmelzpunkt 125-130°.

Beispiel 77

Eine methanolische Lösung von 4,10 g (0,0085 Mol) (R,S)-N-[4-(2-Hydroxy-3-isopropylaminopropoxy)phenyl]-4-phenyl -piperazinhexanamid und 0,986 g (0,0085 Mol) Maleinsäure wird eingeengt und der Rückstand aus Acetonitril/

Aethylacetat umkristallisiert; man erhält (R,S)-N-[4-(2-Hydroxy-3-isopropylaminopropoxy)phenyl]-4-phenyl-1-piperazinhexanamid-maleat in Form von farblosen Kristallen, Schmelzpunkt 144,5-146,5°.

Beispiel 78

Eine Mischung aus 94,7 g (0,357 Mol) 11-Bromundecansäure und 70 ml Thionylchlorid wird mit einem Oelbad von 90° erwärmt, bis die Gasentwicklung im wesentlichen aufgehört hat. Ueberschüssiges Thionylchlorid wird unter Vakuum aus der Reaktionsmischung abdestilliert. Man gibt Toluol (50 ml) zu und destilliert dieses ebenfalls unter Vakuum ab. Die gekühlte Reaktionsmischung wird dann zu einem Brei von 78,0 g (0,715 Mol) 4-Aminophenol in 500 ml Dioxan zugegeben. Nach einem 30-minütigem Rühren wird die Mischung filtriert,und der Feststoff mit heissem Dioxan gewaschen. Die vereinigten Filtrate werden eingeengt,und der Rückstand aus Chloroform umkristallisiert; man erhält 11-Brom-N-(4-hydroxyphenyl)-undecanamid in Form von farblosen Kristallen, Schmelzpunkt 116-117°.

Beispiel 79

Eine Lösung von 66,8 g (0,187 Mol) 11-Brom-N-(4-hydroxyphenyl)undecanamid, 30,3 g (0,187 Mol) 1-Phenylpiperazin und 27,0 ml (19,6 g, 0,194 Mol) Triäthylamin in 600 ml Aethanol wird über Nacht unter Rückfluss erhitzt und dann abgekühlt. Man schüttelt den entstandenen Feststoff mit Natriumbicarbonatlösung und Methylenchlorid und filtriert die Suspension. Der Feststoff wird aus 95%igem Aethanol umkristallisiert,und man erhält N-(4-Hydroxyphenyl)-4-phenyl-1-piperazinundecanamid in Form von farblosen Kristallen, Schmelzpunkt 160,5-162,5°.

## Beispiel 80

Zu einer gerührten Lösung von 14,0 g (0,20 Mol) Kaliummethoxid in 400 ml Methanol werden 44,8 g (0,10 Mol) N-(4-Hydroxyphenyl)-4-phenyl-1-piperazinundecanamid zugegeben. 15 Minuten später werden 90 ml Epichlorhydrin zugegeben; das Rühren wird über Nacht fortgesetzt. Man engt die Mischung unter Vakuum ein, mischt den Rückstand mit Natriumbicarbonatlösung und extrahiert mit Chloroform. Die Extrakte werden getrocknet und eingeengt; der feste Rückstand wird aus Chloroform und aus Aethylacetat umkristallisiert, und man erhält 34 g eines farblosen Feststoffes. Man löst diesen in 200 ml Methanol auf, gibt 200 ml Isopropylamin zu, erhitzt die Lösung über Nacht unter Rückfluss und engt anschliessend unter Vakuum ein. Der Rückstand wird mit Natriumbicarbonatlösung vermischt und mit Chloroform extrahiert. Die Extrakte werden getrocknet und eingeengt, und man erhält einen farblosen Feststoff. Dieser wird in Chloroform aufgelöst und an 500 g Silicagel chromatographiert. Eluieren mit Methanol/Chloroform (1:1) ergibt ein Material, welches beim Umkristallisieren aus Methanol (R,S)-N-[4-(2-Hydroxy-3-isopropylaminopropoxy)phenyl]-4-phenyl-1-piperazinundecanamid in Form von farblosen Kristallen ergibt, Schmelzpunkt 141,5-142,5°.

## Beispiel 81

Eine Mischung von 0,200 g (0,36 mMol) (R,S)-N-[4-(2-Hydroxy-3-isopropylaminopropoxy)phenyl]-4-phenyl-1-piperazinundecanamid und 42 mg (0,36 mMol) Maleinsäure wird aus Methanol/Aethylacetat umkristallisiert, und man erhält (R,S)-N-[4-(2-Hydroxy-3-isopropylaminopropoxy)phenyl]-4-phenyl-1-piperazinundecanamid-maleat in Form von farblosen Kristallen, Schmelzpunkt 141-142°.

## Beispiel 82

Chlorwasserstoff wird in eine Lösung von 5,53 g (0,010 Mol) (R,S)-N-[4-(2-Hydroxy-3-isopropylaminopropoxy)-phenyl]-4-phenyl-1-piperazinundecanamid in einer Mischung von Methylenchlorid und Chloroform eingeleitet. Die Mischung wird unter Vakuum eingeengt, und der Rückstand aus Methanol/Aethylacetat umkristallisiert. Man erhält (R,S)-N-[4-(2-Hydroxy-3-isopropylaminopropoxy)phenyl]-4-phenyl-1-piperazinundecanamid-hydrochlorid, Schmelzpunkt 210-212°.

## Beispiel 83

Zu einer rasch gerührten Lösung von 2,243 g (0,01 Mol) 4-(2-Hydroxy-3-isopropylaminopropoxy)anilin in 100 ml Methylenchlorid wird tropfenweise eine Lösung von 0,80 ml (0,91 g, 0,01 Mol) 2-Propenoylchlorid zugegeben. Die Mischung wird gerührt, bis sich das Zwischenprodukt abgesetzt hat, wobei eine klare überstehende Flüssigkeit zurückbleibt, welche dekantiert wird. Der Rückstand wird in 25 ml Aethanol aufgelöst. Man gibt 5,0 ml (5,3 g, 0,033 Mol) 1-Phenylpiperazin zu und erhitzt die Lösung über Nacht unter Rückfluss. Die Reaktionsmischung wird abgekühlt und filtriert. Man löst den Feststoff in Wasser auf, stellt die Lösung mit Natriumbicarbonat basisch und extrahiert mit Methylenchlorid. Die Extrakte werden getrocknet und eingeengt. Der amorphe Niederschlag wird mehrere Male aus Aether wieder ausgefällt, und man erhält (R,S)-N-[4-(2-Hydroxy-3-isopropylaminopropoxy)phenyl]-4-phenyl-1-piperazinpropanamid in Form eines farblosen, amorphen Feststoffes, Schmelzpunkt 151-157°. Die Analysen werden mit diesem amorphen Feststoff ausgeführt. Anschliessend wird das Material aus Aether kristallin erhalten und umkristallisiert, und man erhält (R,S)-N-[4-(2-Hydroxy-3-isopropylaminopropoxy)-phenyl]-1-piperazinpropanamid in Form von farblosen Kristallen, Schmelzpunkt 250-252°.

## Beispiel A

Tabletten der nachfolgenden Zusammensetzung werden hergestellt:

| | mg/Tablette | mg/Tablette |
|---|---|---|
| (S)-1-[2-(4-(2-Hydroxy-3-isopropylaminopropoxy)-phenoxy)äthyl]-4-phenyl-piperazin-bis-maleat | 25 | 50 |
| Vorgelatinierte Stärke | 12,5 | 15 |
| Lactose | 155 | 162 |
| Mikrokristalline Cellulose | 30 | 40 |
| Modifizierte Stärke | 25 | 30 |
| Magnesiumstearat | 2,5 | 3 |
| | 250 mg | 300 mg |

Verfahren:

Die ersten fünf Bestandteile werden in einem geeigneten Mischer vermischt. Es wird mit Wasser granuliert, in einem Ofen getrocknet und gemahlen. Man gibt das Magnesiumstearat zu, mischt während 5 Minuten und verpresst auf einer geeigneten Tablettenpresse.

## Beispiel B

Tabletten der nachfolgenden Zusammensetzung werden hergestellt:

| | mg/Tablette | mg/Tablette |
|---|---|---|
| (S)-1-[2-(4-(2-Hydroxy-3-isopropylaminopropoxy)-phenoxy)äthyl]-4-phenyl-piperazin-bis-maleat | 25 | 50 |
| Lactose | 147,5 | 157 |
| Modifizierte Stärke | 25 | 30 |
| Mikrokristalline Cellulose | 50 | 60 |
| Magnesiumstearat | 2,5 | 3 |
| | 250 mg | 300 mg |

Verfahren:

Die ersten vier Bestandteile werden in einem geeigneten Mischer vermischt. Man gibt das Magnesiumstearat anschliessend zu und mischt während 5 Minuten. Die Mischung wird auf einer geeigneten Tablettenpresse verpresst.

## Beispiel C

Kapseln der folgenden Zusammensetzung werden hergestellt:

| | mg/Kapsel | mg/Kapsel |
|---|---|---|
| (S)-1-[2-(4-(2-Hydroxy-3-isopropylaminopropoxy)-phenoxy)äthyl]-4-phenyl-piperazin-bis-maleat | 25 | 50 |
| Lactose | 125 | 170 |
| Maisstärke | 40 | 60 |
| Talk | 15 | 20 |
| | 250 mg | 300 mg |

Verfahren:

Die ersten drei Bestandteile werden in einem geeigneten Mischer vermischt, worauf der Talk zugesetzt wird. Die Mischung wird während 5 Minuten vermischt und auf einer geeigneten Kapselvorrichtung abgefüllt.

## Beispiel D

Wenn man nach den in den Beispielen A bis C beschriebenen Verfahren arbeitet, können aus den folgenden bevorzugten Verbindungen und ihren pharmazeutisch annehmbaren Salzen Tabletten oder Kapseln hergestellt werden:

(S)-4-(2-Hydroxy-3-isopropylaminopropoxyphenyl)-N-[2-(4-phenyl-1-piperazinyl)äthyl]acetamid.

(S)-4-(2-Hydroxy-3-isopropylaminopropoxyphenyl)-N-[6-(4-phenyl-1-piperazinyl)hexyl]acetamid.

(S)-1-[6-(4-(2-Hydroxy-3-isopropylaminopropoxy)-phenoxy)hexyl]-4-phenylpiperazin.

## Patentansprüche

1. Phenylpiperazin-Derivate der allgemeinen Formel

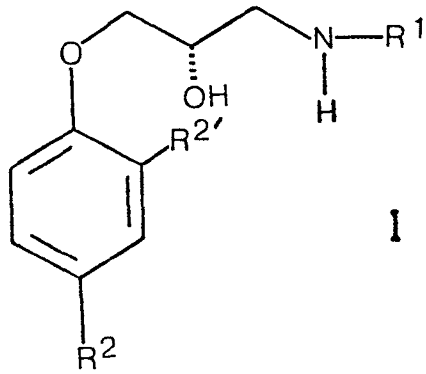

I

worin $R^1$ Niederalkyl, einer der Reste $R^2$ und $R^{2'}$ Wasserstoff und der andere die Gruppe

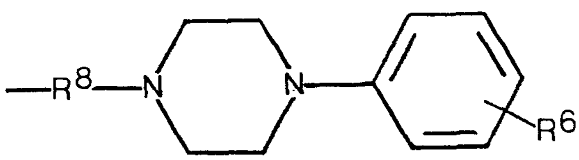

,

$R^8$ eine der Gruppen $-O-(CH_2)_n-$, $-NH\underset{\overset{\|}{O}}{C}-(CH_2)_n-$ und $-CH_2\underset{\overset{\|}{O}}{C}NH(CH_2)_n-$, $R^6$ Wasserstoff oder Niederalkoxy und n eine ganze Zahl von 2-20 bedeuten, deren Racemate sowie die pharmazeutisch annehmbaren Säureadditionssalze dieser Verbindungen.

2. Verbindungen gemäss Anspruch 1, worin $R^{2'}$ Wasserstoff bedeutet.

3. Verbindungen gemäss Anspruch 2, worin $R^1$ eine verzweigtkettige Alkylgruppe mit 3 oder 4 Kohlenstoffatomen, $R^8$ die Gruppe $-O-(CH_2)_n-$ oder $-CH_2CONH(CH_2)_n-$, n eine ganze Zahl von 2-10 und $R^6$ Wasserstoff bedeuten.

4. Verbindungen gemäss Anspruch 3, worin $R^1$ Isopropyl

oder tert.Butyl und n die Zahl 2 bedeuten.

5. Verbindungen gemäss einem der Ansprüche 2 bis 4, welche die absolute S-Konfiguration haben.

6. (S)-1-[2-(4-Hydroxy-3-isopropylaminopropoxy)-phenoxy)äthyl]-4-phenylpiperazin.

7. (R,S)-1-[2-(4-(2-Hydroxy-3-isopropylaminopropoxy)-phenoxy)äthyl]-4-phenylpiperazin.

8. (S)-1-[6-(4-(2-Hydroxy-3-isopropylaminopropoxy)-phenoxy)hexyl]-4-phenylpiperazin.

9. 4-[(2-Hydroxy-3-isopropylaminopropoxy)phenyl]-N-[2-(4-phenyl-1-piperazinyl)äthyl]acetamid.

10. 4-[(2-Hydroxy-3-isopropylaminopropoxy)phenyl]-N-[6-(4-phenyl-1-piperazinyl)hexyl]acetamid.

11. (S)-1-[2-(4-(2-Hydroxy-3-tert-butylaminopropoxy)-phenoxy)äthyl]-4-phenylpiperazin, (S)-1-[2-(4-(2-Hydroxy-3-isopropylaminopropoxy)phenoxy)äthyl]-4-(2-methoxyphenyl)piperazin, (S)-1-[11-(4-(2-Hydroxy-3-isopropyl-aminopropoxy)phenoxy)undecanyl]-4-phenylpiperazin, (S)-1-[3-(2-(2-Hydroxy-3-isopropylaminopropoxy)phenoxy)propyl]-4-phenylpiperazin, (S)-1-[6-(2-(2-Hydroxy-3-isopropylamino-propoxy)phenoxy)hexyl]-4-phenylpiperazin, 4-[(2-Hydroxy-3-isopropylaminopropoxy)phenyl]-N-[11-(4-phenyl-1-piperazinyl)undecyl]acetamid, (S)-4-(2-Hydroxy-3-isopropyl-aminopropoxy)phenyl-N-[2-(4-phenyl-1-piperazinyl)äthyl]-acetamid, (S)-4-(2-Hydroxy-3-isopropylaminopropoxy)-phenyl-N-[6-(4-phenyl-1-piperazinyl)hexyl]acetamid, (R,S)-N-[4-(2-Hydroxy-3-isopropylaminopropoxy)phenyl]-4-phenyl-1-piperazinpropanamid, (R,S)-N-[4-(2-Hydroxy-3-isopropylaminopropoxy)phenyl]-4-phenyl-1-piperazinhexanamid oder (R,S)-N-[4-(2-Hydroxy-3-isopropylaminopropoxy)-phenyl]-4-phenyl-1-piperazinundecanamid.

12. Verbindungen der allgemeinen Formel

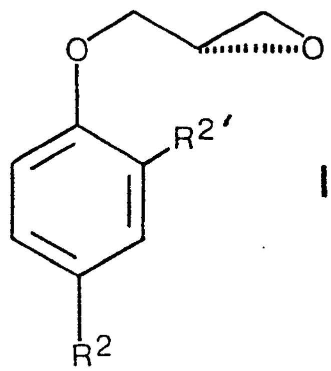

II

worin einer der Reste $R^2$ und $R^{2'}$ Wasserstoff und der andere die Gruppe

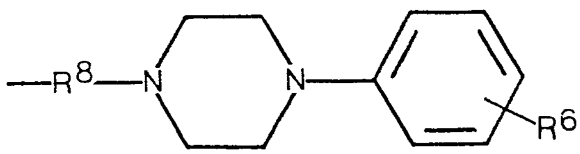

,

$R^8$ eine der Gruppen $-O-(CH_2)_n-$, $-NHCO(CH_2)_n-$ und $-CH_2CONH(CH_2)_n-$, $R^6$ Wasserstoff oder Niederalkoxy und n eine ganze Zahl von 2-20 bedeuten,
und deren Racemate.

13. Verbindungen der allgemeinen Formel

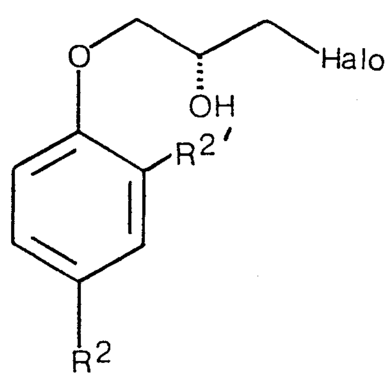

II'

worin einer der Reste $R^2$ und $R^{2'}$ Wasserstoff und der andere die Gruppe

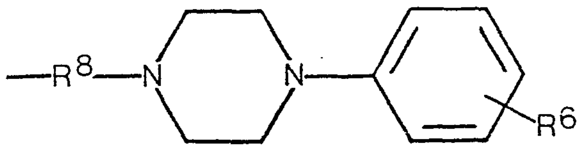

$R^8$ eine der Gruppen $-O-(CH_2)_n-$, $-NHCO(CH_2)_n-$ und $-CH_2CONH(CH_2)_n-$, $R^6$ Wasserstoff oder Niederalkoxy und n eine ganze Zahl von 2-20 bedeuten,
und deren Racemate.

14. Verbindungen der allgemeinen Formel

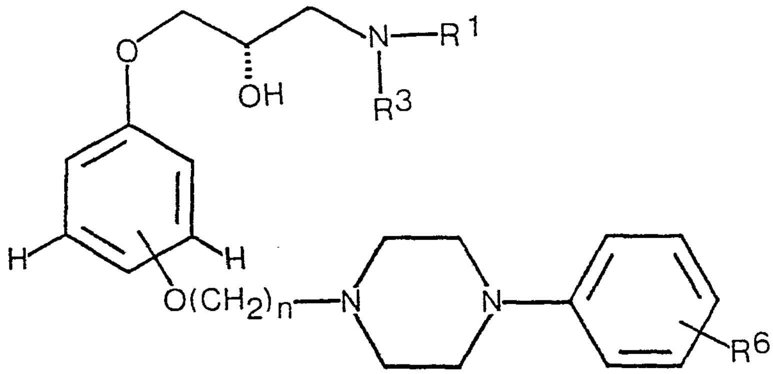

IV

worin $R^1$ Niederalkyl, $R^3$ eine Schutzgruppe, $R^6$ Wasserstoff oder Niederalkoxy und n eine ganze Zahl von 2-20 bedeuten,
und deren Racemate.

15. Verbindungen der allgemeinen Formel

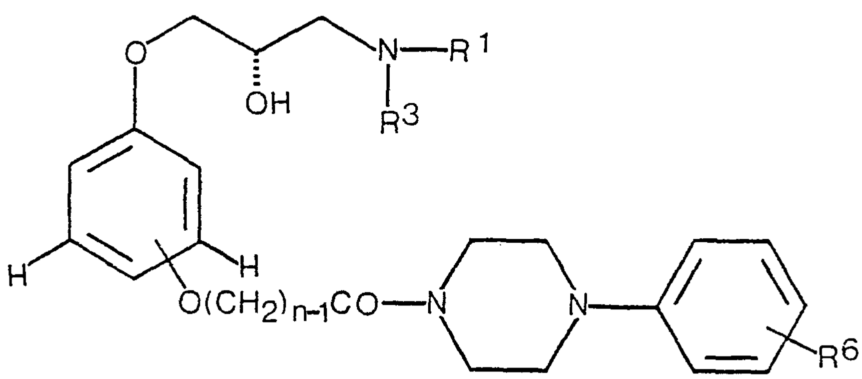

V

worin $R^1$ Niederalkyl, $R^3$ eine Schutzgruppe, $R^6$ Wasserstoff oder Niederalkoxy und n eine ganze Zahl von 2-20 bedeuten,

und deren Racemate.

16. Verbindungen der allgemeinen Formel

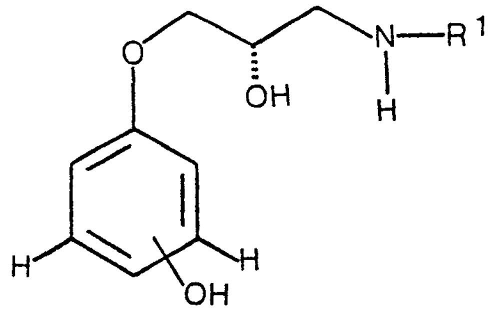

VI

worin $R^1$ Niederalkyl bedeutet, und deren Racemate.

17. Verbindungen der allgemeinen Formel

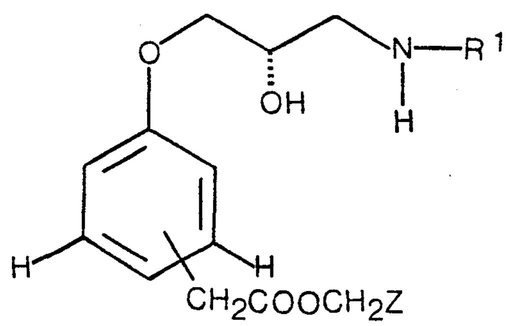

VIII

worin $R^1$ Niederalkyl und Z Wasserstoff oder Cyan bedeuten, und deren Racemate.

18. Verbindungen der allgemeinen Formel

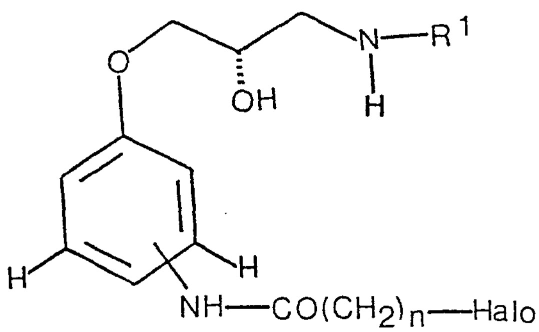

X

worin $R^1$ Niederalkyl bedeutet, und deren Racemate.

19. Verbindungen der allgemeinen Formel

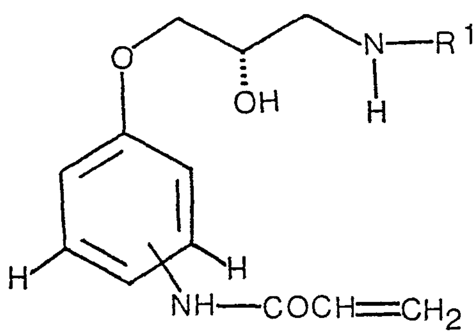

XII

worin $R^1$ Niederalkyl bedeutet, und deren Racemate.

20. Verbindungen der allgemeinen Formel

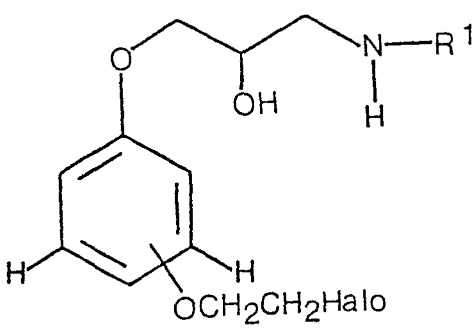

XIII

worin $R^1$ Niederalkyl bedeutet.

21. Phenylpiperazin-Derivate gemäss einem der Ansprüche 1-11 als pharmazeutische Wirkstoffe.

22. Phenylpiperazin-Derivate gemäss einem der Ansprüche 1-11 als α- und β-adrenergische Blocker und Sekretionshemmer.

23. (S)-1-[2-(4-(2-Hydroxy-3-isopropylaminopropoxy)-phenoxy)äthyl]-4-phenylpiperazin als pharmazeutischer Wirkstoff.

24. (R,S)-1-[2-(4-(2-Hydroxy-3-isopropylaminopropoxy)-phenoxy)äthyl]-4-phenylpiperazin als pharmazeutischer Wirkstoff.

25. (S)-1-[6-(4-(2-Hydroxy-3-isopropylaminopropoxy)-phenoxy)hexyl]-4-phenylpiperazin als pharmazeutischer Wirkstoff.

26. 4-[(2-Hydroxy-3-isopropylaminopropoxy)phenyl]-N-[2-(4-phenyl-1-piperazinyl)äthyl]acetamid als pharmazeutischer Wirkstoff.

27. 4-[(2-Hydroxy-3-isopropylaminopropoxy)phenyl]-N-[6-(4-phenyl-1-piperazinyl)hexyl]acetamid als pharmazeutischer Wirkstoff.

28. (S)-1-[2-(4-(2-Hydroxy-3-isopropylaminopropoxy)-phenoxy)äthyl]-4-phenylpiperazin als α- und β-adrenergischer Blocker und Sekretionshemmer.

29. (R,S)-1-[2-(4-(2-Hydroxy-3-isopropylaminopropoxy)-phenoxy)äthyl]-4-phenylpiperazin als α- und β-adrenergischer Blocker und Sekretionshemmer.

30. (S)-1-[6-(4-(2-Hydroxy-3-isopropylaminopropoxy)-phenoxy)hexyl]-4-phenylpiperazin als α- und β-adrenergischer Blocker und Sekretionshemmer.

31. 4-[(2-Hydroxy-3-isopropylaminopropoxy)phenyl]-N-[2-(4-phenyl-1-piperazinyl)äthyl]acetamid als α- und β-adrenergischer Blocker und Sekretionshemmer.

32. 4-[(2-Hydroxy-3-isopropylaminopropoxy)phenyl]-N-[6-(4-phenyl-1-piperazinyl)hexyl]acetamid als α- und β-adrenergischer Blocker und Sekretionshemmer.

33. Verfahren zur Herstellung von Verbindungen der in Anspruch 1 wiedergegebenen Formel I, deren Racematen und der pharmazeutisch annehmbaren Säureadditionssalze dieser Verbindungen, dadurch gekennzeichnet, dass man

a) eine Verbindung der allgemeinen Formel

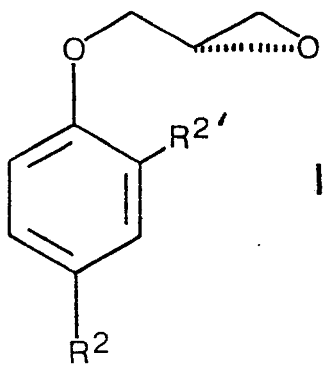

II

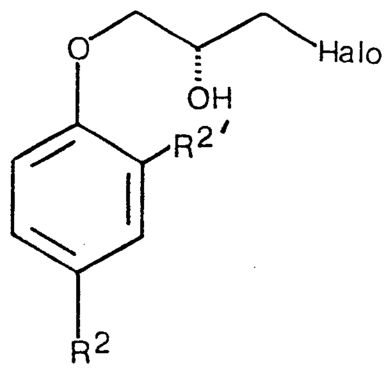

II'

worin $R^2$ und $R^{2'}$ die oben angegebene Bedeutung haben,

oder ein entsprechendes Racemat mit einem Amin der allgemeinen Formel

$$R^1NH_2 \qquad III$$

worin $R^1$ die oben angegebene Bedeutung hat,

umsetzt, oder

b) zur Herstellung einer Verbindung der obigen Formel I oder eines entsprechenden Racemates, worin $R^8$ die Gruppe $-O-(CH_2)_n-$ bedeutet und die übrigen Symbole die oben angegebene Bedeutung haben, in einer Verbindung der allgemeinen Formel

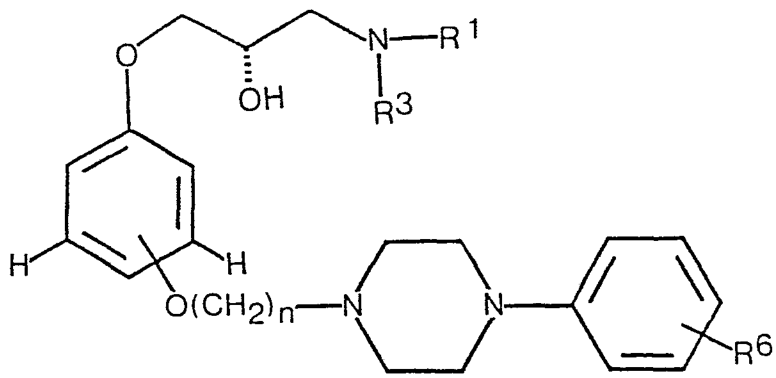

IV

worin $R^3$ eine Schutzgruppe bedeutet und $R^1$, $R^6$ und n die oben angegebene Bedeutung haben,

oder in einem entsprechenden Racemat die Schutzgruppe reduktiv abspaltet,

oder

c) zur Herstellung einer Verbindung der obigen Formel I oder eines entsprechenden Racemates, worin $R^8$ die Gruppe $-O-(CH_2)_n-$ bedeutet und die übrigen Symbole die oben angegebene Bedeutung haben, eine Verbindung der allgemeinen Formel

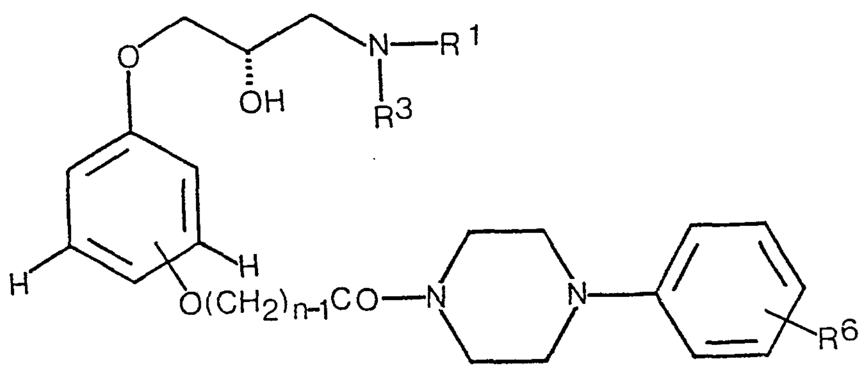

V

worin $R^1$, $R^3$, $R^6$ und n die oben angegebene Bedeutung haben,

oder ein entsprechendes Racemat mit einem geeigneten Reduktionsmittel behandelt,

oder

d) zur Herstellung einer Verbindung der obigen Formel I oder eines entsprechenden Racemates, worin $R^8$ die Gruppe $-O(CH_2)_n-$ bedeutet und die übrigen Symbole die oben angegebene Bedeutung haben, eine Verbindung der allgemeinen Formel

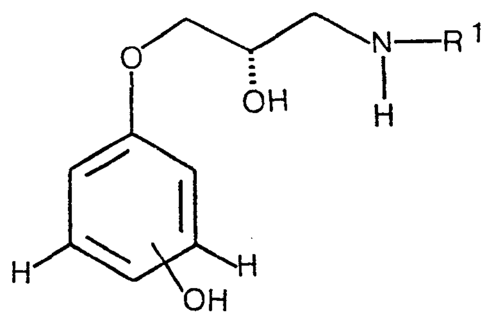

VI

worin $R^1$ die oben angegebene Bedeutung hat,

oder ein entsprechendes Racemat mit einer Verbindung der allgemeinen Formel

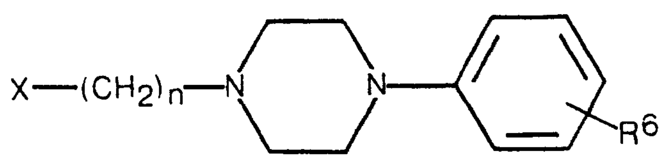

VII

worin X eine Abgangsgruppe bedeutet und $R^6$ und n die oben angegebene Bedeutung haben,

behandelt,

oder

e) zur Herstellung einer Verbindung der obigen Formel I oder eines entsprechenden Racemates, worin $R^8$ die Gruppe $-CH_2CONH(CH_2)_n-$ bedeutet und die übrigen Symbole die oben angegebene Bedeutung haben, eine Verbindung der allgemeinen Formel

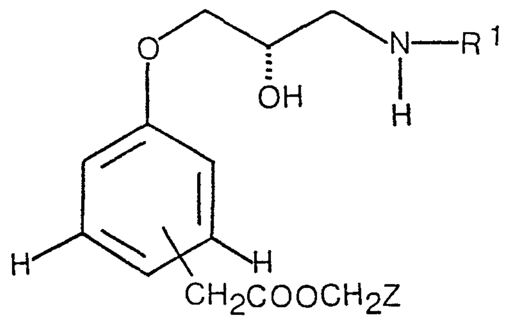

VIII

worin Z Wasserstoff oder Cyan bedeutet und $R^1$ die oben angegebene Bedeutung hat, oder ein entsprechendes Racemat mit einer Verbindung der allgemeinen Formel

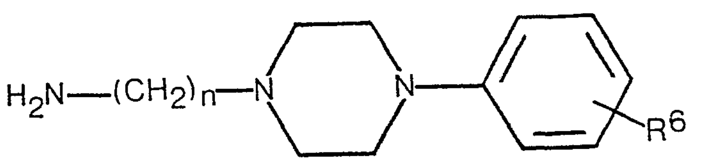

IX

worin $R^6$ und n die oben angegebene Bedeutung haben, behandelt, oder

f) zur Herstellung einer Verbindung der obigen Formel I oder eines entsprechenden Racemates, worin $R^8$ die Gruppe $-NHCO(CH_2)_n-$ bedeutet und die übrigen Symbole die oben angegebene Bedeutung haben, eine Verbindung der allgemeinen Formel

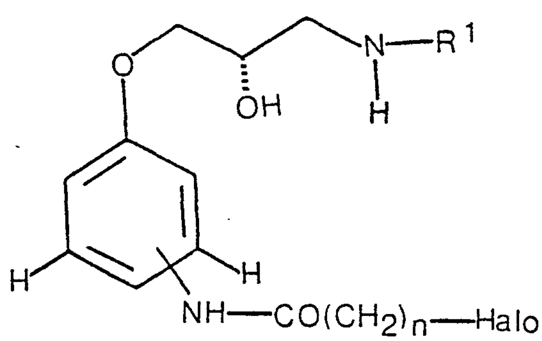

X

worin $R^1$ und n die oben angegebene Bedeutung haben, oder ein entsprechendes Racemat mit einer Verbindung der allgemeinen Formel

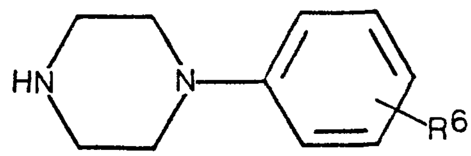

XI

worin $R^6$ die oben angegebene Bedeutung hat, behandelt,
oder

g) zur Herstellung einer Verbindung der obigen Formel I oder eines entsprechenden Racemates, worin $R^8$ die Gruppe $-NHCO(CH_2)_2-$ bedeutet und die übrigen Symbole die oben angegebene Bedeutung haben, eine Verbindung der allgemeinen Formel

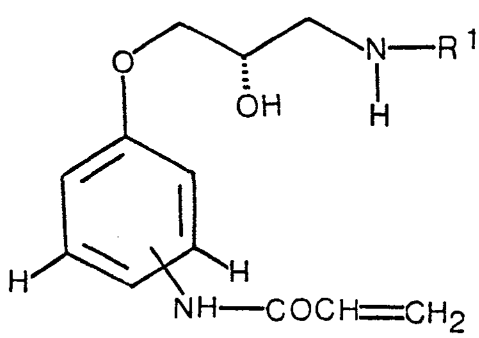

XII

worin $R^1$ die oben angegebene Bedeutung hat, oder ein entsprechendes Racemat mit einer Verbindung der obigen Formel XI behandelt,
oder

h) zur Herstellung des Racemates einer Verbindung der obigen Formel I, worin $R^8$ die Gruppe $-O(CH_2)_2-$ bedeutet und die übrigen Symbole die oben angegebene Bedeutung haben, eine Verbindung der allgemeinen Formel

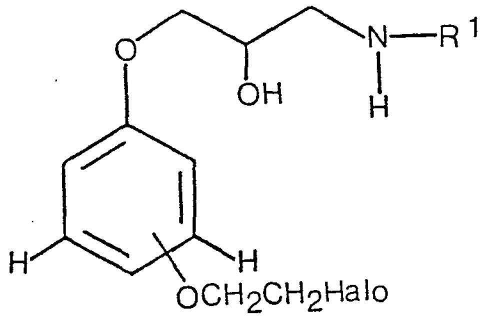

XIII

worin $R^1$ die oben angegebene Bedeutung hat, mit einer Verbindung der obigen Formel XI behandelt, und

i) erwünschtenfalls, eine erhaltene racemische Mischung in die optischen Antipoden auftrennt, und

j) erwünschtenfalls, eine erhaltene Verbindung in ein pharmazeutisch annehmbares Säureadditionssalz überführt.

34. Arzneimittel, enthaltend ein Phenylpiperazin-Derivat der in Anspruch 1 wiedergegebenen Formel I, ein entsprechendes Racemat oder ein pharmazeutisch annehmbares Säureadditionssalz davon.

35. α- und β-Adrenergisch blockierendes und sekretionshemmendes Mittel, enthaltend ein Phenylpiperazin-Derivat der in Anspruch 1 wiedergegebenen Formel I, ein entsprechendes Racemat oder ein pharmazeutisch annehmbares Säureadditionssalz davon.

36. Arzneimittel, enthaltend (S)-1-[2-(4-(2-Hydroxy-3-isopropylaminopropoxy)phenoxy)äthyl]-4-phenylpiperazin.

37. Arzneimittel, enthaltend (R,S)-1-[2-(4-(2-Hydroxy-3-isopropylaminopropoxy)phenoxy)äthyl]-4-phenylpiperazin.

38. Arzneimittel, enthaltend (S)-1-[6-(4-(2-Hydroxy-3-isopropylaminopropoxy)phenoxy)hexyl]-4-phenylpiperazin.

39. Arzneimittel, enthaltend 4-[(2-Hydroxy-3-isopropylaminopropoxy)phenyl]-N-[2-(4-phenyl-1-piperazinyl)-äthyl]acetamid.

40. Arzneimittel, enthaltend 4-[(2-Hydroxy-3-isopropylaminopropoxy)phenyl]-N-[6-(4-phenyl-1-piperazinyl)-hexyl]acetamid.

41. α- und β-Adrenergisch blockierendes und sekretionshemmendes Mittel, enthaltend (S)-1-[2-(4-(2-Hydroxy-3-isopropylaminopropoxy)phenoxy)äthyl]-4-phenylpiperazin.

42. α- und β-Adrenergisch blockierendes und sekretionshemmendes Mittel, enthaltend (R,S)-1-[2-(4-(2-Hydroxy-3-isopropylaminopropoxy)phenoxy)äthyl]-4-phenylpiperazin.

43. α- und β-Adrenergisch blockierendes und sekretionshemmendes Mittel, enthaltend (S)-1-[6-(4-(2-Hydroxy-3-isopropro-

0005142

pylaminopropoxy)phenoxy)hexyl]-4-phenylpiperazin.

44. α- und β-Adrenergisch blockierendes und sekretionshemmendes Mittel, enthaltend 4-[(2-Hydroxy-3-isopropylaminopropoxy)-phenyl]-N-[2-(4-phenyl-1-piperazinyl)äthyl]acetamid.

45. α- und β-Adrenergisch blockierendes und sekretionshemmendes Mittel, enthaltend 4-[(2-Hydroxy-3-isopropylaminopropoxy)-phenyl]-N-[6-(4-phenyl-1-piperazinyl)hexyl]acetamid.

46. Verwendung eines Phenylpiperazin-Derivates der in Anspruch 1 wiedergegebenen Formel I, eines entsprechenden Racemates oder eines pharmazeutisch annehmbaren Säureadditionssalzes davon bei der Bekämpfung bzw. Verhütung von Krankheiten.

47. Verwendung eines Phenylpiperazin-Derivates der in Anspruch 1 wiedergegebenen Formel I, eines entsprechenden Racemates oder eines pharmazeutisch annehmbaren Säureadditionssalzes davon bei der Bekämpfung von Bluthochdruck und Spasmen.

48. Verwendung von (S)-1-[2-(4-(2-Hydroxy-3-isopropylaminopropoxy)phenoxy)äthyl]-4-phenylpiperazin bei der Bekämpfung bzw. Verhütung von Krankheiten.

49. Verwendung von (R,S)-1-[2-(4-(2-Hydroxy-3-isopropylaminopropoxy)phenoxy)äthyl]-4-phenylpiperazin bei der Bekämpfung bzw. Verhütung von Krankheiten.

50. Verwendung von (S)-1-[6-(4-(2-Hydroxy-3-isopropylaminopropoxy)phenoxy)hexyl]-4-phenylpiperazin bei der Bekämpfung bzw. Verhütung von Krankheiten.

51. Verwendung von 4-[(2-Hydroxy-3-isopropylaminopropoxy)phenyl]-N-[2-(4-phenyl-1-piperazinyl)äthyl]acetamid bei der Bekämpfung bzw. Verhütung von Krankheiten.

52. Verwendung von 4-[(2-Hydroxy-3-isopropylaminopropoxy)phenyl]-N-[6-(4-phenyl-1-piperazinyl)hexyl]acetamid bei der Bekämpfung bzw. Verhütung von Krankheiten.

53. Verwendung von (S)-1-[2-(4-(2-Hydroxy-3-isopropylaminopropoxy)phenoxy)äthyl]-4-phenylpiperazin bei der Bekämpfung von Bluthochdruck und Spasmen.

54. Verwendung von (R,S)-1-[2-(4-(2-Hydroxy-3-isopropylaminopropoxy)phenoxy)äthyl]-4-phenylpiperazin bei der

Bekämpfung von Bluthochdruck und Spasmen.

55. Verwendung von (S)-1-[6-(4-(2-Hydroxy-3-isopropylaminopropoxy)phenoxy)hexyl]-4-phenylpiperazin bei der Bekämpfung von Bluthochdruck und Spasmen.

56. Verwendung von 4-[(2-Hydroxy-3-isopropylaminopropoxy)phenyl]-N-[2-(4-phenyl-1-piperazinyl)äthyl]acetamid bei der Bekämpfung von Bluthochdruck und Spasmen.

57. Verwendung von 4-[(2-Hydroxy-3-isopropylaminopropoxy)phenyl]-N-[6-(4-phenyl-1-piperazinyl)hexyl]acetamid bei der Bekämpfung von Bluthochdruck und Spasmen.

* * *

0005142

Nummer der Anmeldung

Europäisches Patentamt

# EUROPÄISCHER RECHERCHENBERICHT

EP 79 100 363.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 1 670 200 (BYK-GULDEN LOMBERG)<br>*Anspruch 2*<br>-- | 33 |
| | DE - A - 2 024 001 (YOSHITOMI)<br>*Anspruch 25*<br>-- | 33 |
| | DE - A - 2 503 751(CIBA-GEIGY)<br>*Anspruch 15*<br>-- | 16 |
| | CH - A - 491 857(MERCK)<br>*Formel I, Anspruch 1*<br>-- | 16,33 |
| | GB - A - 1 185 045 (IMPERIAL CHEMICAL INDUSTRIES)<br>*Anspruch 15*<br>-- | 33 |
| A | G. EHRHART u.a. "Arzneimittel", Band 2 1972<br>Verlag Chemie, Weinheim /Bergstr.<br>*Seite 177-181*<br>---- | |

KLASSIFIKATION DER ANMELDUNG (Int.Cl.²)

C 07 D 295/08
A 61 K 31/495
C 07 C 89/02
C 07 C 93/06
C 07 C 103/38
C 07 C 103/60
C 07 D 295/12
C 07 D 295/14
C 07 D 295/18
C 07 D 405/12

RECHERCHIERTE SACHGEBIETE (Int. Cl.²)

A 61 K 31/495
C 07 C 89/02
C 07 C 93/06
C 07 C 103/38
C 07 C 103/60
C 07 D 295/08
C 07 D 295/12
C 07 D 295/14
C 07 D 295/18
C 07 D 405/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 07-05-1979 | FROELICH |

EPA form 1503.1 06.78